(19) Europäisches Patentamt
European Patent Office
Office européen des brevets

(11) EP 1 782 689 A1

(12) **EUROPEAN PATENT APPLICATION**
published in accordance with Art. 158(3) EPC

(43) Date of publication:
**09.05.2007 Bulletin 2007/19**

(21) Application number: **05780367.8**

(22) Date of filing: **22.08.2005**

(51) Int Cl.:
***A01N 37/18*** *(2006.01)* ***A01N 41/10*** *(2006.01)*
***A01N 43/40*** *(2006.01)* ***C07C 323/42*** *(2006.01)*
***C07D 213/64*** *(2006.01)* ***C07D 213/70*** *(2006.01)*
***C07D 213/75*** *(2006.01)* ***C07D 239/42*** *(2006.01)*

(86) International application number:
**PCT/JP2005/015208**

(87) International publication number:
**WO 2006/022225 (02.03.2006 Gazette 2006/09)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **23.08.2004 JP 2004242259**

(71) Applicant: **NIHON NOHYAKU CO., LTD.**
**Tokyo 103-8236 (JP)**

(72) Inventors:
- **NAKAO, Hayami,**
  **Res. & Dev., NIHONNOHYAKU CO., LTD.**
  **Kawachinagano-shi,**
  **Osaka 5860094 (JP)**
- **MATSUZAKI, Yoshihiro,**
  **Research and Development**
  **Kawachinagano-shi,**
  **Osaka 5860094 (JP)**
- **FUJIOKA, Shinsuke,**
  **Research and Development**
  **Kawachinagano-shi, Osaka 5860094 (JP)**
- **MORIMOTO, Masayuki,**
  **Research and Development**
  **Kawachinagano-shi, Osaka 5860094 (JP)**
- **TOHNISHI, Masanori,**
  **Research and Development**
  **Kawachinagano-shi, Osaka 5860094 (JP)**
- **FISCHER, Rudiger**
  **Pulheim 50259 (DE)**
- **FUNKE, Christian**
  **42799 (DE)**
- **MALSAM, Olga**
  **Bonn 53227 (DE)**
- **ARNOLD, Christian**
  **Wachtberg-Adensdorf 53343 (DE)**
- **SANWALD, Erich**
  **Kiel 24159 (DE)**
- **HEMPEL, Waltraud**
  **Liederbach 65835 (DE)**
- **RECKMANN, Udo**
  **Keln 50823 (DE)**

(74) Representative: **Grünecker, Kinkeldey,**
**Stockmair & Schwanhäusser**
**Anwaltssozietät**
**Maximilianstrasse 58**
**80538 München (DE)**

(54) **OPTICALLY ACTIVE PHTHALAMIDE DERIVATIVE, AGRICULTURAL OR HORTICULTURAL INSECTICIDE, AND METHOD OF USING THE SAME**

(57) The present invention relates to an optically active phthalamide derivative represented by the following formula (I):

EP 1 782 689 A1

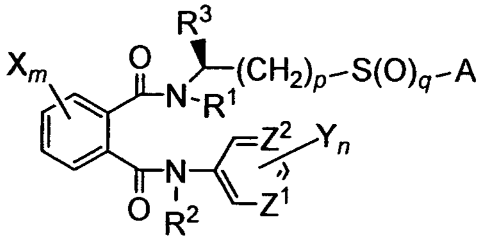

(I)

(wherein each of $R^1$ and $R^2$ represents H, optionally substituted C1-C6 alkyl, or C1-C6 alkoxycarbonyl; $R^3$ represents C1-C6 alkyl; A represents H, C1-C6 alkyl, C3-C6 alkenyl, C3-C6 alkynyl, optionally substituted phenoxy C1-C6 alkyl, or the like; p represents 0, 1, 2, 3, or 4; q represents 0, 1, or 2; X represents halogen, CN, $NH_2$, C1-C6 alkyl, C2-C6 alkenyl, C1-C6 alkylthio, or the like; m represents 0, 1, 2, 3, or 4; Y represents halogen, CN, $NH_2$, C1-C6 alkyl, optionally substituted phenyl, or the like; n represents 1, 2, 3, 4, or 5; and each of $Z^1$ and $Z^2$ represents C-Y or N; and further X and Y may form a condensed ring together with carbon atoms adjacent thereto on a phenyl ring), a salt thereof, an agrohorticultural insecticide comprising the above derivative as an active ingredient, and a method of use thereof. The optically active phthalamide derivative of the present invention has superior control activity as an agrohorticultural insecticide, and has excellent effects even when the insectide is used in admixture with other insecticides.

## Description

TECHNICAL FIELD

The present invention relates to an optically active phthalamide derivative, an agrohorticultural insecticide containing the compound as an active ingredient, and a method of using the same.

BACKGROUND ART

It has previously been known that a certain type of phthalamide derivative is useful as an agrohorticultural insecticide (see JP-A-11-240857, for example). In addition, it has also been known that a phthalamide derivative having a skeleton that is similar to that of the present invention is useful as an agrohorticultural insecticide (see JP-A-2001-131141, for example). However, these previous publications have disclosed only a racemic body and a part of optically active substances having a certain substituent. Thus, the optically active substance having a substituent of the present invention is a novel compound and it has not yet been known that it has superior performance as an insecticide.

DISCLOSURE OF INVENTION

PROBLEMS TO BE SOLVED BY THE INVENTION

In crop production in the agricultural and horticultural fields, damage caused by insect pests is still serious. For such reasons as emergence of insect pests that are resistant to the existing insecticides, the development of a novel agrohorticultural insecticide has been desired. In addition, for such reasons as aging of farmer population, various types of labor-saving methods have been desired. At the same time, the development of agrohorticultural insecticides suitable for such labor-saving methods has also been desired.

MEANS TO SOLVE THE PROBLEM

As a result of intensive studies directed towards the development of a novel agrohorticultural insecticide, the present inventors have found that the optically active phthalamide derivative represented by formula (I) of the present invention is a novel compound that had not been described in any publications, and that when compared with the compounds described in the aforementioned publications, it exhibits superior insecticidal effects at a low dosage. Further, they also have found that the optically active phthalamide derivative of the present invention can be a superior agrohorticultural insecticide exhibiting excellent activity to be absorbed from plant roots and translocated into the whole plants, in particular, when soil or the like is treated with this insecticide. Thus, the present inventors have completed the present invention.

That is to say, the present invention relates to an optically active phthalamide derivative represented by formula (I) indicated below:

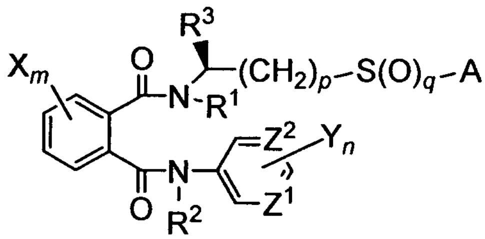

(I)

(wherein $R^1$ and $R^2$ may be same or different, and each of $R^1$ and $R^2$ represents a hydrogen atom; a C1-C6 alkyl group; a substituted C1-C6 alkyl group having one or more substituents that may be same or different and are selected from the group consisting of a halogen atom, a C1-C6 alkoxy group, a C1-C6 alkylthio group, a mono-C1-C6 alkylamino group, and a di-C1-C6 alkylamino group in which the alkyl groups may be same or different; or a C1-C6 alkoxycarbonyl group;
$R^3$ represents a C1-C6 alkyl group;
A represents a hydrogen atom; a C1-C6 alkyl group; a halo C1-C6 alkyl group; a C3-C6 alkenyl group; a halo C3-C6 alkenyl group; a C3-C6 alkynyl group; a halo C3-C6 alkynyl group; a C1-C6 alkoxy C1-C6 alkyl group; a halo C1-C6 alkoxy C1-C6 alkyl group; a C1-C6 alkylthio C1-C6 alkyl group; a halo C1-C6 alkylthio C1-C6 alkyl group; a C1-C6

alkylsulfinyl C1-C6 alkyl group; a halo C1-C6 alkylsulfinyl C1-C6 alkyl group; a C1-C6 alkylsulfonyl C1-C6 alkyl group; a halo C1-C6 alkylsulfonyl C1-C6 alkyl group; a C1-C6 alkylcarbonyl group; a mono-C1-C6 alkylcarbamoyl group; a di-C1-C6 alkylcarbamoyl group in which the alkyl groups may be same or different; a phenoxy C1-C6 alkyl group; a substituted phenoxy C1-C6 alkyl group having one or more substituents that may be same or different and are selected from the group consisting of a halogen atom, a C1-C6 alkyl group, a halo C1-C6 alkyl group, a C1-C6 alkoxy group, a halo C1-C6 alkoxy group, a C1-C6 alkylthio group, a halo C1-C6 alkylthio group, a C1-C6 alkylsulfinyl group, a halo C1-C6 alkylsulfinyl group, a C1-C6 alkylsulfonyl group, a halo C1-C6 alkylsulfonyl group, a mono-C1-C6 alkylamino group, a di-C1-C6 alkylamino group in which the alkyl groups may be same or different, a mono(halo C1-G6 alkyl) amino group, and a di(halo C1-C6 alkyl)amino group in which the alkyl groups may be same or different; a phenyl C1-C6 alkyl group; or a substituted phenyl C1-C6 alkyl group having, on the ring thereof, one or more substituents that may be same or different and are selected from the group consisting of a halogen atom, a C1-C6 alkyl group, a halo C1-C6 alkyl group, a C1-C6 alkoxy group, a halo C1-C6 alkoxy group, a C1-C6 alkylthio group, a halo C1-C6 alkylthio group, a C1-C6 alkylsulfinyl group, a halo C1-C6 alkylsulfinyl group, a C1-C6 alkylsulfonyl group, a halo C1-C6 alkylsulfonyl group, a mono-C1-C6 alkylamino group, a di-C1-C6 alkylamino group in which the alkyl groups may be same or different, a mono(halo C1-C6 alkyl)amino group, and a di(halo C1-C6 alkyl)amino group in which the alkyl groups may be same or different;

p represents an integer between 0 and 4;

q represents an integer between 0 and 2;

X may be same or different and represents a halogen atom, a cyano group, an amino group, a nitro group, a C1-C6 alkyl group, a halo C1-C6 alkyl group, a C2-C6 alkenyl group, a halo C2-C6 alkenyl group, a C2-C6 alkynyl group, a halo C2-C6 alkynyl group, a C1-C6 alkoxy group, a halo C1-C6 alkoxy group, a C1-C6 alkylthio group, a halo C1-C6 alkylthio group, a C1-C6 alkylsulfinyl group, a halo C1-C6 alkylsulfinyl group, a C1-C6 alkylsulfonyl group, a halo C1-C6 alkylsulfonyl group, a mono-C1-C6 alkylamino group, or a di-C1-C6 alkylamino group in which the alkyl groups may be same or different; and

m represents an integer between 0 and 4; and

X may form a condensed ring together with carbon atoms adjacent thereto on a phenyl ring, and such a condensed ring may have one or more substituents that may be same or different and are selected from the group consisting of a halogen atom; a C1-C6 alkyl group; a halo C1-C6 alkyl group; a C1-C6 alkoxy group; a halo C1-C6 alkoxy group; a C1-C6 alkylthio group; a halo C1-C6 alkylthio group; a C1-C6 alkylsulfinyl group; a halo C1-C6 alkylsulfinyl group; a C1-C6 alkylsulfonyl group; a halo C1-C6 alkylsulfonyl group; a mono-C1-C6 alkylamino group; a di-C1-C6 alkylamino group in which the alkyl groups may be same or different; a mono(halo C1-C6 alkyl)amino group; a di(halo C1-C6 alkyl) amino group in which the alkyl groups may be same or different; a phenyl group; a substituted phenyl group having one or more substituents that may be same or different and are selected from the group consisting of a halogen atom, a C1-C6 alkyl group, a halo C1-C6 alkyl group, a C1-C6 alkoxy group, a halo C1-C6 alkoxy group, a C1-C6 alkylthio group, a halo C1-C6 alkylthio group, a C1-C6 alkylsulfinyl group, a halo C1-C6 alkylsulfinyl group, a C1-C6 alkylsulfonyl group, a halo C1-C6 alkylsulfonyl group, a mono-C1-C6 alkylamino group, a di-C1-C6 alkylamino group in which the alkyl groups may be same or different, a mono(halo C1-C6 alkyl)amino group, and a di(halo C1-C6 alkyl)amino group in which the alkyl groups may be same or different; a heterocyclic group; and a substituted heterocyclic group having one or more substituents that may be same or different and are selected from the group consisting of a halogen atom, a C1-C6 alkyl group, a halo C1-C6 alkyl group, a C1-C6 alkoxy group, a halo C1-C6 alkoxy group, a C1-C6 alkylthio group, a halo C1-C6 alkylthio group, a C1-C6 alkylsulfinyl group, a halo C1-C6 alkylsulfinyl group, a C1-C6 alkylsulfonyl group, a halo C1-C6 alkylsulfonyl group, a mono-C1-C6 alkylamino group, a di-C1-C6 alkylamino group in which the alkyl groups may be same or different, a mono(halo C1-C6 alkyl)amino group, and a di(halo C1-C6 alkyl)amino group in which the alkyl groups may be same or different;

Y may be same or different and represents a hydrogen atom;a halogen atom; a cyano group; an amino group; a nitro group; a C1-C6 alkyl group; a halo C1-C6 alkyl group; a C2-C6 alkenyl group; a halo C2-C6 alkenyl group; a C2-C6 alkynyl group; a halo C2-C6 alkynyl group; a cyclo C3-C6 alkyl group; a halocyclo C3-C6 alkyl group; a C1-C6 alkoxy group; a halo C1-C6 alkoxy group; a C1-C6 alkylthio group; a halo C1-C6 alkylthio group; a C1-C6 alkylsulfinyl group; a halo C1-C6 alkylsulfinyl group; a C1-C6 alkylsulfonyl group; a halo C1-C6 alkylsulfonyl group; a mono-C1-C6 alkylamino group; a di-C1-C6 alkylamino group in which the alkyl groups may be same or different; a tri-C1-C6 alkylsilyl group in which the alkyl groups may be same or different; a phenyl group; a substituted phenyl group having one or more substituents that may be same or different and are selected from the group consisting of a halogen atom, a C1-C6 alkyl group, a halo C1-C6 alkyl group, a C1-C6 alkoxy group, a halo C1-C6 alkoxy group, a C1-C6 alkylthio group, a halo C1-C6 alkylthio group, a C1-C6 alkylsulfinyl group, a halo C1-C6 alkylsulfinyl group, a C1-C6 alkylsulfonyl group, a halo C1-C6 alkylsulfonyl group, a mono-C1-C6 alkylamino group, a di-C1-C6 alkylamino group in which the alkyl groups may be same or different, a mono(halo C1-C6 alkyl)amino group, and a di(halo C1-C6 alkyl)amino group in which the alkyl groups may be same or different; a heterocyclic group; or a substituted heterocyclic group having one or more substituents that may be same or different and are selected from the group consisting of a halogen atom, a C1-C6 alkyl group, a

halo C1-C6 alkyl group, a C1-C6 alkoxy group, a halo C1-C6 alkoxy group, a C1-C6 alkylthio group, a halo C1-C6 alkylthio group, a C1-C6 alkylsulfinyl group, a halo C1-C6 alkylsulfinyl group, a C1-C6 alkylsulfonyl group, a halo C1-C6 alkylsulfonyl group, a mono-C1-C6 alkylamino group, a di-C1-C6 alkylamino group in which the alkyl groups may be same or different, a mono(halo C1-C6 alkyl)amino group, and a di(halo C1-C6 alkyl)amino group in which the alkyl groups may be same or different; and

n represents an integer between 1 and 5; and

Y may form a condensed ring together with carbon atoms adjacent thereto on a phenyl ring, and such a condensed ring may have one or more substituents that may be same or different and are selected from the group consisting of a halogen atom; a C1-C6 alkyl group; a halo C1-C6 alkyl group; a C1-C6 alkoxy group; a halo C1-C6 alkoxy group; a C1-C6 alkylthio group; a halo C1-C6 alkylthio group; a C1-C6 alkylsulfinyl group; a halo C1-C6 alkylsulfinyl group; a C1-C6 alkylsulfonyl group; a halo C1-C6 alkylsulfonyl group; a mono-C1-C6 alkylamino group; a di-C1-C6 alkylamino group in which the alkyl groups may be same or different; a mono(halo C1-C6 alkyl)amino group; a di(halo C1-C6 alkyl)amino group in which the alkyl groups may be same or different; a phenyl group; a substituted phenyl group having one or more substituents that may be same or different and are selected from the group consisting of a halogen atom, a C1-C6 alkyl group, a halo C1-C6 alkyl group, a C1-C6 alkoxy group, a halo C1-C6 alkoxy group, a C1-C6 alkylthio group, a halo C1-C6 alkylthio group, a C1-C6 alkylsulfinyl group, a halo C1-C6 alkylsulfinyl group, a C1-C6 alkylsulfonyl group, a halo C1-C6 alkylsulfonyl group, a mono-C1-C6 alkylamino group, a di-C1-C6 alkylamino group in which the alkyl groups may be same or different, a mono(halo C1-C6 alkyl)amino group, and a di(halo C1-C6 alkyl)amino group in which the alkyl groups may be same or different; a heterocyclic group; or a substituted heterocyclic group having one or more substituents that may be same or different and are selected from the group consisting of a halogen atom, a C1-C6 alkyl group, a halo C1-C6 alkyl group, a C1-C6 alkoxy group, a halo C1-C6 alkoxy group, a C1-C6 alkylthio group, a halo C1-C6 alkylthio group, a C1-C6 alkylsulfinyl group, a halo C1-C6 alkylsulfinyl group, a C1-C6 alkylsulfonyl group, a halo C1-C6 alkylsulfonyl group, a mono-C1-C6 alkylamino group, a di-C1-C6 alkylamino group in which the alkyl groups may be same or different, a mono(halo C1-C6 alkyl)amino group, and a di(halo C1-C6 alkyl)amino group in which the alkyl group may be same or different; and

$Z^1$ and $Z^2$ may be same or different, and each of $Z^1$ and $Z^2$ represents C-Y (wherein Y has the same meaning as described above) or nitrogen atom;

provided that, when both $R^1$ and $R^2$ represent hydrogen atoms, $R^3$ represents a methyl group, X represents an iodine atom, m represents 1, Yn represents a 2-methyl-4-[1,2,2,2-tetrafluoro-1-(trifluoromethyl)ethyl] group, p represents 1, and q represents 0, then A is not a methyl group and ethyl group);

a salt thereof, an agrohorticultural insecticide comprising these substances as active ingredients, and a method of using the same.

The optically active phthalamide derivative represented by the formula (I) of the present invention exhibits excellent control effects as an agrohorticultural insecticide. In addition, even when it is used in admixture with other agrohorticultural insecticides, acaricides, nematicides, fungicides, herbicides, plant growth regulators, biotic pesticides, and the like, it exhibits excellent control effects.

BEST MODE FOR CARRYING OUT THE INVENTION

In the formula (I) representing the optically active phthalamide derivative of the present invention, the term "halogen atom" is used to mean a chlorine atom, bromine atom, iodine atom, or fluorine atom. The term "C1-C6 alkyl group" is used to mean a linear or branched alkyl group containing 1 to 6 carbon atoms. Examples of such a C1-C6 alkyl group may include a methyl group, ethyl group, n-propyl group, isopropyl group, n-butyl group, isobutyl group, s-butyl group, t-butyl group, n-pentyl group, neopentyl group, and n-hexyl group. The term "halo C1-C6 alkyl group" is used to mean a linear or branched alkyl group containing 1 to 6 carbon atoms, which is substituted with one or more halogen atoms that may be same or different. Examples of such a halo C1-C6 alkyl group may include a trifluoromethyl group, a difluoromethyl group, a perfluoroethyl group, a 1,2,2,2-tetrafluoro-1-(trifluoromethyl)ethyl group, a chloromethyl group, a bromomethyl group, a 1-bromoethyl group, and a 2,3-dibromopropyl group. The term "C1-C6 alkoxy group" is used to mean a linear or branched alkoxy group containing 1 to 6 carbon atoms. Examples of such a C1-C6 alkoxy group may include a methoxy group, ethoxy group, n-propoxy group, isopropoxy group, n-butoxy group, s-butoxy group, t-butoxy group, n-pentyloxy group, isopentyloxy group, neopentyloxy group, and n-hexyloxy group.

The term "halo C1-C6 alkoxy group" is used to mean a linear or branched C1-C6 alkyl group containing 1 to 6 carbon atoms, which is substituted with one or more halogen atoms that may be same or different. Examples of such a halo C1-C6 alkoxy group may include a difluoromethoxy group, a trifluoromethoxy group, and a 2,2,2-trifluoroethoxy group. The term "C1-C6 alkoxycarbonyl group" is used to mean a linear or branched alkoxycarbonyl group containing 1 to 6 carbon atoms. Examples of such a C1-C6 alkoxycarbonyl group may include a methoxycarbonyl group, ethoxycarbonyl group, n-propoxycarbonyl group, isopropoxycarbonyl group, n-butoxycarbonyl group, and t-butoxycarbonyl group. The term "C1-C6 alkylthio group" is used to mean a linear or branched alkylthio group containing 1 to 6 carbon

atoms. Examples of such a C1-C6 alkylthio group may include a methylthio group, ethylthio group, n-propylthio group, isopropylthio group, n-butylthio group, s-butylthio group, t-butylthio group, n-pentylthio group, isopentylthio group, and n-hexylthio group. The term "C1-C6 alkylsulfinyl group" is used to mean a linear or branched alkylsulfinyl group containing 1 to 6 carbon atoms. Examples of such a C1-C6 alkylsulfinyl group may include a methylsulfinyl group, ethylsulfinyl group, n-propylsulfinyl group, isopropylsulfinyl group, n-butylsulfinyl group, s-butylsulfinyl group, t-butylsulfinyl group, n-pentylsulfinyl group, isopentylsulfinyl group, and n-hexylsulfinyl group. The term "C1-C6 alkylsulfonyl group" is used to mean a linear or branched alkylsulfonyl group containing 1 to 6 carbon atoms. Examples of such a C1-C6 alkylsulfonyl group may include a methylsulfonyl group, ethylsulfonyl group, n-propylsulfonyl group, isopropylsulfonyl group, n-butylsulfonyl group, s-butylsulfonyl group, t-butylsulfonyl group, n-pentylsulfonyl group, isopentylsulfonyl group, and n-hexylsulfonyl group.

Examples of a "heterocyclic group" may include a pyridyl group, a pyridin-N-oxide group, a pyrimidinyl group, a furyl group, a tetrahydrofuryl group, a thienyl group, a tetrahydrothienyl group, a tetrahydropyranyl group, a tetrahydrothiopyranyl group, an oxazolyl group, an isoxazolyl group, an oxadiazolyl group, a thiazolyl group, an isothiazolyl group, a thiadiazolyl group, an imidazolyl group, a triazolyl group, and a pyrazolyl group. Examples of a "condensed ring" may include naphthalene, tetrahydronaphthalene, indene, indan, quinoline, quinazoline, indole, indoline, chromane, isochromane, benzodioxane, benzodioxole, benzofuran, dihydrobenzofuran, benzothiophene, dihydrobenzothiophene, benzoxazole, benzothiazole, benzimidazole, and indazole.

Examples of a salt of the optically active phthalamide derivative represented by the formula (I) of the present invention may include: inorganic acid salts such as hydrochloride, sulfate, nitrate, or phosphate; organic acid salts such as acetate, fumarate, maleate, oxalate, methanesulfonate, benzenesulfonate, or paratoluenesulfonate; and salts formed with sodium ion, potassium ion, or calcium ion.

In the optically active phthalamide derivative represented by the formula (I) of the present invention, $R^1$ and $R^2$ are particularly preferably hydrogen atoms, and $R^3$ is particularly preferably a methyl group. A is preferably a C1-C6 alkyl group, and particularly preferably a methyl group or ethyl group. p is preferably 1 or 2, and particularly preferably 1. q is an integer between 0 and 2, preferably 1 or 2, and particularly preferably 2. X is preferably a halogen atom, and particularly preferably a chlorine atom, bromine atom, or iodine atom. m is preferably 1 or 2, and particularly preferably 1. Y is preferably a C1-C6 alkyl group, halogen atom, halo C1-C6 alkyl group, or halo C1-C6 alkoxy group. n is preferably 2 or 3. $Z^1$ and $Z^2$ are particularly preferably CH.

The absolute stereochemistry of the optically active phthalamide derivative represented by the formula (I) of the present invention is a (S) form. It can be produced by the production method shown in the following formula, for example:

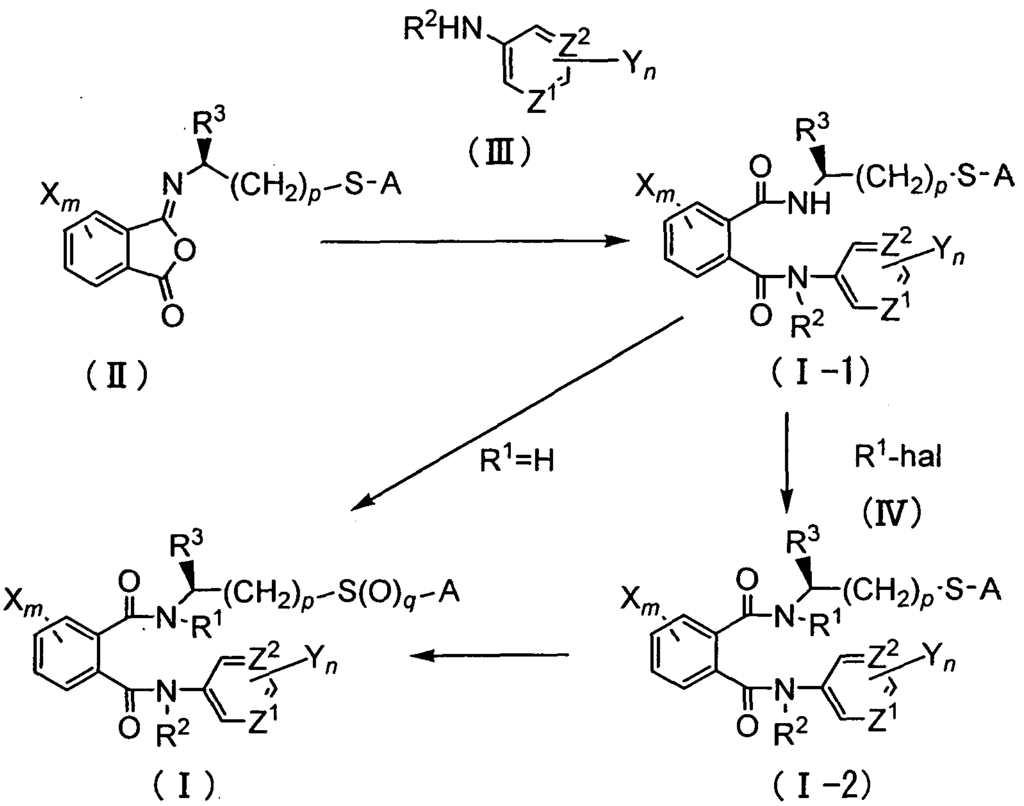


wherein $R^1$, $R^2$, $R^3$, A, X, Y, $Z^1$,$Z^2$,p, q, m, and n have the same meanings as described above, and hal represents a halogen atom.

A phthalisoimide derivative represented by formula (II) is allowed to react with an aniline derivative represented by formula (III) in the presence or absence of an acid or base in an inert solvent, so as to obtain a phthalamide derivative represented by formula (I-1). The phthalamide derivative (I-1) is then allowed to react with a halide represented by formula (IV) in the presence of a dehydrohalogenating agent and an inert solvent, so as to obtain a phthalamide derivative represented by formula (I-2). The phthalamide derivative (I-2) is then allowed to react with an oxidizing agent in the presence of an inert solvent, so as to produce a phthalamide derivative represented by the formula (I). When $R^1$ is a hydrogen atom, the phthalamide derivative represented by the formula (I) can be produced by allowing the phthalamide derivative represented by the formula (I-1) to react with an oxidizing agent in the presence of an inert solvent, without the process of producing the phthalamide derivative represented by the formula (I-2).

(1) Formula (II) → Formula (I-1)

In the present reaction, a product of interest can be produced by the method described in J. Med. Chem., 1967, Vol. 10, p. 982. Any inert solvent is used in the present invention, unless it significantly inhibits the progress of the reaction. Examples of such an inert solvent may include tetrahydrofuran, diethyl ether, methyl t-butyl ether, dioxane, chloroform, methylene chloride, chlorobenzene, toluene, acetonitrile, ethyl acetate, and butyl acetate. Examples of an acid used in the present reaction may include: organic acids such as acetic acid or trifluoroacetic acid; and inorganic acids such as hydrochloric acid or sulfuric acid. Such an acid may be used as appropriate in the range between a catalytic amount and an excessive molar amount with respect to the phthalisoimide derivative represented by the formula (II). Examples of a base may include: organic bases such as triethylamine or pyridine; and inorganic bases such as potassium carbonate, sodium bicarbonate, sodium carbonate, or sodium hydroxide. Such a base may be used as appropriate in the range between a catalytic amount and an excessive molar amount with respect to the phthalisoimide derivative represented by the formula (II). The reaction temperature may be in the range between 0°C and the boiling point of an inert solvent used. The reaction time is different depending on the reaction scale, the reaction temperature, etc. It is in the range between several minutes and 48 hours. After completion of the reaction, a product of interest may be isolated from the reaction mixture containing it according to common methods. The obtained product is purified by recrystallization, column chromatography, or the like, if necessary, so as to produce a product of interest.

(2) Formula (I-1) → Formula (I-2)

Any inert solvent is used in the present invention, unless it significantly inhibits the progress of the reaction. Examples of such an inert solvent may include: aromatic hydrocarbons such as benzene, toluene, or xylene; halogenated aromatic hydrocarbons such as fluorobenzene, chlorobenzene, or dichlorobenzene; halogenated hydrocarbons such as methylene chloride, chloroform, or carbon tetrachloride; chain or cyclic ethers such as diethyl ether, dioxane, or tetrahydrofuran; esters such as ethyl acetate; amides such as dimethylformamide or dimethylacetamide; acids such as acetic acid; dimethyl sulfoxide; and 1,3-dimethyl-2-imidazolidinone. These inert solvents may be used singly or in combination of two or more types. Examples of a dehydrohalogenating agent may include: organic bases such as triethylamine or pyridine; and inorganic bases such as potassium carbonate, sodium bicarbonate, sodium carbonate, or sodium hydroxide. Since the present reaction is an equimolar reaction, all reactants may be used at an equimolar amount. However, it is also possible to use a certain reactant at an excessive amount. The reaction temperature may be between room temperature and the reflux temperature of an inert solvent used. The reaction time is different depending on the reaction scale, the reaction temperature, etc. It may be selected as appropriate from the range between several minutes and 48 hours. After completion of the reaction, a product of interest may be isolated from the reaction mixture containing it according to common methods. The obtained product is purified by recrystallization, column chromatography, or the like, if necessary, so as to produce a product of interest. In addition, it is also possible to subject the obtained product of interest to the following reaction without isolation of the product from the reaction mixture.

(3) Formula (I-1) or (I-2) → Formula (I)

Examples of an inert solvent used in the present reaction may include: halogenated hydrocarbons such as methylene chloride or chloroform; aromatic hydrocarbons such as toluene or xylene; halogenated aromatic hydrocarbons such as fluorobenzene, chlorobenzene, or dichlorobenzene; acids such as acetic acid; and alcohols such as methanol, ethanol, or propanol. Examples of an oxidizing agent may include metachloroperbenzoic acid, peracetic acid, potassium metaperiodate, potassium hydrogen persulfate (oxone), and hydrogen peroxide. Such an oxidizing agent may be used in an amount between 0.5 and 3 equivalents with respect to the phthalamide derivative represented by the formula (I-1) or (I-2). The reaction temperature may be in the range between -50°C and the boiling point of an inert solvent used. The reaction time is different depending on the reaction scale, the reaction temperature, etc. It may be selected as appropriate from the range between several minutes and 24 hours. After completion of the reaction, a product of interest may be isolated from the reaction mixture containing it according to common methods. The obtained product can be purified by recrystallization, column chromatography, or the like, if necessary, so as to produce a product of interest.

The phthalisoimide derivative represented by the formula (II) that is used as a raw material compound of the present invention can be produced from an optically active amine represented by formula (V) according to the production methods described in JP-A-11-240857 and JP-A-2001-131141, as shown in the following formula:

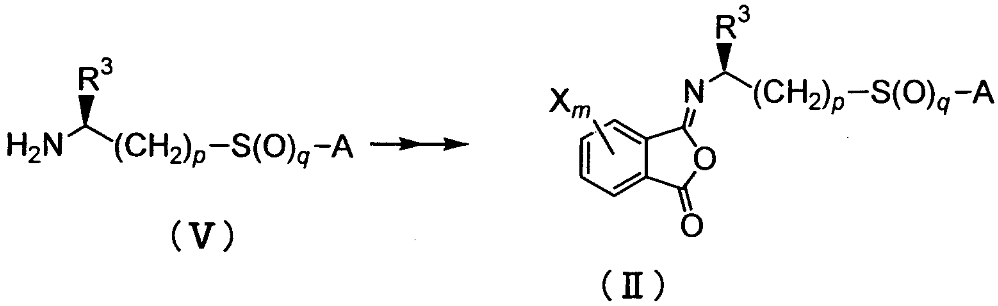

(V)

(II)

wherein $R^3$, A, X, p, q, and m have the same meanings as described above.

The optically active amine represented by the formula (V) can be produced from an optically active amino alcohol according to known methods (for example, Tetrahedron Lett., 1989, Vol. 30, p. 2653), by the production method indicated below, for example.

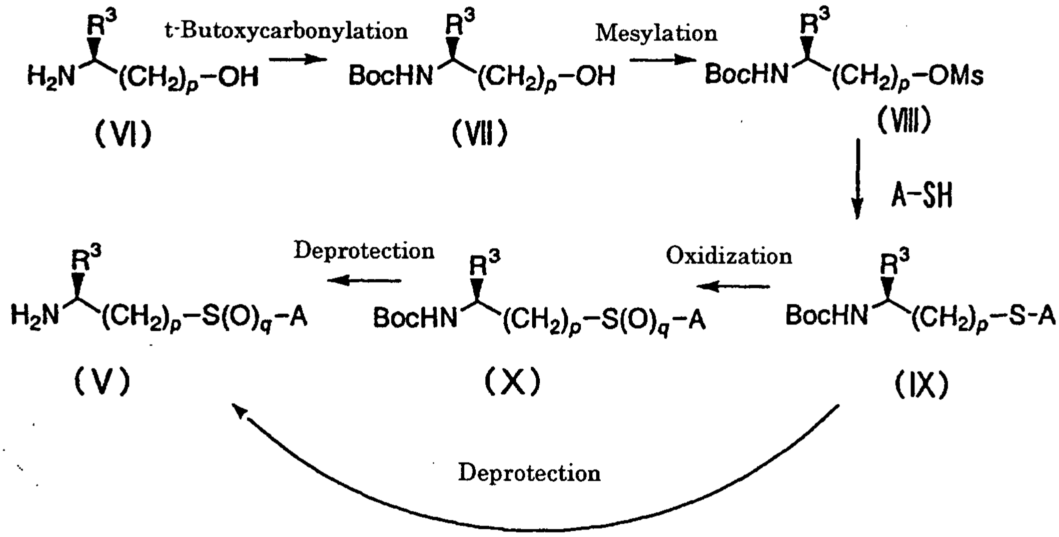


wherein $R^3$, A, p, and q have the same meanings as described above; Boc represents a t-butoxycarbonyl group; and Ms represents a mesyl group.

An optically active amino alcohol represented by formula (VI) is subjected to t-butoxycarbonylation, so as to obtain a carbamate represented by formula (VII). The carbamate is then subjected to mesylation, so as to obtain a mesylate represented by formula (VIII). The obtained mesylate is then allowed to react with a thiol, so as to obtain a sulfide represented by formula (IX). When q is 0, the obtained sulfide is directly deprotected, so as to produce an optically active amine of interest represented by the formula (V). In contrast, when q is 1 or 2, the sulfur atom of the sulfide represented by the formula (IX) is oxidized to obtain a sulfoxide or sulfone represented by formula (X), followed by the deprotection of the obtained product, so as to produce an optically active amine of interest represented by the formula (V).

The agrohorticultural insecticide, containing an optically active phthalamide derivative represented by the formula (I) of the present invention as an active ingredient, are suitable for controlling various insect pests such as agrohorticultural insect pests, stored grain insect pests, house insect pests, sanitary insect pests, nematodes, etc., which are injurious to paddy rice, fruit trees, vegetables, other crops, flowers, ornamental plants, etc. They have a marked insecticidal effect, for example, on LEPIDOPTERA including summer fruit tortrix (Adoxophes orana fasciata), smaller tea tortrix (Adoxophyes sp.), Manchurian fruit moth (Grapholita inopinata), oriental fruit moth (Grapholita molesta), soybean pod border (Leguminovora glycinivorella), mulberry leafroller (Olethreutes mori), tea leafroller (Caloptilia thevivora), Caloptilia sp. (Caloptilia zachrysa), apple leafminer (Phyllonorycter ringoniella), pear barkminer (Spulerrina astaurota), common white (Piers rapae crucivora), tobacco budworm (Heliothis sp.), codling moth (Laspeyresia pomonella), diamondback moth (Plutella xylostella), apple fruit moth (Argyresthia conjugella), peach fruit moth (Carposina niponensis), rice stem borer (Chilo suppressalis), rice leafroller (Cnaphalocrocis medinalis), tobacco moth (Ephestia elutella), mulberry pyralid (Glyphodes pyloalis), yellow rice borer (Scirpophaga incertulas), rice skipper (Parnara guttata), rice armyworm (Pseudaletia separata), pink borer (Sesamia inferens), common cutworm (Spodoptera litura), beet armyworm (Spodoptera exigua), etc.; HEMIPTERA including aster leafhopper (Macrosteles fascifrons), green rice leafhopper (Nephotettix cincticepts), brown rice planthopper (Nilaparvata lugens), whitebacked rice planthopper (Sogatella furcifera), citrus psylla (Diaphorina citri), grape whitefly (Aleurolibus taonabae), sweetpotato whitefly (Bemisia tabaci), greenhouse whitefly (Trialeurodes vaporariorum), turnup aphid (Lipaphis erysimi), green peach aphid (Myzus persicae), Indian wax scale (Ceroplastes ceriferus), cottony citrus scale (Pulvinaria aurantii), camphor scale (Pseudaonidia duplex), san Jose scale (Comstockaspis perniciosa), arrowhead scale (Unapsis yanonensis), etc.; TYLENCHIDA including root-lesion nematode (Pratylenchus sp.), soybean beetle (Anomala rufocuprea), Japanese beetle (Popillia japonica), tobacco beetle (Lasioderma serricorne), powderpost beetle (Lyctus brunneus), twenty-eight-spotted ladybird (Epilachna vigintiotopunctata), azuki bean weevil (Callosobruchus chinensis), vegetable weevil (Listroderes costirostris), maize weevil (Sitophilus zeamais), boll weevil (Anthonomus grandis grandis), rice water weevil (Lissorhoptrus oryzophilus), cucurbit leaf beetle (Aulacophora femoralis), rice leaf beetle (Oulema oryzae), striped flea beetle (Phyllotreta striolata), pine shoot beetle (Tomicus piniperda), Colorado potato beetle (Leptinotarsa decemlineata), Mexican bean beetle (Epilachna varivestis), corn rootworm (Diabrotica sp.), etc.; DIPTERA including (Dacus(Zeugodacus) cucurbitae), oriental fruit fly (Dacus(Bactrocera) dorsalis), rice leafminer (Agnomyza oryzae), onion maggot (Delia antiqua), seedcorn maggot (Delia platura), soybean pod gall midge (Asphondylia sp.), muscid fly (Musca domestica), house mosquito (Culex pipiens pipiens), etc.; TYLENCHIDA including coffee root-lesion nematode (Pratylenchus coffeae), potato cyst nematode (Globodera rostochiensis), root-knot nematode (Meloidogyne sp.), citrus nematode (Tylenchulus semipenetrans), Aphelenchus sp. (Aphelenchus avenae), chrysanthemum foliar (Aphelenchoides ritzemabosi), etc.; and ACARINA including citrus red mite (Panonychus citri), fruit tree red spider mite (Panonychus ulmi), carmine spider mite (Tetranychus cinnabarinus), Kanzawa spider mite (Tetranychus Kanzawai Kishida), two-spotted spider mite (Tetranychus urticae Koch), pink tea rust mite (Acaphylla theae), pink citrus rust mite (Aculops pelekassi), purple tea mite (Calacarus carinatus), pear rust mite (Epitrimerus pyri), etc.

The agrohorticultural insecticide, containing an optically active phthalamide derivative represented by the formula (I) of the present invention as an active ingredient, exhibit a significant controlling effect against all species of termites harmful to houses, construction materials, furniture, leathers, fibers, vinyl articles, wires and cables, such as, Rhinotermitidae including formosan subterranean termite (Coptotermes formosanus Shiraki) and Reticulitermes speratus (Kolbe); Reticulitermes hesperus, Reticulitermes tibialis and Reticulitermes flavipes inhabiting North America; Reticulitermes lucifugus and Reticulitermes santonesis inhabiting the coast of Mediterranean Sea; Incisitermes minor (Hagen); termitidae including Odontotermes formosanus (Shiraki); Kalotermitidae including Cryptotermes domesticus (Haviland); and Termopsidae including Hodotermopsis japonica (Holmgren), etc. It also exhibit a significant controlling effect against ants harmful to agricultural plants or human by coming into houses or public facilities such as parks, in a low dosage, the ants including Formicidae including Monomorium pharaoni Linnes, Monomorium nipponense Wheeler, Camponotus kiusiuensis Santschi, Formica japonica Motschulsky and Lasius fuliginosus (Latreille); fireants (Solenopsis richteri, Solenopsis invicta, Solenopsis geminata (F)) inhabiting North America.

The agrohorticultural insecticide, which contains an optically active phthalamide derivative represented by the formula (I) of the present invention as an active ingredient has a marked controlling effect on the above-exemplified insect pests, sanitary pests and/or nematodes, which are injurious to paddy field crops, upland crops, fruit trees, vegetables and other crops, flowers and ornament plants, and the like. Therefore, the desired effect of the agrohorticultural insecticide of the present invention can be exhibited by applying the insecticide to the nursery facility, paddy field, upland field, fruit trees, vegetables, other crops, seeds of flowers and ornament plants, paddy field water, stalks and leaves, or soil at a season at which the insect pests, sanitary pests or nematodes are expected to appear, before their appearance or at the time when their appearance is confirmed. Particularly, a preferable application for using the agrohorticultural insecticide of the present invention is the application for which both of "penetration and translocation" are utilized, wherein the present agrohorticultural insecticide is applied to the nursery soil of crops, ornamental plants or the like; the picking-in hole soil at a transplantation; the plant roots; the irrigation water; or the cultural water of a water culture; so as to absorb the optically active phthalamide derivatives of the present invention from the roots through or not through the soil. Moreover, in recent years, IPM (integrated pest management) technology using genetically modified products (herbicide-resistant products, pest-resistant products into which an insecticidal protein-generating gene has been incorporated, disease-resistant products into which a gene generating a substance inducing resistance to disease has been incorporated, products with improved taste, products with improved keeping quality, products with improved yield, etc.), insect pheromones (communication-disturbing agents used for Tortricidae or Mamestra, etc.), or natural enemy insects, has been developed. The agrohorticultural insecticide of the present invention can be used in admixture with such a technique, or can be used in systematization therewith.

Plants to which the agrohorticultural insecticide of the present invention can be applied are not particularly limited. Such plants include the following examples.

Examples of such plants may include cereals (e.g. rice, barley, wheat, rye, oat, corn, etc.), beans (soybeans, adzuki beans, horse beans, peas, red beans, peanuts, etc.), orchards/fruits (apples, citrus fruits, pomes, grapes, peaches, ume apricots, cherries, walnuts, chestnuts, almonds, bananas, strawberries, etc.), leave/fruit crops (cabbage, tomato, spinach, broccoli, lettuce, onion, green onion, bell pepper, egg plant, green pepper, etc.), root crops (carrot, potato, sweet potato, aroid, Japanese radish, lotus root, turnip, burdock, garlic, etc.), processed products (cotton, hemp, beet, hop, sugar cane, sugar beet, olive, gum, coffee, tobacco, tea, etc.), pepos (pumpkin, cucumber, Cucumis melo, watermelon, melon, etc.), pasture plants (orchard grass, sorgum, timothy, clover, alfalfa, etc.), turf grasses (lawn, bent grass, etc.), ornamental plants such as perfume (lavender, rosemary, thyme, parsley, pepper, ginger, etc.), flowering plants (chrysanthemum, rose, carnation, orchid, etc.), garden trees (ginkgo, cherry tree, Japanese laurel, etc.), and forest trees (Abies *sachalinensis,* Picea jezoensis, pine, thuja, Japanese cedar, Japanese cypress, etc.). Moreover plants to which the agrohorticultural insecticide of the present invention can be applied include transgenic plants or plant cultivars. The transgenic plants or plant cultivars (i.e. those obtained by genetic engineering) include all plants which, in the genetic modification, received genetic material which imparts particularly advantageous useful traits to these plants. Examples of transgenic plants which may be mentioned are the important crop plants, such as cereals (wheat, rice), maize, soya beans, potatoes, cotton, tobacco, oilseed rape and also fruit plants (with the fruits apples, pears, citrus fruits and grapes), and particular emphasis is given to maize, soya beans, potatoes, cotton, tobacco and oilseed rape. Examples of such traits are better plant growth, increased tolerance to high or low temperatures, increased tolerance to drought or to water or soil salt content, increased flowering performance, easier harvesting, accelerated maturation, higher harvest yields, better quality and/or a higher nutritional value of the harvested products, better storage stability and/or processability of

the harvested products. Further and particularly emphasized examples of such traits are a better defence of the plants against animal and microbial pests, such as against insects, mites, phytopathogenic fungi, bacteria and/or viruses, and also increased tolerance of the plants to certain herbicidally active compounds. Traits that are particularly emphasized are the increased defence of the plants against insects, arachnids, nematodes and worms by toxins formed in the plants, in particular those formed in the plants by the genetic material from Bacillus thuringiensis (for example by the genes CryIA(a), CryIA(b), CryIA(c), CryIIA, CryIIIA, CryIIIB2, Cry9c Cry2Ab, Cry3Bb and CryIF and also combinations thereof) (hereinbelow referred to as "Bt plants"). Traits that are also particularly emphasized are the increased defence of plants against fungi, bacteria and viruses by systemic acquired resistance (SAR), systemin, phytoalexins, elicitors and also resistance genes and correspondingly expressed proteins and toxins. Traits that are furthermore particularly emphasized are the increased tolerance of the plants to certain herbicidally active compounds, for example imidazolinones, sulphonylureas, glyphosate or phosphinotricin (for example the "PAT" gene). The genes in question which impart the desired traits can also be present in combination with one another in the transgenic plants. Examples of "Bt plants" which may be mentioned are maize varieties, cotton varieties, soya bean varieties and potato varieties which are sold under the trade names YIELD GARD® (for example maize, cotton, soya beans), KnockOut® (for example maize), StarLink® (for example maize), Bollgard® (cotton), Nucotn® (cotton) and NewLeaf® (potato). Examples of herbicide-tolerant plants which may be mentioned are maize varieties, cotton varieties and soya bean varieties which are sold under the trade names Roundup Ready® (tolerance to glyphosate, for example maize, cotton, soya beans), Liberty Link® (tolerance to phosphinotricin, for example oilseed rape), IMI® (tolerance to imidazolinones) and STS® (tolerance to sulphonylureas, for example maize). Herbicide-resistant plants (plants bred in a conventional manner for herbicide tolerance) which may be mentioned include the varieties sold under the name Clearfield® (for example maize). Of course, these statements also apply to plant cultivars having these or still-to-be-developed genetic traits, which plants will be developed and/or marketed in the future. The above statements also apply to seeds ("seed" being used in the meaning set forth below) having these or still-to-be-developed genetic traits, which plants will be developed and/or marketed in the future.

In order to control various types of insect pests, the agrohorticultural insecticide of the present invention is applied at an amount effective for controlling insect pests or nematodes, directly, or in the form of being diluted with water or the like, or in the form of being suspended in water or the like, to plants regarding which the infestation of such insect pests or nematodes is forecasted. When the present agrohorticultural insecticide is applied to orchards, cereals, vegetables, and the like, infested with insect pests or nematodes, for example, it is applied to leaves or stems thereof, or it can also be applied by treatments including: seed treatments such as immersion of seeds in the agent, or dust coating of seeds; and soil treatments in which the soil is treated with the agent, so as to allow plants to absorb the agent from roots thereof, such as mixing of the agent into all layers of the soil, row treatment, mixing the agent into bed soil, cell nursery treatment, planting pit treatment, bedside treatment, top dressing, rice box treatment, or submerged application. In addition, addition of the agent to the solution in solution (slop) culture, fumigation, or injection of the agent into tree trunks, may also be applied.

Moreover, the agrohorticultural insecticide of the present invention may be applied at an amount effective for controlling insect pests or nematodes, directly, or in the form of being diluted with water or the like, or in the form of being suspended in water or the like, to plants regarding which the infestation of such insect pests or nematodes is forecasted. For example, the present agrohorticultural insecticide is applied to stored grain insect pests, house insect pests, sanitary insect pests, forest insect pests, or the like. Otherwise, it may also be used by methods such as application to house construction materials, fumigation, or baiting.

Examples of a seed treatment method may include: a method which comprises diluting or not diluting a liquid or solid formulation with water and then immersing seeds in the obtained solution , so as to allow an active ingredient to permeate into the seeds; a method which comprises mixing a solid or liquid formulation with seeds or dust-coating, so as to allow an active ingredient to attach onto the surface of the seeds; a method which comprises mixing an active ingredient with an adhesive carrier such as a resin or polymer and then coating seeds with the resultant product; and a method of applying the agent around seeds at the same time of planting.

The term "seed" that is subjected to the above seed treatments means a plant body that is at the initial stage of culture for the reproduction of plants. Examples of such a seed may include a seed, a bulb, a tuber, a seed tuber, a stock bud, a propagule, a bulblet, and a plant body used for vegetative reproduction in cutting culture.

The term "soil" or "culture carrier" for plants in the case of applying the use method of the present invention means a supporting medium for the culture of plants, and particularly, a supporting medium in which plant roots are allowed to extend. The material of the soil or culture carrier is not specifically limited, as long as plants can grow thereon. Thus, such a soil or culture carrier may be what is called soil, a raising planting mat, water, or the like. Examples of a specific material may include sand, pumice, vermiculite, diatomite, agar, a gelatinous substance, a polymer, a rock wool, a glass wool, a wood chip, and a bark.

Examples of a method of applying the agent to the stems or leaves of plants, stored grain insect pests, house insect pests, sanitary insect pests, forest insect pests, or the like, may include: a method which comprises diluting a liquid formulation such as an emulsion or flowable or a solid formulation such as a wettable powder or water dispersible

granule with water, as appropriate, and then applying the obtained solution to the target; a method of applying a dust; and fumigation.

Examples of a method of applying the agent to the soil may include: a method which comprises diluting or not diluting a liquid formulation and then applying the obtained solution to the bottom portion of a plant body or a nursery bed for raising seedling; a method which comprises applying a granule to the bottom portion of a plant body or a nursery bed for raising seedling; a method which comprises applying the agent that is in the form of a dust, a wettable powder, a water dispersible granule , a granule, or the like, to the soil before sowing or transplantation, so as to mix the agent into the soil as a whole; and a method which comprises applying the agent that is in the form of a dust, a wettable powder, a water dispersible granule, a granule, or the like, to planting pits or rows before sowing or planting of plant bodies.

With regard to a method of applying the agent to a nursery box used for paddy rice, a formulation may be different depending on the period of application of the agent, such as application during sowing, application during greening period, or application during transplantation. The agent may be applied in a formulation such as a dust, a water dispersible granule, or a granule. The agent may also be applied by mixing it with molding. Mixing of the molding with a dust, a water dispersible granule , or a granule, may be applied. For example, the agent may be mixed into seedbed soil, cover soil, or the molding as a whole. The agent may simply be applied by placing the molding and various types of formulations alternately in a layer form.

As a method of applying the agent to the paddy field, the agent that is in a solid formulation, such as a jumbo formulation, a pack formulation, a granule, or a water dispersible granule, or the formulation that is in a liquid form, such as a flowable or an emulsion, is generally applied in the paddy field filled with water. In addition, during the rice planting period, the agent that is in an appropriate formulation may be applied to or injected into the soil, directly or after being mixed with fertilizer. Moreover, the agent that is in the form of an emulsion or a flowable is applied to the source of water supply to the paddy field, such as a waterspout or irrigation equipment, so as to apply the agent together with the supply of water in a labor-saving manner.

For field crops, the agent can be applied to seeds or a culture carrier that is adjacent to plant bodies, during the sowing and raising seedling periods. In the case of plants that are directly sown to the field, direct application of the agent to seeds, and application of the agent to the bottom portions of plants during culture, are preferable. In addition, a method of applying the agent that is in a granule form and a method of applying the agent that has been diluted or undiluted with water in a liquid state are also possible. A method which comprises mixing the agent that is in a granule form with a culture carrier before sowing and then sowing seeds on the carrier is also preferable.

As a method of applying the agent during the sowing and raising planting periods of culture plants to be transplanted, a method of directly applying the agent to seeds, a method of applying the agent that is in a liquid state to a nursery bed for raising planting, and a method of applying the agent that is in a granule form, are preferable. In addition, a method of applying the agent that is in a granule form into planting pits during fix planting, and a method of mixing the agent with a culture carrier around the place to which the plants to be transplanted, are also preferable.

The agrohorticultural insecticide of the present invention is generally prepared into suitable formulations according to a conventional manner for preparation of agrochemicals.

That is, the optically active phthalamide derivative represented by the formula (I) and, optionally, an adjuvant are blended with a suitable inert carrier in a proper proportion and prepared into a suitable formulation such as a flowable, emulsion, soluble concentrate, wettable powder, granules, dust, tablets, pack or the like through dissolution, dispersion, suspension, mixing, impregnation, adsorption or sticking.

The inert carrier usable in the present invention may be either solid or liquid. As a material usable as the solid carrier, there can be exemplified soybean flour, cereal flour, wood flour, bark flour, saw dust, powdered tobacco stalks, powdered walnut shells, bran, powdered cellulose, extraction residue of vegetables, powdered synthetic polymers or resins, clays (e.g. kaolin, bentonite, and acid clay), talcs (e.g. talc and pyrophyllite), silica powders or flakes (e.g. diatomaceous earth, silica sand, mica and white carbon [synthetic, high-dispersion silicic acid, also called finely divided hydrated silica or hydrated silicic acid, some of commercially available products contain calcium silicate as the major component]), activated carbon, powdered sulfur, pumice, calcined diatomaceous earth, ground brick, fly ash, sand, calcium carbonate, calcium phosphate and other inorganic or mineral powders, plastic carriers such as polyethylene, polypropylene, polyvinylidene chloride and the like, chemical fertilizers (e.g. ammonium sulfate, ammonium phosphate, ammonium nitrate, urea and ammonium chloride), and compost. These carriers may be used alone or as a mixture thereof.

A material usable as the liquid carrier is selected from materials that have solubility in themselves or which are without such solubility but are capable of dispersing an active ingredient with the aid of an adjuvant. The following are typical examples of the liquid carrier and can be used alone or as a mixture thereof: water, alcohols (e.g. methanol, ethanol, isopropanol, butanol and ethylene glycol), ketones (e.g. acetone, methyl ethyl ketone, methyl isobutyl ketone, diisobutyl ketone and cyclohexanone), ethers (e.g. ethyl ether, dioxane, Cellosolve, dipropyl ether and tetrahydrofuran), aliphatic hydrocarbon (e.g. kerosene and mineral oils), aromatic hydrocarbons (e.g. benzene, toluene, xylene, solvent naphtha and alkylnaphthalenes), halogenated hydrocarbons (e.g. dichloroethane, chloroform, carbon tetrachloride and chlorobenzene), esters (e.g. ethyl acetate, diisopropyl phthalate, dibutyl phthalate and dioctyl phthalate), amides (e.g.

dimethylformamide, diethylformamide and dimethylacetamide), nitriles (e.g. acetonitrile), and dimethyl sulfoxide.

The following are typical examples of the adjuvant, which are used depending upon purposes and used alone or in combination is some cases, or need not be used at all.

To emulsify, disperse, dissolve and/or wet a compound as active ingredient, a surfactant can be used. As the surfactant, there can be exemplified polyoxyethylene alkyl ethers, polyoxyethylene alkylaryl ethers, polyoxyethylene higher fatty acid esters, polyoxyethylene resonates, polyoxyethylene sorbitan monolaurate, polyoxyethylene sorbitan monooleate, alkylarylsulfonates, naphthalene sulfonic acid condensation products, ligninsulfonates and higher alcohol sulfate.

Further, to stabilize the dispersion of a compound as active ingredient, tackify it and/or bind it, the adjuvants exemplified below may also be used, namely, there may also be used adjuvants such as casein, gelatin, starch, methyl cellulose, carboxymethyl cellulose, gum arabic, poly(vinyl alcohol)s, turpentine, bran oil, bentonite and ligninsulfonates.

To improve the flowability of a solid product, the following adjuvants may also be used, namely, there may be used adjuvants such as waxes, stearates, alkyl phosphates, etc.

Adjuvants such as naphthalenesulfonic acid condensation products and polycondensates of phosphates may be used as a peptizer for dispersible products, and adjuvants such as silicone oils may also be used as a defoaming agent.

Adjuvants such as 1,2-benzisothiazoline-3-one, 4-chloro-3,5-xylenol, butyl p-hydroxybenzoate may also be added as a preservative.

Further, if necessary, functional spreading agents, synergists such as metabolic inhibitor like piperonyl butoxide, anti-freezing agents such as propylene glycol, antioxidants such as BHT, ultraviolet absorbers, and the like may also be added.

The content of the compound as active ingredient may be varied as required, and the compound as active ingredient may be used in a proportion properly chosen in the range of 0.01 to 90 parts by weight per 100 parts of the agrohorticultural insecticide. For example, in dusts, granules, emulsion or wettable powder, the suitable content of the compound as active ingredient is from 0.01 to 50 % by weight.

The agrohorticultural insecticide of the present invention is used to control a variety of insect pests in the following manner: it is applied to a crop on which the insect pests are expected to appear, or a site where appearance or growth of the insect pests is undesirable, as it is or after being properly diluted with or suspended in water or the like, in an amount effective for control of the insect pests.

The applying dosage of the agrohorticultural insecticide of the present invention is varied depending upon various factors such as a purpose, insect pests to be controlled, a growth state of a plant, tendency of insect pests appearance, weather, environmental conditions, a preparation form, an application method, an application site and application time. It may be properly chosen in the range of 0.001 g to 10 kg, preferably 0.01 g to 1 kg, (in terms of the compound as active ingredient) per 10 ares depending upon purposes.

The agrohorticultural insecticide of the present invention may be used in admixture with other agrohorticultural insecticides, acaricides, nematocides, fungicides, biotic pesticides or the like in order to expand both spectrum of controllable insect pest species and the period of time when effective application are possible or to reduce the dosage. Furthermore, the agrohorticultural insecticide of the present invention may be used in admixture with herbicides, plant growth regulators, fertilizers or the like, depending upon application situations.

As the other agrohorticultural insecticides, acaricides and nematocides, which are used for the above purpose, there can be exemplified agrohorticultural insecticides, acaricides and nematocides, such as Ethion, Trichlorfon, Metamidophos, Acephate, Dichlorvos, Mevinphos, Monocrotophos, Malathion, Dimethoate, Formothion, Mecarbam, Vamidothion, Thiometon, Disulfoton, Oxydeprofos, Naled, Methylparathion, Fenitrothion, Cyanophos, Propaphos, Fenthion, Prothiofos, Profenofos, Isofenphos, Temephos, Phenthoate, Dimethylvinphos, Chlorfenvinphos, Tetrachlorvinphos, Phoxim, Isoxathion, Pyraclofos, Methidathion, Chlorpyrifos, Chlorpyrifos-methyl, Pyridaphenthion, Diazinon, Pirimiphosmethyl, Phosalone, Phosmet, Dioxabenzophos, Quinalphos, Terbuphos, Ethoprophos, Cadusafos, Mesulfenfos, DPS (NK-0795), Phosphocarb, Fenamiphos, Isoamidophos, Fosthiazate, Isazophos, Ethoprophos, Fenthion, Fostietane, Dichlofenthion, Thionazin, Sulprofos, Fensulfothion, Diamidafos, Pyrethrin, Allethrin, Prallethrin, Resmethrin, Permethrin, Tefluthrin, Bifenthrin, Fenpropathrin, Cypermethrin, $\alpha$-Cypermethrin, Cyhalothrin, $\lambda$-Cyhalothrin, Deltamethrin, Acrinathrin, Fenvalerate, Esfenvalerate, Cycloprothrin, Ethofenprox, Halfenprox, Silafluofen, Flucythrinate, Fluvalinate, Methomyl, Oxamyl, Thiodicarb, Aldicarb, Alanycarb, Cartap, Metolcarb, Xylylcarb, Propoxur, Phenoxycarb, Fenobucarb, Ethiophencarb, Fenothiocarb, Bifenazate, Carbaryl, Pirimicarb, Carbofuran, Carbosulfan, Furathiocarb, Benfuracarb, Aldoxycarb, Diafenthiuron, Diflubenzuron, Teflubenzuron, Hexaflumuron, Novaluron, Lufenuron, Flufenoxuron, Chlorfluazuron, Fenbutatin oxide, tricyclohexyltin hydroxide, sodium oleate, potassium oleate, Methoprene, Hydroprene, Binapacryl, Amitraz, Dicofol, Kersen, Chrorobenzilate, Bromopropylate, Tetradifon, Bensultap, Benzoximate, Tebufenozide, Methoxyfenozide, pyridalyl, Chromafenozide, Propargite, Acequinosyl, Endosulfan, Diofenolan, Chlorfenapyl, Fenpyroximate, Tolfenpyrad, Fipronil, Tebufenpyrad, Triazamate, Etoxazole, Hexythiazox, nicotine sulfate, Nitenpyram, Acetamiprid, Thiacloprid, Imidacloprid, Thiamethoxam, Clothianidin, Dinotefuran, Fluazinam, Pyriproxyfen, Hydramethylnon, Pyrimidifen, Pyridaben, Cyromazin, TPIC (tripropyl isocyanurate), Pymetrozin, Clofentezin, Buprofezin, Thiocyclam, Fenazaquin, Chinomethionate, Indoxacarb, Polynactin complexes, Milbemectin, Abamectin, Emamectin-benzoate, Spinosad, BT (Bacillus thuringiensis), Azadirachtin, Rotenone, hydroxypropyl starch, Levamisole hydrochloride, Metam-sodium, Morantel tartrate, Dazomet, Trichlamide, Pasteuria penetrans, Monacrosporium-phymatophagum, etc.

As the agrohorticultural fungicides used for the same purpose as above, there can be exemplified agrohorticultural fungicides such as sulfur, lime sulfur, copper sulfate basic, Iprobenfos, Edifenfos, Tolclofos-methyl, Thiram, Polycarbamate, Zineb, Maneb, Mancozeb, Propineb, Thiophanate, Thiophanate methyl, Benomyl, Iminoctadin acetate, Iminocutadin albecylate, Mepronil, Flutolanil, Pencycuron, Furametpyl, Thifluzamide, Metalaxyl, Oxadixyl, Carpropamid, Dichlofluanid, Flusulfamide, Chlorothalonil, Kresoximmethyl, Fenoxanil, Himexazol, Etridiazol, Fluoroimide, Procymidone, Vinclozolin, Iprodione, Triadimefon, Triflumizole, Bitertanol, Ipconazole, Fluconazole, Propiconazole, Diphenoconazole, Myclobutanil, Tetraconazole, Hexaconazole, Tebuconazole, Imibenconazole, Prochloraz, Pefurazoate, Cyproconazole, Isoprothiolane, Fenarimol, Pyrimetanil, Mepanipyrim, Pyrifenox, Fluazinam, Triforine, Diclomezine, Azoxystrobin, Trifloxystrobin, Orysastrobin, Thiadiazin, Captan, Tiadinil, Probenazole, Acibenzolar-S-methyl (CGA-245704), Fthalide, Tricyclazole, Pyroquilon, Chinomethionat, Oxolinic acid, Dithianon, Cyazofamid, Diclocymet, Kasugamycin, Validamycin, Polyoxin, Blasticidin, Streptomycin, etc.

Similarly, as the herbicides, there can be exemplified herbicides such as Glyphosate, Sulfosate, Glyfosinate, Bialaphos, Butamifos, Esprocarb, Prosulcarb, Benthiocarb, Pyributycarb, Asulam, Linulon, Dymron, Isouron, Bensulfuron methyl, Cyclosulfamuron, Cinosulfuron, Pyrazosulfuron ethyl, Azimsulfuron, Imazosulfuron, Tenylchlor, Alachlor, Pretilachlor, Clomeprop, Etobenzanid, Mefenacet, Flufenacet, Fentrazamide, Pendimethalin, Bifenox, Acifluorfen, Lactfen, Cyhalofop-butyl, Ioxynil, Bromobutide, Alloxydim, Setoxydim, Napropamide, Indanofan, Pyrazolate, Benzofenap, Pyraflufen-ethyl, Imazapyl, Sulfentrazone, Cafenstrole, Bentoxazon, Oxadiazon, Paraquat, Diquat, Pyriminobac, Simazine, Atrazine, Dimethametryn, Triazyflam, Benfuresate, Fluthiacetmethyl, Quizalofop-ethyl, Bentazone, Oxaziclomefone, Azafenidin, Benzobicyclon, calcium peroxide, etc.

As to the biotic pesticides, the same effect as above can be expected by using the agrohorticultural insecticide of the present invention in admixture with, for example, viral formulations obtained from nuclear polyhedrosis virus (NPV), granulosis virus (GV), cytoplasmic polyhedrosis virus (CPV), entomopox virus (EPV), etc.; microbial pesticides utilized as insecticides or nematicides, such as Monacrosporium phymatophagum, Steinernema carpocapsae, Steinernema kushidai, Pasteuria penetrans, etc.; microbial pesticides utilized as fungicides, such as Trichoderma lignorum, Agrobacterium radiobactor, nonpathogenic Erwinia carotovora, Bacillus subtilis, etc.; and biotic pesticides utilized as herbicides, such as Xanthomonas campestris, etc.

In addition, the agrohorticultural insecticide of the present invention can be used in combination with biotic pesticides including natural enemies such as Parasitic wasp (Encarsia formosa), Parasitic wasp (Aphidius colemani), Gall-mildge (Aphidoletes aphidimyza), Parasitic wasp (Diglyphus isaea), Parasitic mite (Dacnusa sibirica), Predatory mite (Phytoseiulus persimilis), Predatory mite (Amblyseius cucumeris), Predatory bug (Orius sauteri), etc.; microbial pesticides such as Beauveria brongniartii, etc.; and pheromones such as (Z)-10-tetradecenyl acetate, (E,Z)-4,10-tetradecadienyl acetate, (Z)-8-dodecenyl acetate, (Z)-11-tetradecenyl acetate, (Z)-13-icosen-10-one, (Z)-13-icosen-10-one, 14-methyl-1-octadecene, etc.

Examples

The present invention will be further described in the following representative examples. However, these examples are not intended to limit the scope of the present invention.

Example 1: Production of (S)-3-iodo-$N^1$-(2-methyl-4-trifluoromethoxyphenyl)-$N^2$-(1-methyl-2-methylthioethyl)phthalamide (Compound No. I-42)

(1-1) Production of t-butyl (S)-(2-hydroxy-1-methylethyl)carbamate

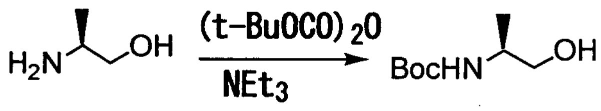


13.8 g of triethylamine was added to 100 ml of a tetrahydrofuran solution containing 10.0 g (133 mmol) of L-alaninol. Thereafter, 30.0 g (137 mmol) of di-t-butyl dicarbonate was added dropwise to the obtained mixture while

cooling in ice water. The temperature of the reaction solution was returned to room temperature, and it was then stirred for 12 hours. Thereafter, the reaction solution was poured into ice water, followed by extraction with ethyl acetate. The organic layer was dried over anhydrous magnesium sulfate and then concentrated under reduced pressure, so as to obtain 24.0 g of t-butyl (S)-(2-hydroxy-1-methylethyl)carbamate.
Yield: Quantitative
Physical properties: [$^1$HNMR, $CDCl_3$, δ (ppm)] 4.62(br, 1H), 3.77(br, 1H), 3.64(br, 1H), 3.49(br, 1H), 2.54(br, 1H), 1.44 (s, 9H), 1.14(d, 3H).

(1-2) Production of (S)-2-(t-butoxycarbonylamino)propyl methanesulfonate

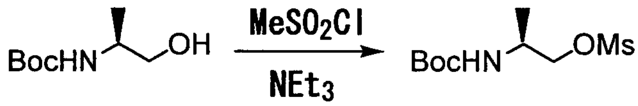


14.5 g (144 mmol) of triethylamine was added to 180 ml of a tetrahydrofuran solution containing 24.0 g (137 mmol) of t-butyl (S)-(2-hydroxy-1-methylethyl)carbamate. Thereafter, 16.4 g (143 mmol) of methanesulfonyl chloride was added dropwise to the obtained mixture while cooling in ice water. The temperature of the reaction solution was returned to room temperature, and it was then stirred for 5 hours. Thereafter, the reaction solution was poured into water, followed by extraction with ethyl acetate. The organic layer was washed with a saturated sodium chloride aqueous solution and then dried over anhydrous magnesium sulfate. The resultant product was then concentrated under reduced pressure, so as to obtain 34.0 g of (S)-2-(t-butoxycarbonylamino)propyl methanesulfonate.
Yield: 98.1%
Physical properties: [$^1$HNMR, $CDCl_3$, δ (ppm)] 4.61(br, 1H), 4.22(dd, 1H), 4.14(dd, 1H), 3.96(br, 1H), 3.03(s, 3H), 1.44 (s, 9H), 1.23(d, 3H).

(1-3) Production of t-butyl (S)-(1-methyl-2-methylthioethyl)carbamate

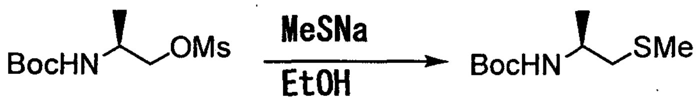


34.0 g (134 mmol) of (S)-2-(t-butoxycarbonylamino)propyl methanesulfonate was added to 100 ml of an ethanol solution containing 73.0 g (156 mmol) of a sodium methyl mercaptan aqueous solution (15%). Thereafter, the obtained mixture was heated to reflux for 5 hours. The temperature of the reaction solution was returned to room temperature, and it was then concentrated under reduced pressure to eliminate ethanol. Thereafter, water was added to the residue, followed by extraction with ethyl acetate. The organic layer was dried over anhydrous magnesium sulfate and then concentrated under reduced pressure, so as to obtain 21.0 g of t-butyl (S)-(1-methyl-2-methylthioethyl)carbamate.
Yield: 76.4%
Physical properties: [$^1$HNMR, $CDCl_3$, δ (ppm)] 4.59(br, 1H), 3.84(br, 1H), 2.65(dd, 1H), 2.54(dd, 1H), 2.14(s, 3H), 1.44 (s, 9H), 1.21(d, 3H).

(1-4) Production of (S)-1-methyl-2-methylthioethylamine

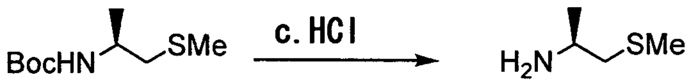


32.0 g of concentrated hydrochloric acid (35%) was added dropwise to 150 ml of an ethyl acetate solution containing 21.0 g (102 mmol) of t-butyl (S)-(1-methyl-2-methylthioethyl)carbamate. The obtained mixture was stirred at room temperature for 3 hours. Thereafter, water was added to the reaction solution, followed by washing with ethyl acetate. The water layer was converted to be basic with addition of sodium hydroxide, and it was then extracted with t-butyl methyl ether. The organic layer was dried over anhydrous magnesium sulfate and then concentrated under reduced pressure, so as to obtain 5.5 g of (S)-1-methyl-2-methylthioethylamine.
Yield: 51.4%
Physical properties: [$^1$HNMR, $CDCl_3$, δ (ppm)] 4.90(br, 2H), 3.07(m, 1H), 2.58(dd, 1H), 2.36(dd, 1H), 2.10(s, 3H), 1.15 (d, 3H).

(1-5) Production of (S)-3-iodo-N-(1-methyl-2-methylthioethyl)phthalamic acid

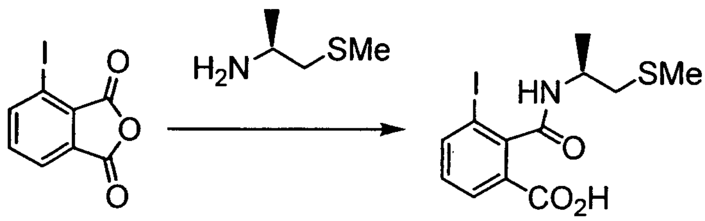

50 ml of an acetonitrile solution containing 5.5 g (52.4 mmol) of (S)-1-methyl-2-methylthioethylamine was added dropwise to 150 ml of an acetonitrile solution containing 14.4 g (52.6 mmol) of 3-iodophthalic anhydride at a slow rate. The obtained mixture was stirred at room temperature for 12 hours. Thereafter, precipitated crystals were filtrated and then washed with acetonitrile, so as to obtain 7.0 g of (S)-3-iodo-N-(1-methyl-2-methylthioethyl)phthalamic acid.
Yield: 35.2%
Physical properties: [$^1$HNMR, $CDCl_3$, δ (ppm)] 8.01(dd, 2H), 7.15(t, 1H), 6.00(d, 1H), 4.43(m, 1H), 2.88(dd, 1H), 2.68 (dd, 1H), 2.17(s, 3H), 1.37(d, 3H).

(1-6) Production of (S)-3-iodo-$N^1$-(2-methyl-4-trifluoromethoxyphenyl)-$N^2$-(1-methyl-2-methylthioethyl)phthalamide (Compound No. 1-42)

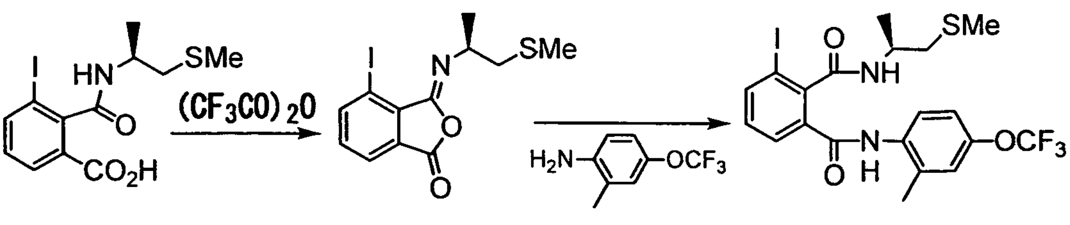


0.67 g (3.2 mmol) of a trifluoroacetic anhydride was added to 8 ml of a t-butyl methyl ether suspension containing 0.8 g (2.1 mmol) of (S)-3-iodo-N-(1-methyl-2-methylthioethyl)phthalamic acid. The obtained mixture was stirred at room temperature for 2 hours. Thereafter, the reaction solution was slowly poured into ice water in which 0.88 g (6.4 mmol) of potassium carbonate had been dissolved, followed by extraction with ethyl acetate. The organic layer was washed

with a saturated sodium chloride aqueous solution and then dried over anhydrous magnesium sulfate. The resultant product was then concentrated under reduced pressure, so as to obtain crude isoimide. The obtained product was then dissolved in 10 ml of acetonitrile. Thereafter, 0.40 g (2.2 mmol) of 2-methyl-4-trifluoromethoxyaniline and 2 drops of trifluoroacetic acid were added to the reaction solution, and the obtained mixture was then stirred at room temperature for 12 hours. Precipitated crystals were filtrated and then washed with acetonitrile, so as to obtain 1.0 g of (S)-3-iodo-$N^1$-(2-methyl-4-trifluoromethoxyphenyl)-$N^2$-(1-methyl-2-methylthioethyl)phthalamide.
Yield: 88.2%
Physical properties: [$^1$HNMR, $CDCl_3$, δ (ppm)] 8.32(br, 1H), 8.13(d, 1H), 7.97(d, 1H), 7.79(d, 1H), 7.22(t, 1H), 7.08(d, 1H), 7.06(s, 1H), 6.20(d, 1H), 4.33(m, 1H), 2.60(m, 2H), 2.33(s, 3H), 1.96(s, 3H), 1.27 (d, 3H).
Specific rotation: $[\alpha]_D^{20}$ = +2.61° (c = 1.04, $CHCl_3$)

Example 2: Production of (S)-3-iodo-$N^1$-(2-methyl-4-trifluoromethoxyphenyl)-$N^2$-(1-methyl-2-methylsulfonylethyl)phthalamide (Compound No. I-44)

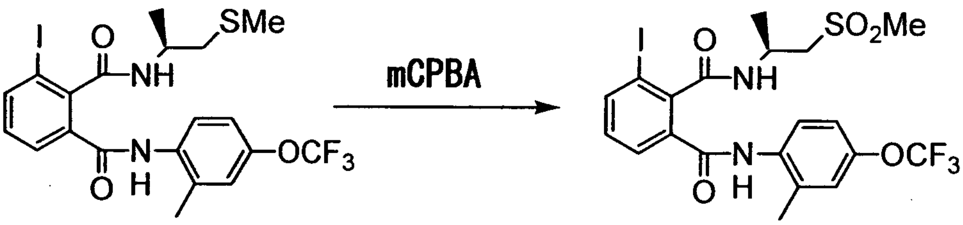


While cooling in ice water, 0.40 g (2.3 mmol) of m-chloroperbenzoic acid was added to 10 ml of a chloroform solution containing 0.38 g (0.70 mmol) of (S)-3-iodo-$N^1$-(2-methyl-4-trifluoromethoxyphenyl)-$N^2$-(1-methyl-2-methylthioethyl)phthalamide. The obtained mixture was stirred at room temperature for 3 hours. Thereafter, a potassium carbonate aqueous solution was added to the reaction solution, followed by extraction with chloroform. The organic layer was washed with a sodium sulfite aqueous solution and then dried over anhydrous magnesium sulfate. The resultant product was then concentrated under reduced pressure. The residue was purified by silica gel column chromatography, so as to obtain 0.26 g of (S)-3-iodo-$N^1$-(2-methyl-4-trifluoromethoxyphenyl)-$N^2$-(1-methyl-2-methylsulfonylethyl)phthalamide.
Yield: 64.9%
Physical properties: [$^1$HNMR, $CDCl_3$, δ (ppm)] 8.07(br, 1H), 7.97(m, 2H), 7.70(d, 1H), 7.21(t, 1H), 7.70(m, 2H), 6.64(d, 1H), 4.61(m, 1H), 3.37(dd, 1H), 3.17(dd, 1H), 2.78(s, 3H), 2.32(s, 3H), 1.51(d, 3H).
Specific rotation: $[\alpha]_D^{20}$ = +3.80° (c = 1.03, $CHCl_3$)

Representative compounds as optically active phthalamide compounds represented by the formula (I) of the present invention, which can be produced in the same manner as in examples, are shown in Tables 1 to 3. However, the present invention is not limited to these compounds. In the tables, the term "physical properties" means a melting point or logP. The symbol "Me" represents a methyl group, "Et" represents an ethyl group, "Pr" represents a propyl group, "-n" represents normal, and "-i" represents iso.

In Table 4, NMR data and specific rotation are shown regarding some compounds, the physical properties of which are not described in Tables 1 to 3.

Formula (I-3)

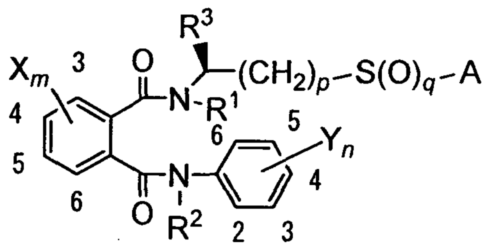

(I - 3)

Table 1 ($R^1$=$R^2$=H, $R^3$=Me, *p*=1)

| No. | X*m* | A | *q* | Y*n* | Physical properties Melting point (°C) |
|---|---|---|---|---|---|
| 1-1 | 3-I | Me | 0 | 4-$CF(CF_3)_2$ | |
| 1-2 | 3-I | Me | 1 | 4-$CF(CF_3)_2$ | |
| 1-3 | 3-I | Me | 2 | 4-$CF(CF_3)_2$ | 138 |
| 1-4 | 3-I | Me | 1 | 2-Me-4-$CF(CF_3)_2$ | 110-129 |
| 1-5 | 3-I | Me | 2 | 2-Me-4-$CF(CF_3)_2$ | |
| 1-6 | 3-I | Et | 0 | 2-Me-4-$CF(CF_3)_2$ | |
| 1-7 | 3-I | Et | 1 | 2-Me-4-$CF(CF_3)_2$ | 103-108 |
| 1-8 | 3-I | Et | 2 | 2-Me-4-$CF(CF_3)_2$ | |
| 1-9 | 3-I | Pr-n | 0 | 2-Me-4-$CF(CF_3)_2$ | |
| 1-10 | 3-I | Pr-n | 1 | 2-Me-4-$CF(CF_3)_2$ | 112-115 |
| 1-11 | 3-I | Pr-n | 2 | 2-Me-4-$CF(CF_3)_2$ | |
| 1-12 | 3-I | Pr-*i* | 0 | 2-Me-4-$CF(CF_3)_2$ | |
| 1-13 | 3-I | Pr-*i* | 1 | 2-Me-4-$CF(CF_3)_2$ | 109-131 |
| 1-14 | 3-I | Pr-*i* | 2 | 2-Me-4-$CF(CF_3)_2$ | |
| 1-15 | 3-I | Me | 0 | 2-Et-4-$CF(CF_3)_2$ | |
| 1-16 | 3-I | Me | 1 | 2-Et-4-$CF(CF_3)_2$ | |
| 1-17 | 3-I | Me | 2 | 2-Et-4-$CF(CF_3)_2$ | |
| 1-18 | 3-I | Me | 0 | 2-OMe-4-$CF(CF_3)_2$ | |
| 1-19 | 3-I | Me | 1 | 2-OMe-4-$CF(CF_3)_2$ | |
| 1-20 | 3-I | Me | 2 | 2-OMe-4-$CF(CF_3)_2$ | |
| 1-21 | 3-I | Me | 0 | 2-Me-4-$CH(CF_3)_2$ | |
| 1-22 | 3-I | Me | 1 | 2-Me-4-$CH(CF_3)_2$ | 87-92 |
| 1-23 | 3-I | Me | 2 | 2-Me-4-$CH(CF_3)_2$ | |
| 1-24 | 3-I | Et | 0 | 2-Me-4-$CH(CF_3)_2$ | |
| 1-25 | 3-I | Et | 1 | 2-Me-4-$CH(CF_3)_2$ | 85-88 |
| 1-26 | 3-I | Et | 2 | 2-Me-4-$CH(CF_3)_2$ | |
| 1-27 | 3-I | Pr-*i* | 0 | 2-Me-4-$CH(CF_3)_2$ | |
| 1-28 | 3-I | Pr-*i* | 1 | 2-Me-4-$CH(CF_3)_2$ | Amorphous |
| 1-29 | 3-I | Pr-*i* | 2 | 2-Me-4-$CH(CF_3)_2$ | |
| 1-30 | 3-I | Me | 0 | 2-Me-4-$CF_2CF_3$ | |
| 1-31 | 3-I | Me | 1 | 2-Me-4-$CF_2CF_3$ | 90-95 |
| 1-32 | 3-I | Me | 2 | 2-Me-4-$CF_2CF_3$ | |
| 1-33 | 3-I | Me | 0 | 2-Me-4-$CF_3$ | |
| 1-34 | 3-I | Me | 1 | 2-Me-4-$CF_3$ | 203-215 |
| 1-35 | 3-I | Me | 2 | 2-Me-4-$CF_3$ | |

(continued)

| No. | X*m* | A | *q* | Y*n* | Physical properties Melting point (°C) |
|---|---|---|---|---|---|
| 1-36 | 3-I | Me | 0 | 2-Me-4-$OCF_2CHFCF_3$ | |
| 1-37 | 3-I | Me | 1 | 2-Me-4-$OCF_2CHFCF_3$ | |
| 1-38 | 3-I | Me | 2 | 2-Me-4-$OCF_2CHFCF_3$ | |
| 1-39 | 3-I | Me | 0 | 2-Me-4-$OCF_2CHF_2$ | |
| 1-40 | 3-I | Me | 1 | 2-Me-4-$OCF_2CHF_2$ | 198-202 |
| 1-41 | 3-I | Me | 2 | 2-Me-4-$OCF_2CHF_2$ | |
| 1-42 | 3-I | Me | 0 | 2-Me-4-$OCF_3$ | |
| 1-43 | 3-I | Me | 1 | 2-Me-4-$OCF_3$ | 197-206 |
| 1-44 | 3-I | Me | 2 | 2-Me-4-$OCF_3$ | |
| 1-45 | 3-I | Et | 0 | 2-Me-4-$OCF_3$ | |
| 1-46 | 3-I | Et | 1 | 2-Me-4-$OCF_3$ | |
| 1-47 | 3-I | Et | 2 | 2-Me-4-$OCF_3$ | |
| 1-48 | 3-I | Me | 0 | 2-Cl-4-$OCF_3$ | |
| 1-49 | 3-I | Me | 1 | 2-Cl-4-$OCF_3$ | 179-180 |
| 1-50 | 3-I | Me | 2 | 2-Cl-4-$OCF_3$ | |
| 1-51 | 3-I | Et | 0 | 2-Cl-4-$OCF_3$ | |
| 1-52 | 3-I | Et | 1 | 2-Cl-4-$OCF_3$ | |
| 1-53 | 3-I | Et | 2 | 2-Cl-4-$OCF_3$ | |
| 1-54 | 3-I | Me | 0 | 2-Br-4-$OCF_3$ | |
| 1-55 | 3-I | Me | 1 | 2-Br-4-$OCF_3$ | |
| 1-56 | 3-I | Me | 2 | 2-Br-4-$OCF_3$ | |
| 1-57 | 3-I | Et | 0 | 2-Br-4-$OCF_3$ | |
| 1-58 | 3-I | Et | 1 | 2-Br-4-$OCF_3$ | |
| 1-59 | 3-I | Et | 2 | 2-Br-4-$OCF_3$ | |
| 1-60 | 3-I | Me | 0 | 2-Cl-3,4-($-CF_2OCF_2O-$) | |
| 1-61 | 3-I | Me | 1 | 2-Cl-3,4-($-CF_2OCF_2O-$) | |
| 1-62 | 3-I | Me | 2 | 2-Cl-3,4-($-CF_2OCF_2O-$) | |
| 1-63 | 3-I | Me | 0 | 2,4-$Cl_2$ | |
| 1-64 | 3-I | Me | 1 | 2,4-$Cl_2$ | 151-159 |
| 1-65 | 3-I | Me | 2 | 2,4-$Cl_2$ | |
| 1-66 | 3-I | Me | 0 | 2,3,4-$Cl_3$ | 203-205 |
| 1-67 | 3-I | Me | 1 | 2,3,4-$Cl_3$ | |
| 1-68 | 3-I | Me | 2 | 2,3,4-$Cl_3$ | 212-213 |
| 1-69 | 3-I | Me | 0 | 2-Cl-4-$CF_3$ | |
| 1-70 | 3-I | Me | 1 | 2-Cl-4-$CF_3$ | |
| 1-71 | 3-I | Me | 2 | 2-Cl-4-$CF_3$ | |
| 1-72 | 3-Br | Me | 0 | 2-Me-4-$CF(CF_3)$ | |
| 1-73 | 3-Br | Me | 1 | 2-Me-4-$CF(CF_3)_2$ | 123-132 |
| 1-74 | 3-Br | Me | 2 | 2-Me-4-$CF(CF_3)_2$ | |
| 1-75 | 3-Cl | Me | 0 | 2-Me-4-$CF(CF_3)_2$ | |
| 1-76 | 3-Cl | Me | 1 | 2-Me-4-$CF(CF_3)_2$ | 74-96 |
| 1-77 | 3-Cl | Me | 2 | 2-Me-4-$CF(CF_3)_2$ | |
| 1-78 | 3-F | Me | 0 | 2-Me-4-$CF(CF_3)_2$ | |
| 1-79 | 3-F | Me | 1 | 2-Me-4-$CF(CF_3)_2$ | 160 |
| 1-80 | 3-F | Me | 2 | 2-Me-4-$CF(CF_3)_2$ | |
| 1-81 | 3-F | Me | 0 | 2-Me-4-$CH(CF_3)_2$ | |
| 1-82 | 3-F | Me | 1 | 2-Me-4-$CH(CF_3)_2$ | 93-103 |
| 1-83 | 3-F | Me | 2 | 2-Me-4-$CH(CF_3)_2$ | |

(continued)

| No. | Xm | A | q | Yn | Physical properties Melting point (°C) |
|---|---|---|---|---|---|
| 1-84 | 3-$NO_2$ | Me | 0 | 2-Me-4-$CF(CF_3)_2$ | |
| 1-85 | 3-$NO_2$ | Me | 1 | 2-Me-4-$CF(CF_3)_2$ | 191-194 |
| 1-86 | 3-$NO_2$ | Me | 2 | 2-Me-4-$CF(CF_3)_2$ | |
| 1-87 | H | Me | 0 | 2-Me-4-$CF(CF_3)_2$ | |
| 1-88 | H | Me | 1 | 2-Me-4-$CF(CF_3)_2$ | 93-104 |
| 1-89 | H | Me | 2 | 2-Me-4-$CF(CF_3)_2$ | |
| 1-90 | 3-$OCF_3$ | Me | 0 | 2-Me-4-$CF(CF_3)_2$ | |
| 1-91 | 3-$OCF_3$ | Me | 1 | 2-Me-4-$CF(CF_3)_2$ | |
| 1-92 | 3-$OCF_3$ | Me | 2 | 2-Me-4-$CF(CF_3)_2$ | |
| 1-93 | 3,4-$Cl_2$ | Me | 0 | 2-Me-4-$CF(CF_3)_2$ | |
| 1-94 | 3,4-$Cl_2$ | Me | 1 | 2-Me-4-$CF(CF_3)_2$ | |
| 1-95 | 3,4-$Cl_2$ | Me | 2 | 2-Me-4-$CF(CF_3)_2$ | |
| 1-96 | 3-I | Et | 0 | 2-Et-4-$CF(CF_3)_2$ | |
| 1-97 | 3-I | Et | 1 | 2-Et-4-$CF(CF_3)_2$ | |
| 1-98 | 3-I | Et | 2 | 2-Et-4-$CF(CF_3)_2$ | |
| 1-99 | 3-I | Me | 0 | 2-Et-4-$CH(CF_3)_2$ | |
| 1-100 | 3-I | Me | 1 | 2-Et-4-$CH(CF_3)_2$ | |
| 1-101 | 3-I | Me | 2 | 2-Et-4-$CH(CF_3)_2$ | |
| 1-102 | 3-I | Et | 0 | 2-Et-4-$CH(CF_3)_2$ | |
| 1-103 | 3-I | Et | 1 | 2-Et-4-$CH(CF_3)_2$ | |
| 1-104 | 3-I | Et | 2 | 2-Et-4-$CH(CF_3)_2$ | |
| 1-105 | 3-I | Et | 0 | 2-Me-3-F-4-$CF(CF_3)_2$ | 4.71 |
| 1-106 | 3-I | Et | 1 | 2-Me-3-F-4-$CF(CF_3)_2$ | 2.97 |
| 1-107 | 3-I | Et | 2 | 2-Me-3-F-4-$CF(CF_3)_2$ | 3.47 |
| 1-108 | 3-I | Et | 0 | 2-Me-3-F-4-$CF_2CF_3$ | 4.36 |
| 1-109 | 3-I | Et | 1 | 2-Me-3-F-4-$CF_2CF_3$ | |
| 1-110 | 3-I | Et | 2 | 2-Me-3-F-4-$CF_2CF_3$ | 3.15 |
| 1-111 | 3-I | Et | 0 | 2-Me-3-Cl-4-$CF_2CF_3$ | 4.53 |
| 1-112 | 3-I | Et | 1 | 2-Me-3-Cl-4-$CF_2CF_3$ | |
| 1-113 | 3-I | Et | 2 | 2-Me-3-Cl-4-$CF_2CF_3$ | 3.28 |
| 1-114 | 3-I | Et | 0 | 2-Br-4-$OCF_3$-5-F | 4.28 |
| 1-115 | 3-I | Et | 1 | 2-Br-4-$OCF_3$-5-F | |
| 1-116 | 3-I | Et | 2 | 2-Br-4-$OCF_3$-5-F | 3.09 |
| 1-117 | 3-I | Et | 0 | 2-Cl-4-$OCF_3$-5-F | 4.26 |
| 1-118 | 3-I | Et | 1 | 2-Cl-4-$OCF_3$-5-F | |
| 1-119 | 3-I | Et | 2 | 2-C1-4-$OCF_3$-5-F | 3.07 |
| 1-120 | 3-I | Et | 0 | 2-Me-3-F-4-Br | 3.65 |
| 1-121 | 3-I | Et | 1 | 2-Me-3-F-4-Br | |
| 1-122 | 3-I | Et | 2 | 2-Me-3-F-4-Br | 2.49 |
| 1-123 | 3-I | Et | 0 | 2-Me-3-F-4-$OCF_3$ | 3.90 |
| 1-124 | 3-I | Et | 1 | 2-Me-3-F-4-$OCF_3$ | |
| 1-125 | 3-I | Et | 2 | 2-Me-3-F-4-$OCF_3$ | 2.77 |
| 1-126 | 3-I | Et | 0 | 2-Me-3-F-4-$CF_3$ | 3.90 |
| 1-127 | 3-I | Et | 1 | 2-Me-3-F-4-$CF_3$ | |
| 1-128 | 3-I | Et | 2 | 2-Me-3-F-4-$CF_3$ | 2.74 |
| 1-129 | 3-I | Et | 0 | 2-Me-3,4-$Br_2$ | 3.89 |
| 1-130 | 3-I | Et | 1 | 2-Me-3,4-$Br_2$ | |
| 1-131 | 3-I | Et | 2 | 2-Me-3,4-$Br_2$ | 2.67 |

(continued)

| No. | Xm | A | q | Yn | Physical properties Melting point (°C) |
|---|---|---|---|---|---|
| 1-132 | 3-I | Et | 0 | 2-Me-3-F-4-I | 3.77 |
| 1-133 | 3-I | Et | 1 | 2-Me-3-F-4-I | |
| 1-134 | 3-I | Et | 2 | 2-Me-3-F-4-I | 2.59 |
| 1-135 | 3-I | Me | 0 | 2-Me-3-F-4-$CF_2CF_3$ | 4.03 |
| 1-136 | 3-I | Me | 1 | 2-Me-3-F-4-$CF_2CF_3$ | 2.50 |
| 1-137 | 3-I | Me | 2 | 2-Me-3-F-4-$CF_2CF_3$ | 2.97 |
| 1-138 | 3-I | Me | 0 | 2-Me-3-F-4-$OCF_3$ | 3.59 |
| 1-139 | 3-I | Me | 1 | 2-Me-3-F-4-$OCF_3$ | |
| 1-140 | 3-I | Me | 2 | 2-Me-3-F-4-$OCF_3$ | 2.60 |
| 1-141 | 3-I | Me | 0 | 2-Me-3-F-4-$CF(CF_3)_2$ | 4.41 |
| 1-142 | 3-I | Me | 1 | 2-Me-3-F-4-$CF(CF_3)_2$ | 2.78 |
| 1-143 | 3-I | Me | 2 | 2-Me-3-F-4-$CF(CF_3)_2$ | 3.27 |
| 1-144 | 3-I | Me | 0 | 2-Me-3-Cl-4-$CF_2CF_3$ | 4.20 |
| 1-145 | 3-I | Me | 1 | 2-Me-3-Cl-4-$CF_2CF_3$ | |
| 1-146 | 3-I | Me | 2 | 2-Me-3-Cl-4-$CF_2CF_3$ | 3.10 |
| 1-147 | 3-I | Me | 0 | 2-Me-3-F-4-Br | 3.30 |
| 1-148 | 3-I | Me | 1 | 2-Me-3-F-4-Br | |
| 1-149 | 3-I | Me | 2 | 2-Me-3-F-4-Br | 2.29 |
| 1-150 | 3-I | Me | 0 | 2-Me-3-F-4-I | 3.42 |
| 1-151 | 3-I | Me | 1 | 2-Me-3-F-4-I | |
| 1-152 | 3-I | Me | 2 | 2-Me-3-F-4-I | 2.40 |
| 1-153 | 3-I | Me | 0 | 2-Me-3,4-$Br_2$ | 3.55 |
| 1-154 | 3-I | Me | 1 | 2-Me-3,4-$Br_2$ | |
| 1-155 | 3-I | Me | 2 | 2-Me-3,4-$Br_2$ | 2.49 |
| 1-156 | 3-I | Me | 0 | 2-Me-3-F-4-$CF_3$ | 3.58 |
| 1-157 | 3-I | Me | 1 | 2-Me-3-F-4-$CF_3$ | |
| 1-158 | 3-I | Me | 2 | 2-Me-3-F-4-$CF_3$ | |
| 1-159 | 3-I | Et | 0 | 2,3-$Cl_2$-4-$OCF_3$ | 4.41 |
| 1-160 | 3-I | Et | 1 | 2,3-$Cl_2$-4-$OCF_3$ | |
| 1-161 | 3-I | Et | 2 | 2,3-$Cl_2$-4-$OCF_3$ | 3.18 |
| 1-162 | 3-I | Et | 0 | 2-Cl-3-Br-4-$OCF_3$ | 4.42 |
| 1-163 | 3-I | Et | 1 | 2-Cl-3-Br-4-$OCF_3$ | |
| 1-164 | 3-I | Et | 2 | 2-Cl-3-Br-4-$OCF_3$ | 3.19 |
| 1-165 | 3-Br | Me | 0 | 2-Me-3-F-4-$CF_2CF_3$ | 3.93 |
| 1-166 | 3-Br | Me | 1 | 2-Me-3-F-4-$CF_2CF_3$ | 2.42 |
| 1-167 | 3-Br | Me | 2 | 2-Me-3-F-4-$CF_2CF_3$ | 2.86 |
| 1-168 | 3-Br | Me | 0 | 2-Me-3-F-4-$CF(CF_3)_2$ | 4.31 |
| 1-169 | 3-Br | Me | 1 | 2-Me-3-F-4-$CF(CF_3)_2$ | 2.72 |
| 1-170 | 3-Br | Me | 2 | 2-Me-3-F-4-$CF(CF_3)_2$ | 3.21 |
| 1-171 | 3-Br | Et | 0 | 2-Me-3-F-4-$CF_2CF_3$ | 4.26 |
| 1-172 | 3-Br | Et | 1 | 2-Me-3-F-4-$CF_2CF_3$ | 2.59 |
| 1-173 | 3-Br | Et | 2 | 2-Me-3-F-4-$CF_2CF_3$ | 3.06 |
| 1-174 | 3-Br | Et | 0 | 2-Me-3-Cl-4-$CF_2CF_3$ | 4.43 |
| 1-175 | 3-Br | Et | 1 | 2-Me-3-Cl-4-$CF_2CF_3$ | |
| 1-176 | 3-Br | Et | 2 | 2-Me-3-Cl-4-$CF_2CF_3$ | 3.19 |
| 1-177 | 3-Br | Et | 0 | 2-Me-3-F-4-Br | 3.54 |
| 1-178 | 3-Br | Et | 1 | 2-Me-3-F-4-Br | |
| 1-179 | 3-Br | Et | 2 | 2-Me-3-F-4-Br | 2.39 |

(continued)

| No. | Xm | A | q | Yn | Physical properties Melting point (°C) |
|---|---|---|---|---|---|
| 1-180 | 3-Br | Et | 0 | 2-Me-3-F-4-$CF_3$ | 3.80 |
| 1-181 | 3-Br | Et | 1 | 2-Me-3-F-4-$CF_3$ | |
| 1-182 | 3-Br | Et | 2 | 2-Me-3-F-4-$CF_3$ | 2.66 |
| 1-183 | 3-Br | Et | 0 | 2-Me-3-F-4-I | 3.66 |
| 1-184 | 3-Br | Et | 1 | 2-Me-3-F-4-I | |
| 1-185 | 3-Br | Et | 2 | 2-Me-3-F-4-I | 2.51 |
| 1-186 | 3-Br | Et | 0 | 2-Me-3,4-$Br_2$ | 3.78 |
| 1-187 | 3-Br | Et | 1 | 2-Me-3,4-$Br_2$ | |
| 1-188 | 3-Br | Et | 2 | 2-Me-3,4-$Br_2$ | 2.59 |
| 1-189 | 3-Br | Et | 0 | 2-Me-3-F-4-$OCF_3$ | 3.81 |
| 1-190 | 3-Br | Et | 1 | 2-Me-3-F-4-$OCF_3$ | |
| 1-191 | 3-Br | Et | 2 | 2-Me-3-F-4-$OCF_3$ | 2.69 |
| 1-192 | 3-Br | Et | 0 | 2-Cl-4-$OCF_3$-5-F | 4.16 |
| 1-193 | 3-Br | Et | 1 | 2-Cl-4-$OCF_3$-5-F | |
| 1-194 | 3-Br | Et | 2 | 2-Cl-4-$OCF_3$-5-F | 2.96 |
| 1-195 | 3-Br | Et | 0 | 2-Br-4-$OCF_3$-5-F | 4.18 |
| 1-196 | 3-Br | Et | 1 | 2-Br-4-$OCF_3$-5-F | |
| 1-197 | 3-Br | Et | 2 | 2-Br-4-$OCF_3$-5-F | 2.99 |
| 1-198 | 3-Br | Et | 0 | 2-Me-3-F-4-$CF(CF_3)_2$ | 4.63 |
| 1-199 | 3-Br | Et | 1 | 2-Me-3-F-4-$CF(CF_3)_2$ | 2.90 |
| 1-200 | 3-Br | Et | 2 | 2-Me-3-F-4-$CF(CF_3)_2$ | 3.39 |
| 1-201 | 3-Cl | Et | 0 | 2-Me-3-F-4-$CF(CF_3)_2$ | 4.54 |
| 1-202 | 3-Cl | Et | 1 | 2-Me-3-F-4-$CF(CF_3)_2$ | 2.86 |
| 1-203 | 3-Cl | Et | 2 | 2-Me-3-F-4-$CF(CF_3)_2$ | 3.34 |
| 1-204 | 3-Cl | Me | 0 | 2-Me-3-F-4-$CF(CF_3)_2$ | 4.23 |
| 1-205 | 3-Cl | Me | 1 | 2-Me-3-F-4-$CF(CF_3)_2$ | 2.69 |
| 1-206 | 3-Cl | Me | 2 | 2-Me-3-F-4-$CF(CF_3)_2$ | |
| 1-207 | 3-Cl | Et | 0 | 2-Me-3-F-4-$CF_2CF_3$ | 4.21 |
| 1-208 | 3-Cl | Et | 1 | 2-Me-3-F-4-$CF_2CF_3$ | 2.56 |
| 1-209 | 3-Cl | Et | 2 | 2-Me-3-F-4-$CF_2CF_3$ | 3.01 |
| 1-210 | 3-Cl | Et | 0 | 2-Me-3-Cl-4-$CF_2CF_3$ | 4.37 |
| 1-211 | 3-C1 | Et | 1 | 2-Me-3-Cl-4-$CF_2CF_3$ | |
| 1-212 | 3-Cl | Et | 2 | 2-Me-3-Cl-4-$CF_2CF_3$ | 3.14 |
| 1-213 | 3-C1 | Me | 0 | 2-Me-3-F-4-$CF_2CF_3$ | 3.88 |
| 1-214 | 3-C1 | Me | 1 | 2-Me-3-F-4-$CF_2CF_3$ | |
| 1-215 | 3-C1 | Me | 2 | 2-Me-3-F-4-$CF_2CF_3$ | 2.82 |
| 1-216 | 3-C1 | Me | 0 | 2-Et-4-$CF(CF_3)_2$ | 4.36 |
| 1-217 | 3-C1 | Me | 1 | 2-Et-4-$CF(CF_3)_2$ | |
| 1-218 | 3-Cl | Me | 2 | 2-Et-4-$CF(CF_3)_2$ | 3.26 |
| 1-219 | 3-Br | Me | 0 | 2-Et-4-$CF(CF_3)_2$ | 4.42 |
| 1-220 | 3-Br | Me | 1 | 2-Et-4-$CF(CF_3)_2$ | |
| 1-221 | 3-Br | Me | 2 | 2-Et-4-$CF(CF_3)_2$ | 3.31 |
| 1-222 | 3-I | Me | 0 | 2-Cl-4-$CF(CF_3)_2$ | |
| 1-223 | 3-I | Me | 1 | 2-Cl-4-$CF(CF_3)_2$ | |
| 1-224 | 3-I | Me | 2 | 2-Cl-4-$CF(CF_3)_2$ | |
| 1-225 | 3-I | Me | 0 | 2-Br-4-$CF(CF_3)_2$ | |
| 1-226 | 3-I | Me | 1 | 2-Br-4-$CF(CF_3)_2$ | |
| 1-227 | 3-I | Me | 2 | 2-Br-4-$CF(CF_3)_2$ | |

(continued)

| No. | X*m* | A | *q* | Y*n* | Physical properties Melting point (°C) |
|---|---|---|---|---|---|
| 1-228 | 3-I | Me | 0 | 2-CN-4-CF$(CF_3)_2$ | |
| 1-229 | 3-I | Me | 1 | 2-CN-4-CF$(CF_3)_2$ | |
| 1-230 | 3-I | Me | 2 | 2-CN-4-CF$(CF_3)_2$ | 196-198 |
| 1-231 | 3-I | Me | 0 | 2-F-4-CF $(CF_3)_2$ | |
| 1-232 | 3-I | Me | 1 | 2-F-4-CF $(CF_3)_2$ | |
| 1-233 | 3-I | Me | 2 | 2-F-4-CF $(CF_3)_2$ | |
| 1-234 | 3-CH=CH-CH=CH-4 | Me | 0 | 2-Me-4-CF$(CF_3)_2$ | |
| 1-235 | 3-CH=CH-CH=CH-4 | Me | 1 | 2-Me-4-CF$(CF_3)_2$ | |
| 1-236 | 3-CH=CH-CH=CH-4 | Me | 2 | 2-Me-4-CF$(CF_3)_2$ | |
| 1-237 | 3-I | Me | 0 | 2-I-4-CF$(CF_3)_2$ | |
| 1-238 | 3-I | Me | 1 | 2-I-4-CF$(CF_3)_2$ | |
| 1-239 | 3-I | Me | 2 | 2-I-4-CF$(CF_3)_2$ | |
| 1-240 | 3-Br | Et | 0 | 2-Me-4-CF$(CF_3)_2$ | |
| 1-241 | 3-Br | Et | 1 | 2-Me-4-CF$(CF_3)_2$ | 133-144 |
| 1-242 | 3-Br | Et | 2 | 2-Me-4-CF$(CF_3)_2$ | |
| 1-243 | 3-Br | Et | 0 | 2-Me-4-CH$(CF_3)_2$ | |
| 1-244 | 3-Br | Et | 1 | 2-Me-4-CH$(CF_3)_2$ | 205-209 |
| 1-245 | 3-Br | Et | 2 | 2-Me-4-CH$(CF_3)_2$ | |
| 1-246 | 3-Br | Me | 0 | 2-Me-4-CH$(CF_3)_2$ | |
| 1-247 | 3-Br | Me | 1 | 2-Me-4-CH$(CF_3)_2$ | 110-122 |
| 1-248 | 3-Br | Me | 2 | 2-Me-4-CH$(CF_3)_2$ | |
| 1-249 | 3-Cl | Me | 0 | 2-Me-4-CH$(CF_3)_2$ | |
| 1-250 | 3-Cl | Me | 1 | 2-Me-4-CH$(CF_3)_2$ | 113-126 |
| 1-251 | 3-Cl | Me | 2 | 2-Me-4-CH$(CF_3)_2$ | |
| 1-252 | 3-Cl | Et | 0 | 2-Me-4-CF$(CF_3)_2$ | |
| 1-253 | 3-Cl | Et | 1 | 2-Me-4-CF$(CF_3)_2$ | 136-143 |
| 1-254 | 3-Cl | Et | 2 | 2-Me-4-CF$(CF_3)_2$ | |
| 1-255 | 3-Cl | Et | 0 | 2-Me-4-CH$(CF_3)_2$ | |
| 1-256 | 3-Cl | Et | 1 | 2-Me-4-CH$(CF_3)_2$ | 121-129 |
| 1-257 | 3-Cl | Et | 2 | 2-Me-4-CH$(CF_3)_2$ | |
| 1-258 | 3-Cl | Me | 0 | 2-Me-4-CF$(CF_3)CF_2CF_3$ | |
| 1-259 | 3-Cl | Me | 1 | 2-Me-4-CF$(CF_3)CF_2CF_3$ | |
| 1-260 | 3-Cl | Me | 2 | 2-Me-4-CF$(CF_3)CF_2CF_3$ | |
| 1-261 | 3-Cl | Me | 0 | 2-Me-4-CH$(CF_3)CF_2CF_3$ | |
| 1-262 | 3-Cl | Me | 1 | 2-Me-4-CH$(CF_3)CF_2CF_3$ | |
| 1-263 | 3-Cl | Me | 2 | 2-Me-4-CH$(CF_3)CF_2CF_3$ | |
| 1-264 | 3-Cl | Me | 0 | 2-Me-3-F-4-CF$(CF_3)_2$ | |
| 1-265 | 3-Cl | Me | 1 | 2-Me-3-F-4-CF$(CF_3)_2$ | |
| 1-266 | 3-Cl | Me | 2 | 2-Me-3-F-4-CF$(CF_3)_2$ | |
| 1-267 | 3-Cl | Me | 0 | 2-Me-4-$OCF_3$ | |
| 1-268 | 3-Cl | Me | 1 | 2-Me-4-$OCF_3$ | |
| 1-269 | 3-Cl | Me | 2 | 2-Me-4-$OCF_3$ | |
| 1-270 | 3-Cl | Me | 0 | 2-Cl-4-$OCF_3$ | |
| 1-271 | 3-Cl | Me | 1 | 2-Cl-4-$OCF_3$ | |
| 1-272 | 3-Cl | Me | 2 | 2-Cl-4-$OCF_3$ | |
| 1-273 | 3-Cl | Me | 0 | 2-Br-4-$OCF_3$ | |
| 1-274 | 3-Cl | Me | 1 | 2-Br-4-$OCF_3$ | |
| 1-275 | 3-Cl | Me | 2 | 2-Br-4-$OCF_3$ | |

(continued)

| No. | X*m* | A | *q* | Y*n* | Physical properties Melting point (°C) |
|---|---|---|---|---|---|
| 1-276 | 3-I | Me | 0 | 2-Me-4-CF($CF_3$)$CF_2CF_3$ | |
| 1-277 | 3-I | Me | 1 | 2-Me-4-CF($CF_3$)$CF_2CF_3$ | |
| 1-278 | 3-I | Me | 2 | 2-Me-4-CF($CF_3$)$CF_2CF_3$ | |
| 1-279 | 3-I | Me | 0 | 2-Me-4-CH($CF_3$)$CF_2CF_3$ | |
| 1-280 | 3-I | Me | 1 | 2-Me-4-CH($CF_3$)$CF_2CF_3$ | |
| 1-281 | 3-I | Me | 2 | 2-Me-4-CH($CF_3$)$CF_2CF_3$ | |
| 1-282 | 3-I | Me | 0 | 2-Me-4-O-(2-(3-Cl-5-$CF_3$) pyridyl) | |
| 1-283 | 3-I | Me | 1 | 2-Me-4-O-(2-(3-Cl-5-CF3) pyridyl) | 201-202 |
| 1-284 | 3-I | Me | 2 | 2-Me-4-O-(2-(3-Cl- | |
| 1-285 | 3-Cl | Me | 0 | 5-$CF_3$)pyridyl) 2-Me-4-O-(2-(3-Cl- | |
| 1-286 | 3-Cl | Me | 1 | 5-$CF_3$)pyridyl) 2-Me-4-O-(2-(3-Cl- | |
| 1-287 | 3-Cl | Me | 2 | 5-$CF_3$)pyridyl) 2-Me-4-O-(2-(3-Cl- | |
| 1-288 | 3-Cl | Me | 0 | 5-$CF_3$)pyridyl) 2-Me-4-$CF_2CF_3$ | |
| 1-289 | 3-Cl | Me | 1 | 2-Me-4-$CF_2CF_3$ | |
| 1-290 | 3-Cl | Me | 2 | 2-Me-4-$CF_2CF_3$ | 140-143 |
| 1-291 | 3-Br | Me | 0 | 2-Me-4-$CF_2CF_3$ | 197 |
| 1-292 | 3-Br | Me | 1 | 2-Me-4-$CF_2CF_3$ | |
| 1-293 | 3-Br | Me | 2 | 2-Me-4-$CF_2CF_3$ | 160-173 |
| 1-294 | 3-F | Me | 0 | 2-Me-4-$OCHF_2$ | 165-167 |
| 1-295 | 3-F | Me | 1 | 2-Me-4-$OCHF_2$ | 174-175 |
| 1-296 | 3-F | Me | 2 | 2-Me-4-$OCHF_2$ | |
| 1-297 | 3-F | Me | 0 | 2-Me-4-$OCF_3$ | 177-179 |
| 1-298 | 3-F | Me | 1 | 2-Me-4-$OCF_3$ | 201-210 |
| 1-299 | 3-F | Me | 2 | 2-Me-4-$OCF_3$ | 204-208 |
| 1-300 | 3-F | Me | 0 | 2-Me-4-F | 188-190 |
| 1-301 | 3-F | Me | 1 | 2-Me-4-F | 212-218 |
| 1-302 | 3-F | Me | 2 | 2-Me-4-F | 201-206 |
| 1-303 | 3-F | Me | 0 | 2-F-3-$CF_3$ | 112-114 |
| 1-304 | 3-F | Me | 1 | 2-F-3-$CF_3$ | 165-172 |
| 1-305 | 3-F | Me | 2 | 2-F-3-$CF_3$ | 166-177 |
| 1-306 | 3-F | Me | 0 | 2-Me-4-$CF_2CF_3$ | 148-158 |
| 1-307 | 3-F | Me | 1 | 2-Me-4-$CF_2CF_3$ | 75-82 |
| 1-308 | 3-F | Me | 2 | 2-Me-4-$CF_2CF_3$ | 121-125 |
| 1-309 | 3-F | Me | 0 | 2,4-$Cl_2$ | 70-77 |
| 1-310 | 3-F | Me | 1 | 2,4-$Cl_2$ | 164 |
| 1-311 | 3-F | Me | 2 | 2,4-$Cl_2$ | 195-199 |
| 1-312 | 3-F | Me | 0 | 2-Me-4-$OCF_2CHF_2$ | 143-160 |
| 1-313 | 3-F | Me | 1 | 2-Me-4-$OCF_2CHF_2$ | 197 |
| 1-314 | 3-F | Me | 2 | 2-Me-4-$OCF_2CHF_2$ | 173-176 |
| 1-315 | 3-F | Me | 0 | 2-Cl-4-$OCF_3$ | 140 |
| 1-316 | 3-F | Me | 1 | 2-Cl-4-$OCF_3$ | 161-162 |
| 1-317 | 3-F | Me | 2 | 2-Cl-4-$OCF_3$ | 164-165 |
| 1-318 | 3-F | Me | 0 | 2-Me-4-Cl | 183-185 |
| 1-319 | 3-F | Me | 1 | 2-Me-4-Cl | 192-196 |

(continued)

| No. | Xm | A | q | Yn | Physical properties Melting point (°C) |
|---|---|---|---|---|---|
| 1-320 | 3-F | Me | 2 | 2-Me-4-Cl | 210-211 |
| 1-321 | 3-F | Me | 0 | 2-Me-4-$OCF_2CHFCF_3$ | 141-142 |
| 1-322 | 3-F | Me | 1 | 2-Me-4-$OCF_2CHFCF_3$ | 166-168 |
| 1-323 | 3-F | Me | 2 | 2-Me-4-$OCF_2CHFCF_3$ | 203-206 |
| 1-324 | 3-F | Me | 0 | 2-Me-4-$OCH_2CF_3$ | 182-184 |
| 1-325 | 3-F | Me | 1 | 2-Me-4-$OCH_2CF_3$ | 191-208 |
| 1-326 | 3-F | Me | 2 | 2-Me-4-$OCH_2CF_3$ | 200-209 |
| 1-327 | 3-$CF_3$ | Me | 0 | 2-Me-4-$CH(CF_3)_2$ | 211 |
| 1-328 | 3-$CF_3$ | Me | 1 | 2-Me-4-$CH(CF_3)_2$ | 208-209 |
| 1-329 | 3-$CF_3$ | Me | 2 | 2-Me-4-$CH(CF_3)_2$ | 238-243 |
| 1-330 | 3-Cl | Me | 0 | 2-Cl-3-$CF(CF_3)CF_2O$-4 | 95-98 |
| 1-331 | 3-Cl | Me | 1 | 2-Cl-3-$CF(CF_3)CF_2O$-4 | 128-132 |
| 1-332 | 3-Cl | Me | 2 | 2-Cl-3-$CF(CF_3)CF_2O$-4 | 95-97 |
| 1-333 | 3-I | Me | 0 | 2-Cl-3-$CF(CF_3)CF_2O$-4 | 156-158 |
| 1-334 | 3-I | Me | 1 | 2-Cl-3-$CF(CF_3)CF_2O$-4 | 117-121 |
| 1-335 | 3-I | Me | 2 | 2-Cl-3-$CF(CF_3)CF_2O$-4 | 158-160 |
| 1-336 | 3-Cl | Me | 0 | 2-Me-4-S-(2-(5-$CF_3$)pyndyl) | 130-132 |
| 1-337 | 3-Cl | Me | 1 | 2-Me-4-S-(2-(5-$CF_3$)pyridyl) | |
| 1-338 | 3-Cl | Me | 2 | 2-Me-4-S-(2-(5-$CF_3$)pyridyl) | |

Formula (I-3)

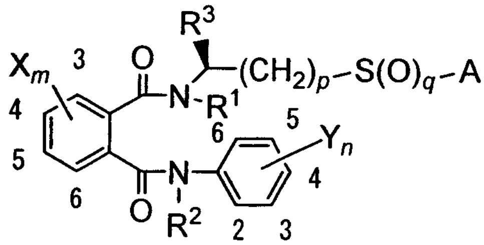

(I - 3)

Table 2 ($R^1$=$R^2$=H, *p*=1)

| No. | Xm | A | $R^3$ | q | Yn | Physical properties logP(pH2.3) |
|---|---|---|---|---|---|---|
| 1-339 | 3-Cl | Me | Et | 0 | 2-Me-4-$CF(CF_3)_2$ | 4.47 |
| 1-340 | 3-Cl | Me | Et | 1 | 2-Me-4-$CF(CF_3)_2$ | 2.78 |
| 1-341 | 3-Cl | Me | Et | 2 | 2-Me-4-$CF(CF_3)_2$ | 3.31 |
| 1-342 | 3-Br | Me | Et | 0 | 2-Me-4-$CF(CF_3)$2 | 4.54 |
| 1-343 | 3-Br | Me | Et | 1 | 2-Me-4-$CF(CF_3)_2$ | 2.83 |
| 1-344 | 3-Br | Me | Et | 2 | 2-Me-4-$CF(CF_3)_2$ | 3.37 |
| 1-345 | 3-I | Me | Et | 0 | 2-Me-4-$CF(CF_3)_2$ | 4.62 |
| 1-346 | 3-I | Me | Et | 1 | 2-Me-4-$CF(CF_3)_2$ | 2.90 |
| 1-347 | 3-I | Me | Et | 2 | 2-Me-4-$CF(CF_3)_2$ | 3.45 |
| 1-348 | 3-Cl | Me | i-Pr | 0 | 2-Me-4-$CF(CF_3)_2$ | 4.77 |
| 1-349 | 3-Cl | Me | i-Pr | 1 | 2-Me-4-$CF(CF_3)_2$ | 3.03 |
| 1-350 | 3-Cl | Me | i-Pr | 2 | 2-Me-4-$CF(CF_3)_2$ | 3.54 |
| 1-351 | 3-Br | Me | i-Pr | 0 | 2-Me-4-$CF(CF_3)_2$ | 4.80 |
| 1-352 | 3-Br | Me | i-Pr | 1 | 2-Me-4-$CF(CF_3)_2$ | 3.06 |
| 1-353 | 3-Br | Me | i-Pr | 2 | 2-Me-4-$CF(CF_3)_2$ | 3.62 |

(continued)

| No. | Xm | A | $R^3$ | q | Yn | Physical properties logP(pH2.3) |
|---|---|---|---|---|---|---|
| 1-354 | 3-I | Me | i-Pr | 0 | 2-Me-4-$CF(CF_3)_2$ | 4.97 |
| 1-355 | 3-I | Me | i-Pr | 1 | 2-Me-4-$CF(CF_3)_2$ | 3.13 |
| 1-356 | 3-I | Me | i-Pr | 2 | 2-Me-4-$CF(CF_3)_2$ | 3.70 |
| 1-357 | 3-Cl | Me | i-Bu | 0 | 2-Me-4-$CF(CF_3)_2$ | 5.11 |
| 1-358 | 3-C1 | Me | i-Bu | 1 | 2-Me-4-$CF(CF_3)_2$ | 3.27 |
| 1-359 | 3-C1 | Me | i-Bu | 2 | 2-Me-4-$CF(CF_3)_2$ | 3.77 |
| 1-360 | 3-Br | Me | i-Bu | 0 | 2-Me-4-$CF(CF_3)_2$ | 5.16 |
| 1-361 | 3-Br | Me | i-Bu | 1 | 2-Me-4-$CF(CF_3)_2$ | 3.30 |
| 1-362 | 3-Br | Me | i-Bu | 2 | 2-Me-4-$CF(CF_3)_2$ | 3.82 |
| 1-363 | 3-I | Me | i-Bu | 0 | 2-Me-4-$CF(CF_3)_2$ | 5.24 |
| 1-364 | 3-I | Me | i-Bu | 1 | 2-Me-4-$CF(CF_3)_2$ | 3.39 |
| 1-365 | 3-I | Me | i-Bu | 2 | 2-Me-4-$CF(CF_3)_2$ | 3.87 |

Formula (I-4)

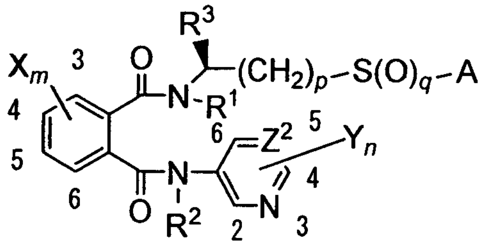

(I - 4)

Table 3 ($R^1$=$R^2$=H, $R^3$=Me, *p*=1)

| No. | X*m* | A | q | Y*n* | $Z^2$ | Physical properties Melting point (°C) |
|---|---|---|---|---|---|---|
| 2-1 | 3-I | Me | 0 | 4-$CF(CF_3)_2$ | CH | |
| 2-2 | 3-I | Me | 1 | 4-$CF(CF_3)_2$ | CH | |
| 2-3 | 3-I | Me | 2 | 4-$CF(CF_3)_2$ | CH | |
| 2-4 | 3-I | Me | 0 | 2-Me-4-$CF(CF_3)_2$ | CH | |
| 2-5 | 3-I | Me | 1 | 2-Me-4-$CF(CF_3)_2$ | CH | 91-96 |
| 2-6 | 3-I | Me | 2 | 2-Me-4-$CF(CF_3)_2$ | CH | |
| 2-7 | 3-I | Me | 0 | 2-Cl-4-$CF(CF_3)_2$ | CH | |
| 2-8 | 3-I | Me | 1 | 2-Cl-4-$CF(CF_3)_2$ | CH | |
| 2-9 | 3-I | Me | 2 | 2-Cl-4-$CF(CF_3)_2$ | CH | |
| 2-10 | 3-I | Me | 0 | 2-Me-4-$CH(CF_3)_2$ | CH | |
| 2-11 | 3-I | Me | 1 | 2-Me-4-$CH(CF_3)_2$ | CH | |
| 2-12 | 3-I | Me | 2 | 2-Me-4-$CH(CF_3)_2$ | CH | |
| 2-13 | 3-Cl | Me | 0 | 2-Me-4-$CF(CF_3)_2$ | CH | |
| 2-14 | 3-Cl | Me | 1 | 2-Me-4-$CF(CF_3)_2$ | CH | |
| 2-15 | 3-Cl | Me | 2 | 2-Me-4-$CF(CF_3)_2$ | CH | |
| 2-16 | 3-F | Me | 0 | 2-Me-4-$CF(CF_3)_2$ | CH | 186 |
| 2-17 | 3-F | Me | 1 | 2-Me-4-$CF(CF_3)_2$ | CH | 99-104 |
| 2-18 | 3-F | Me | 2 | 2-Me-4-$CF(CF_3)_2$ | CH | 96-105 |
| 2-19 | 3-F | Me | 0 | 2-Me-4-$OCH(CF_3)_2$ | CH | 203-204 |
| 2-20 | 3-F | Me | 1 | 2-Me-4-$OCH(CF_3)_2$ | CH | 90-97 |
| 2-21 | 3-F | Me | 2 | 2-Me-4-$OCH(CF_3)_2$ | CH | 172-175 |

(continued)

| No. | Xm | A | q | Yn | $Z^2$ | Physical properties Melting point (°C) |
|---|---|---|---|---|---|---|
| 2-22 | 3-F | Me | 0 | 2,4-$Cl_2$ | CH | 115-122 |
| 2-23 | 3-F | Me | 1 | 2,4-$Cl_2$ | CH | |
| 2-24 | 3-F | Me | 2 | 2,4-$Cl_2$ | CH | |
| 2-25 | 3-F | Me | 0 | 2-Me-4-$CH(CF_3)_2$ | CH | 138-148 |
| 2-26 | 3-F | Me | 1 | 2-Me-4-$CH(CF_3)_2$ | CH | |
| 2-27 | 3-F | Me | 2 | 2-Me-4-$CH(CF_3)_2$ | CH | 77 |
| 2-28 | 3-Cl | Me | 0 | 2-Me-4-$CH(CF_3)_2$ | CH | 196-197 |
| 2-29 | 3-Cl | Me | 1 | 2-Me-4-$CH(CF_3)_2$ | CH | |
| 2-30 | 3-Cl | Me | 2 | 2-Me-4-$CH(CF_3)_2$ | CH | 109-119 |
| 2-31 | 3-Br | Me | 0 | 2-Me-4-$CH(CF_3)_2$ | CH | |
| 2-32 | 3-Br | Me | 1 | 2-Me-4-$CH(CF_3)_2$ | CH | |
| 2-33 | 3-Br | Me | 2 | 2-Me-4-$CH(CF_3)_2$ | CH | |
| 2-34 | 3-Cl | Me | 0 | 2-Me-4-$CF_3$ | CH | 2.76 |
| 2-35 | 3-Cl | Me | 1 | 2-Me-4-$CF_3$ | CH | |
| 2-36 | 3-Cl | Me | 2 | 2-Me-4-$CF_3$ | CH | |
| 2-37 | 3-Cl | Me | 0 | 2-Me-4-$CF_2CF_3$ | CH | 3.33 |
| 2-38 | 3-Cl | Me | 1 | 2-Me-4-$CF_2CF_3$ | CH | |
| 2-39 | 3-Cl | Me | 2 | 2-Me-4-$CF_2CF_3$ | CH | |
| 2-40 | 3-Cl | Me | 0 | 2-Me-4-$CF_3$ | N | 2.55 |
| 2-41 | 3-Cl | Me | 1 | 2-Me-4-$CF_3$ | N | |
| 2-42 | 3-Cl | Me | 2 | 2-Me-4-$CF_3$ | N | |
| 2-43 | 3-Br | Me | 0 | 2-Me-4-$CF_3$ | N | 2.60 |
| 2-44 | 3-Br | Me | 1 | 2-Me-4-$CF_3$ | N | |
| 2-45 | 3-Br | Me | 2 | 2-Me-4-$CF_3$ | N | |
| 2-46 | 3-I | Me | 0 | 2-Me-4-$CF_3$ | N | 2.70 |
| 2-47 | 3-I | Me | 1 | 2-Me-4-$CF_3$ | N | |
| 2-48 | 3-I | Me | 2 | 2-Me-4-$CF_3$ | N | |

Table 4

| No. | NMR data $^1H$-NMR [$CDCl_3$/TMS, $\delta$ (ppm)] | Specific rotation $[\alpha]_D^{20}(CHCl_3)$ |
|---|---|---|
| 1-3 | | +32.50° (c=1.06) |
| 1-5 | 8.26(d,1H),8.13(br,1H),8.00(d,1H),7.74(d,1H), 7.47(d,1H),7.45(s,1H), 7.24(t,1H),6.59(d,1H), 4.62(m,1H),3.35(dd,1H),3.17(dd,1H),2.73(s,3H), 2.38(s,3H),1.52(d,3H) | -0.39° (c=1.03) |
| 1-6 | 8.48 (br, 1H), 8.40 (d, 1H), 8.00 (dd, 1H), 7.82 (dd, 1H), 7.46 (d, 1H), 7.42 (s, 1H), 7.24 (t, 1H), 6.13 (d, 1H), 4.32 (m, 1H), 2.64 (dd, 1H), 2.56 (dd, 1H), 2.39 (s, 3H), 2.34 (m, 2H), 1.26 (d, 3H), 1.08 (t, 3H) | -4.26° (c=0.99) |
| 1-8 | 8.26 (d, 1H), 8.18 (br, 1H), 7.99 (d, 1H), 7.74 (d, 1H), 7.46 (d, 1H), 7.44 (s, 1H), 7.23 (t, 1H), 6.64 (d, 1H), 4.63 (m, 1H), 3.30 (dd, 1H), 3.08 (dd, 1H), 2.82 (m, 2H), 2.38 (s, 3H), 1.52 (d, 3H), 1.22 (t, 3H) | -4.48° (c=0.46) |
| 1-9 | 8.47 (br, 1H), 8.40 (d, 1H), 7.99 (d, 1H), 7.83 (d, 1H), 7.44(d,1H),7.41(s, 1H),7.24(t,1H),6.15(d,1H), 4.31(m,1H),2.59(m,2H),2.39(s,3H),2.31(m, 2H), 2.44(m,2H),1.25(d,3H),0.87(t,3H) | -2.25° (c=1.01) |
| 1-11 | 8.28(br,1H),8.20(d,1H),7.97(d,1H),7.71(d,1H), 7.44(d,1H),7.43(s,1H), 7.22(t,1H),6.72(d,1H), 4.61(m,1H),3.31(dd,1H),3.09(dd,1H),2.84(m,2H), 2.37(s,3H),1.73(m,2H),1.50(d,3H),0.96(t,3H) | +3.70° (c=1.04) |
| 1-12 | 8.48(br,1H),8.41(d,1H),8,00(d,1H),7.83(d,1H), 7-45(d,1H),7.41(s,1H), 7.24(t,1H),6.13(d,1H), 4.32(m,1H),2.74(m,1H),2.60(m,2H),2.39(s,3H), 1.25(d,3H),1.10(dd,6H) | -12.26° (c=1.28) |

(continued)

| No. | NMR data $^1$H-NMR [$CDCl_3$/TMS, δ (ppm)] | Specific rotation $[\alpha]_D^{20}$($CHCl_3$) |
|---|---|---|
| 1-14 | 8.29(d,1H),8.23(br,1H),7.99(d,1H),7.75(d,1H), 7.45(d,1H),7.43(s,1H), 7.24(t,1H),6.66(d,1H), 4.68(m,1H),3.27(dd,1H),3.03(dd,1H),2.93(m,1H), 2.38(s,3H),1.53(d,3H),1.22(dd,6H) | -9.68° (c=1.03) |
| 1-15 | 8.48(br,1H),8.32(d,1H),7.99(d,1H),7.80(d,1H), 7.45(d,1H),7.44(s,1H), 7.23(t,1H),6.16(d,1H), 4.33(m,1H),2.71(q,2H),2.58(dd,2H),1.89(s,3H), 1.27(t,3H),1.26(d,3H) | -5.75° (c=1.01) |
| 1-17 | 8.23(br,1H),8.16(d,1H),7.97(d,1H),7.69(d,1H), 7.46(s,1H),7.45(d,1H), 7.21(t,1H),6.72(d,1H), 4.59(m,1H),3.36(dd,1H),3.16(dd,1H),2.72(s,3H), 2.70(q,2H),1.50(d,3H),1.26(t,3H) | +4.39° (c=1.06) |
| 1-18 | 8.77(br,1H),8.58(d,1H),7.98(d,1H),7.71(d,1H), 7.20-7.26(m,2H),7.08(s, 1H),6.05(d,1H), 4.32(m,1H),3.94(s,3H),2.64(dd,1H),2.55(dd,1H), 1.95(s, 3H),1.24(d,3H) | -3.31° (c=1.07) |
| 1-20 | 8.52-8.55(m,2H),8.00(d,1H),7.67(d,1H), 7-20-7.27(m,2H),7.10(s,1H), 6.43(d,1H), 4.62(m,1H),3.95(s,3H),3.45(dd,1H),3.21(dd,1H), 2.87(s,3H), 1.53(d,3H) | -3.06° (c=0.93) |
| 1-21 | 8.38(br,1H),8.30(d,1H),8.00(d,1H),7.81(d,1H), 7-21-7.29(m,3H),6.09(d, 1H),4.33(m,1H), 3.99(m,1H),2.58(m,2H),2.36(s,3H),1.89(s,3H), 1.25(d, 3H) | -2.45° (c=1.03) |
| 1-23 | 8.17(d,1H),8.03(br,1H),8.00(d,1H),7.73(d,1H), 7.25-7.30(m,2H),7.24(t, 1H),6.53(d,1H), 4.62(m,1H),4.00(m,1H),3.34(dd,1H).3.16(dd,1H), 2.70 (s,3H),2.35(s,3H),1.52(d,3H) | -3.48° (c=1.06) |
| 1-24 | 8.39(br,1H),8.28(d,1H),7.98(d,1H),7.81(d,1H), 7.20-7.28(m,3H),6.15(d, 1H),4.31(m,1H), 3.99(m,1H),2.60(m,2H),2.28-2.35(m,5H), 1.25(d,3H), 1.08(t,3H) | -4.35° (c=0.99) |
| 1-26 | 8.12-8.15(m,2H),7.97(d,1H),7.72(d,1H), 7-25-7.28(m,2H),7.22(t,1H), 7.63(d,1H), 4.62 (m, 1H), 4.00 (m, 1H), 3.29 (dd, 1H), 3.07 (dd, 1H), 2.79 (m,2H),2.34(s,3H),1.52(d,3H),1.20(t,3H) | -4.86° (c=1.05) |
| 1-27 | 8.42(br,1H),8.29(d,1H),7.98(d,1H),7.81(d,1H), 7.19-7.28(m,3H),6.16(d, 1H),4.32(m,1H), 3.99(m,1H),2.65-2.79(m,2H),2.53(dd,1H), 2.35(s,3H), 1.25(d,3H),1.10(dd,6H) | -12.09° (c=1.12) |
| 1-29 | 8.19(br,1H),8.15(d,1H),7.97(d,1H),7.73(d,1H), 7.20-7.26(m,3H),6.66(d, 1H),4.67(m,1H), 4.00(m,1H),3.27(dd,1H),3.02(dd,1H),2.90(m,1H), 2.34 (s,3H),1.54(d,3H),1.20(t,6H) | -10.45° (c=1.14) |
| 1-30 | 8.46(br,1H),8.41(d,1H),8.20(d,1H),8.00(d,1H), 7-46(d,1H),7.41(s,1H), 7.24(t,1H),6.12(d,1H), 4.32(m,1H),2.57(m,2H),2.39(s,3H),1.91(s,3H), 1.25(d,3H) | |
| 1-32 | 8.25(d,1H),8.18(br,1H),7.99(d,1H),7.73(d,1H), 7.45(d,1H),7.44(s,1H), 7.23(t,1H),6.65(d,1H), 4.61(m,1H),3.35(dd,1H),3.16(dd,1H),2.76(s,3H), 2.37(s,3H),1.50(d,3H), | +2.97° (c=1.01) |
| 1-33 | 8.37-8.45 (m, 2H), 8.01 (d, 1H), 7.84 (d, 1H), 7.45-7.52 (m, 2H), 7.26 (t, 1H), 6.12 (d, 1H), 4.34 (m, 1H), 2.59 (dd, 2H), 2.39 (s, 3H), 1.95 (s, 3H), 1.26(d,3H) | +4.99° (c=1.02) |
| 1-35 | 8.22 (d, 1H), 8.14 (br, 1H), 7.99 (d, 1H), 7.73 (d, 1H), 7.48 (d, 1H), 7.47 (s, 1H), 7.23 (t, 1H), 6.62 (d, 1H), 4.61 (m, 1H), 3.35 (dd, 1H), 3.16 (dd, 1H), 2.79 (s, 3H), 2.36(s,3H),1.50(d,3H) | +27.26° (c=0.96) |
| 1-36 | 8.32(br,1H),8.17(d,1H),7.99(d,1H),7.83(d,1H), 7.24(t,1H),7.08(d,1H), 7.06(s,1H),6.12(d,1H), 4.84-5.15(m,1H),4.35(m,1H),2.61(m,2H), 2.34(s, 3H), 1.97(s,3H),1.28(d,3H) | +1.95° (c=1.09) |
| 1-38 | 8.12(br,1H),7.91-7.98(m,2H),7.69(d,1H), 7.20(t,1H),7.06(s,1H),7.05(d, 1H),6.72(d,1H), 4.88-5.09(m,1H),4.60(m,1H),3.36(dd,1H), 3.16(dd,1H), 2.76(s,3H),2.31(s,3H),1.51(d,3H) | -0.16° (c=1.02) |

(continued)

| No. | NMR data $^1$H-NMR [$CDCl_3$/TMS, δ (ppm)] | Specific rotation $[\alpha]_D^{20}$($CHCl_3$) |
|---|---|---|
| 1-39 | 8.30(br,1H),8.15(d,1H),7.98(d,1H),7.81(d,1H), 7.23(t,1H),7.07(d,1H), 7.06(s,1H),6.11(d,1H), 5.90(tt,1H),4,34(m,1H),2.60(br,2H),2.33(s,3H), 1.97(br,3H),1.27(d,3H) | +2.25° (c=1.04) |
| 1-41 | 8.14(br,1H),7.88-7.99(m,2H),7.68(d,1H), 7-19(t,1H),7.08(s,1H),7.06(d, 1H),6.78(d,1H), 5.90(tt,1H),4.60(m,1H),3.36(dd,1H),3.16(dd,1H), 2.76(s, 3H),2.31(s,3H),1.51(d,3H) | |
| 1-42 | 8.32 (br, 1H), 8.13 (d, 1H), 7.97 (d, 1H), 7.79 (d, 1H), 7.22 (t, 1H), 7.08 (d, 1H), 7.06 (s, 1H), 6.20 (d, 1H), 4.33 (m, 1H), 2.60 (m, 2H), 2.33 (s, 3H), 1.96 (s, 3H), 1.27(d,3H) | +2.61° (c=1.04) |
| 1-44 | 8.07 (br, 1H), 7.97 (m, 2H), 7.70 (d, 1H), 7.21 (t, 1H), 7.70 (m, 2H), 6.64 (d, 1H), 4.61 (m, 1H), 3.37 (dd, 1H), 3.17 (dd, 1H), 2.78 (s, 3H), 2.32 (s, 3H), 1.51 (d, 3H) | +3.80° (c=103) |
| 1-45 | 8.36 (br, 1H), 8.12 (d, 1H), 7.96 (d, 1H), 7.78 (d, 1H), 7.21 (t, 1H), 7.05-7.08 (m, 2H), 6.24 (d, 1H), 4.31 (m, 1H), 2.62 (m, 2H), 2.40 (m, 2H), 2.32 (s, 1H), 1.25(d,3H),1.12(t,3H) | +0.26° (c=1.23) |
| 1-47 | 7.96-8.03(m,3H),7.73(d,1H),7.23(t,1H), 7.06-7.10(m,2H),6.58(d,1H), 4.63(m,1H), 3.31(dd,1H),3.09(dd,1H),2.86(q,2H),2.32(s,3H), 1.53(d,3H), 1.25(t,3H) | |
| 1-48 | 8.64 (br, 1H), 8.51 (d, 1H), 8.00 (d, 1H), 7.76 (d, 1H), 7.16-7.31 (m, 3H), 6.06 (d, 1H),4.36 (m, 1H), 2.63(m,2H), 2.00(s,3H),1.28(d,3H) | |
| 1-50 | 8.46(d,1H),8.37(br,1H),8.02(d,1H),7.71(d,1H), 7.33(s,1H),7.24(t,1H), 7.20(d,1H),6.45(d,1H), 4.62(m,1H),3.49(dd,1H),3.24(dd,1H),2.95(s,3H), 1.55(d,3H) | |
| 1-51 | 8.64(br,1H),8.50(d,1H),8.00(d,1H),7.75(d,1H), 7.30(s,1H),7.22(t,1H), 7.18(d,1H),6.07(d,1H), 4.34(m,1H),2.66(m,2H),2.44(m,2H),1.27(d,3H), 1.15(t,3H) | -5.01° (c=1.03) |
| 1-53 | 8.47(d,1H),8.39(br,1H),8.01(d,1H),7.71(d,1H), 7.32(s,1H),7.24(t,1H), 7.20(d,1H),6.47(d,1H), 4.63(m,1H),3.44(dd,1H),3.16(dd,1H),3.02(q,2H), 1.55(d,3H),1.33(t,3H) | +7.35° (c=0.79) |
| 1-54 | 8.58(br,1H),8.46(d,1H),8.00(d,1H),7.74(d,1H), 7.46(s,1H),7.19-7.26(m, 2H),6.07(d,1H), 4.36(m,1H),2.64(m,2H), 2.00(s,3H),1.29(d,3H) | -3.08° (c=1.10) |
| 1-56 | 8.43(d,1H),8.33(br,1H),8.02(d,1H),7.71(d,1H), 7.48(s,1H),7.21-7.27(m, 2H),6.42(d,1H), 4.62(m,1H),3.50(dd,1H),3.25(dd,1H),2.95(s,3H), 1.55(d, 3H) | +10.11° (c=1.07) |
| 1-57 | 8.57 (br, 1H), 8.45 (d, 1H), 8.00 (d, 1H), 7.74 (d, 1H), 7.46 (s, 1H), 7.19-7.26 (m, 2H), 6.08 (d, 1H), 4-35 (m, 1H), 2.44-2.75 (m, 4H), 1.27 (d, 3H), 1.15 (t,3H) | -4.51° (c=1.02) |
| 1-59 | 8.43(d,1H),8.34(br,1H),8.02(d,1H),7.71(d,1H), 7.48(s,1H),7.21-7.27(m, 2H),6.45(d,1H), 4.63(m,1H),3.45(dd,1H),3.17(dd,1H),3.03(m,2H), 1.56 (d,3H),1.33(t,3H) | |
| 1-60 | 8.76(br,1H),8.68(d,1H),8.02(d,1H),7.78(d,1H), 7.25(t,1H),7.12(d,1H), 6.01(d,1H),4.38(m,1H), 2.64(dd,1H),2.01(s,3H),1.31(d,3H) | -8.47° (c=1.00) |
| 1-62 | 8.62(d,1H),8.45(br,1H),8.03(d,1H),7.73(d,1H), 7.26(t,1H),7.14(d,1H), 6.41(d,1H),4.64(m,1H), 3.49(dd,1H),3.25(dd,1H),2.97(s,3H),1.56(d,3H) | |
| 1-63 | 8.64(br,1H),8.51(d,1H),8.00(d,1H),7.76(d,1H), 7.16-7.31(m,3H),6.06(d, 1H),4.36(m,1H), 2.63(m,2H), 2.00(s,3H),1.28(d,3H) | -0.19° (c=1.24) |
| 1-65 | 8.37(d,1H),8.34(br,1H),8.01(dd,1H),7.70(d,1H), 7.43(d,1H),7.29(dd,1H), 7.24(t,1H),6.44(d,1H), 4.61(m,1H),3.49(dd,1H),3.23(dd,1H),2.95(s,3H), 1.54(d,3H) | +9.91° (c=1.06) |
| 1-66 | | -4.37° (c=1.01) |
| 1-72 | 8.53(br,1H),8.38(d,1H),7.81(d,1H),7.75(d,1H), 7.38-7.48(m,3H),6.12(d, 1H),4.34(m,1H), 2.57(dd,2H),2.39(s,3H),1.89(s,3H),1.25(d,3H) | +0.20° (c=1.04) |

(continued)

| No. | NMR data $^1$H-NMR [$CDCl_3$/TMS, δ (ppm)] | Specific rotation $[\alpha]_D^{20}$($CHCl_3$) |
|---|---|---|
| 1-74 | 8.25(d,1H),8.17(br,1H),7.71-7.77(m,2H), 7.43-7.49(m,2H),7.40(t,1H), 6.60(d,1H), 4.63(m,1H),3.35(dd,1H),3.17(dd,1H),2.74(s,3H), 2.38(s,3H), 1.51(d,3H) | -0.36° (c=1.20) |
| 1-75 | 8.55(br,1H),8.36(d,1H),7.78(d,1H),7.57(d,1H), 7.41-7.51(m,3H),6.17(d, 1H),4.35(m,1H), 2.57(dd,2H),2.39(s,3H),1.90(s,3H),1.25(d,3H) | +13.42° (c=0.23) |
| 1-77 | 8.23(d,1H),8.19(br,1H),7.67(d,1H),7.57(d,1H), 7.44-7.50(m,3H),6.66(d, 1H),4.64(m,1H), 3.35(dd,1H),3.17(dd,1H),2.75(s,3H),2.38(s,3H), 1.50(d, 3H) | -0.61° (c=1.02) |
| 1-78 | 8.73(br,1H),8.28(d,1H),7.67(d,1H), 7.42-7.56(m,3H),7.28(t,1H),6.25(d, 1H), 4.38(m,1H),2.62(m,2H),2.40(s,3H),1.97(s,3H), 1.28(d,3H) | +8.70° (c=1.18) |
| 1-80 | 8.28(br,1H),8.13(d,1H),7.43-7.52(m,4H), 7-22-7.29(m,1H),6.80(d,1H), 4.61(m,1H), 3.35(dd,1H),3.19(dd,1H),2.74(s,3H),2.37(s,3H), 1.49(d,3H) | -2.70° (c=1.00) |
| 1-81 | 8.66(br,1H),8.16(d,1H),7.65(d,1H),7.50(m,1H), 7.23-7.30(m,3H),6.27(d, 1H),4.37(m,1H), 4.00(m,1H), 2.62(m,2H),2.36(s,3H),1.97(s,3H), 1,27 (d, 3H) | +9.33° (c=1.01) |
| 1-83 | 8.11(br,1H),8.06(d,1H),7.47-7.55(m,2H), 7.24-7.30(m,3H),6.69(d,1H), 4.63(m,1H), 4.01(m,1H),3.35(dd,1H),3.21(dd,1H),2.73(s,3H), 2.35(s, 3H),2.51(d,3H) | -4.64° (c=1.07) |
| 1-84 | 9.04(br,1H),8.18(d,1H),8.12(d,1H),7.98(d,1H), 7.56(t,1H),7.25-7.33(m, 2H),4.10(m,1H), 2.38(m,2H), 2.28(s,3H),1.67(s,3H),1.07(d,3H) | |
| 1-86 | 8.35(br,1H),8.25-8.31(m,2H),8.08(d,1H), 8.72(t,1H),8.48(d,1H),7.45(s, 1H),6.92(d,1H), 4.60(m,1H),3.28(m,2H),2.79(s,3H),2.38(s,3H), 1.44(d, 3H) | +17.48° (c=1.03) |
| 1-87 | 9.03(br,1H),8.32(d,1H),7.90(m,1H), 7.42-7.60(m,5H),6.25(d,1H),4.35(m, 1H), 2.64(m,2H),2.41(s,3H),2.03(s,3H),1.27(d,3H) | +17.09° (c=0.81) |
| 1-89 | 8.50(br,1H),8.21(d,1H),7.75(m,1H), 7.44-7.62(m,5H),6.84(d,1H),4.62(m, 1H), 3.26(m,2H), 2.81(s,3H),2.39(s,3H),1.47(d,3H) | |
| 1-90 | 8.67(br,1H),8.32(d,1H),7.82(d,1H),7.58(t,1H), 7.42-7.50(m,3H),6.17(d, 1H),4.37(m,1H), 2.57(br,2H),2.40(s,3H),1.90(s,3H),1.26(d,3H) | +2.13° (c=1.02) |
| 1-92 | 8.22(d,1H),8.16(br,1H),7.73(d,1H),7.60(t,1H), 7-45-7.51(m,3H),6.62(d, 1H),4.66(m,1H), 3.33(dd,1H),3.18(dd,1H),2.74(s,3H),2.39(s,3H), 1.51(d, 3H) | |
| 1-93 | 8.86(br,1H),8.01(d,1H),7.38-7.50(m,4H), 7.04(d,1H),4.18(m,1H),2.54(m, 2H),2.33(s,3H), 1.99(s,3H),1.16(d,3H) | |
| 1-95 | 10.17(br,1H),8.68(d,1H),7.87(d,1H),7.84(d,1H), 7.67(d,1H),7.53(d,1H), 7.52(s,1H),4.44(m,1H), 3.25-3.38(m,2H),2.97(s,3H),2.37(s,3H), 1.23(d, 3H) | |
| 1-96 | 8.48(br,1H),8.32(d,1H),7.99(d,1H),7.82(d,1H), 7.46(d,1H),7.44(s,1H), 7.24(t,1H),6.15(d,1H), 4.32(m,1H),2.71(q,2H),2.61(m,2H),2.35(m,2H), 1.27(t,3H),1.26(d,3H),1.08(t,3H) | -3.28° (c=0.69) |
| 1-98 | 8.22(br,1H),8.18(d,1H),7.98(d,1H),7.71(d,1H), 7.46(s,1H),7.45(d,1H), 7.22(t,1H),6.70(d,1H), 4.62(m,1H),3.31(dd,1H),3.08(dd,1H),2.83(q,2H), 2.70(q,2H),1.52(d,3H),1.27(t,3H),1.21(t,3H) | -4.23° (c=0.99) |
| 1-99 | 8.42(br,1H),8.19(d,1H),7.97(d,1H),7.78(d,1H), 7.23-7.29(m,2H),7.21(t, 1H),6.22(d,1H), 4.32(m,1H),4.02(m,1H),2.68(q,2H),2.58(m,2H), 1.89(s, 3H),1.26(d,3H),1.25(t,3H) | -1.48° (c=1.05) |
| 1-101 | 8.18 (br, 1H), 8.05 (d, 1H), 7.96 (d, 1H), 7.68 (d, 1H), 7.25-7.29 (m, 2H), 7.20 (t, 1H), 6.73 (d, 1H), 4.59 (m, 1H), 4.03 (m, 1H), 3.35 (dd, 1H), 3.14 (dd,1H), 2.69(s,3H),2.66(q,2H),1.51(d,3H),1.25(t,3H) | -2.58° (c=1.07) |
| 1-102 | 8.41(br,1H),8.21(d,1H),7.98(d,1H),7.80(d,1H), 7.19-7.29(m,3H),6.17(d, 1H),4.31(m,1H), 4.02(m,1H),2.68(q,2H),2.62(m,2H),2.36(m,2H), 1.26(d, 3H),1.25(t,3H),1.08(t,3H) | -5.44° (c=1.16) |

(continued)

| No. | NMR data $^1$H-NMR [$CDCl_3$/TMS, δ (ppm)] | Specific rotation $[\alpha]_D^{20}$($CHCl_3$) |
|---|---|---|
| 1-104 | 8.17(br,1H),8.06(d,1H),7.97(d,1H),7.69(d,1H), 7.27(d,1H),7.26(s,1H), 7.21(t,1H),6.69(d,1H), 4.61(m,1H),4.03(m,1H),3.20(dd,1H),3.07(dd,1H), 2.80(q,2H),2.66(q,2H),1.52(d,3H),1.25(t,3H), 1.20(t,3H) | -3.63° (c=1.01) |
| 1-222 | 8.82 (br, 1H), 8.69 (d, 1H), 8.02 (d, 1H), 8.76 (d, 1H), 7.62 (s, 1H), 7.52 (d, 1H), 7.24 (t, 1H), 6.04 (d, 1H), 4.36 (m, 1H), 2.62 (m, 2H), 1.97 (s, 3H), 1.28 (d, 3H) | (c=1.10) |
| 1-224 | 8.65(d,1H),8.55(br,1H),8.04(d,1H),8.72(d,1H), 7.64(s,1H),7.54(d,1H), 7.26(t,1H),6.43(d,1H), 4,62(m,1H),3.49(dd,1H),3.25(dd,1H),2.94(s,3H), 1.55(d,3H) | +7.94° (c=1.11) |
| 1-225 | 8.71(br,1H),8.62(d,1H),8.01(d,1H),7.81(s,1H), 7.75(d,1H),7.58(d,1H), 7.24(t,1H),6.02(d,1H), 4.37(m,1H),2.63(m,2H),1.96(s,3H),1.29(d,3H) | -9.28° (c=1.04) |
| 1-227 | 8.59(d,1H),8.48(br,1H),8.04(d,1H),8.82(s,1H), 7.71(d,1H),7.60(d,1H), 7.25(t,1H),6.42(d,1H), 4.63(m,1H),3.50(dd,1H),3.25(dd,1H),2.93(s,3H), 1.56(d,3H) | +8.56° (c=1.02) |
| 1-231 | 8.82(br,1H),8.60(t,1H),8.00(d,1H),7.79(d,1H), 7.37-7.43(m,2H),7.23(t, 1H),6.05(d,1H), 4.37(m,1H), 2.63(br,2H),1.94(s,3H),1.30(d,3H) | |
| 1-233 | 8.49-8.55(m,2H),8.01(d,1H),7.72(d,1H), 7.43(s,1H),7.40(d,1H),7.24(t, 1H),6.51(d,1H), 4.61(m,1H),3.48(dd,1H),3.23(dd,1H),2.89(s,3H), 1.55(d, 3H) | +7.48° (c=1.43) |
| 1-234 | 8.60 (s, 1H), 8.40 (d, 1H), 8.00-8.10 (m, 1H), 7.80-7.90 (m, 2H), 7.60-7.70 (m, 3H), 7.45 (d, 1H), 7.40 (s, 1H), 6.80 (d, 1H), 4.50 (m, 1H), 2.60 (d, 2H), 2.40 (s, 3H), 1.90 (s, 3H), 1.25 (d, 3H) | |
| 1-236 | 8.32 (d, 1H), 8.24 (br, 1H), 8.01 (m, 1H), 7.82-7.89 (m, 2H), 7.61-7.68 (m, 3H), 7.46 (d, 1H), 7.45 (s, 1H), 6.96 (d, 1H), 4.78 (m, 1H), 3.31 (dd, 1H), 3.17 (dd, 1H), 2.69 (s, 3H), 1.44 (d, 3H) | |
| 1-237 | 8.50(br,1H),8.46(d,1H),8.00-8.06(m,2H), 7.76 (d, 1H), 7.61 (d, 1H), 7.24 (t, 1H), 6.08 (d, 1H), 4.38 (m, 1H), 2.65 (m, 2H), 1.99 (s, 3H), 1.30 (d, 3H) | |
| 1-240 | 8.55(br,1H),8.36(d,1H),7.80(d,1H),7.74(d,1H), 7.46(d,1H),7.42(s,1H), 7.40(t,1H),6.22(d,1H), 4.33(m,1H),2.61(m,1H),2.39(s,3H),2.35(m,2H), 1.25(d,3H),1.09(t,3H) | -6.78° (c=1.23) |
| 1-242 | 8.30(br,1H),8.23(d,1H),7.70-7.77(m,2H), 7.46(d,1H),7.44(s,1H),7.40(t, 1H),6.71(d,1H), 4.65(m,1H),3.31(dd,1H),3.09(dd,1H),2.84(q,2H), 2.39(s, 3H),1.52(d,3H),1.23(t,3H) | -2.83° (c=0.99) |
| 1-243 | 8.49(br, 1H) 8.23 (d,1H) 7.77 (d, 1H) 7.72 (d, 1H), 7.37 (t,1H), 7.23-7.27 (m, 2H), 6.24 (d, 1H), 4.32 (m, 1H), 3.99 (m, 1H), 2.61 (m, 2H), 2.35 (s, 3H), 2.31-2.39 (m, 2H), 1.25 (d, 3H), 1.09 (t, 3H) | -6.42° (c=1.06) |
| 1-245 | 8.17 (br, H), 8.13 (d, 1H), 7.69-7.75 (m, 2H), 7.39 (t, 1H), 7.24-7.29 (m, 2H), 6.62 (d, 1H), 4.64 (m, 1H), 4.01 (m, 1H), 3.29 (dd, 1H), 3.08 (dd, 1H), 2.81 (m, 2H), 2.35 (s, 3H), 1.52 (d, 3H), 1.22 (t, 3H) | -3.29° (c=1.05) |
| 1-246 | 8.50 (br, 1H), 8.20 (d, 1H), 7.75 (d, 1H), 7.70 (d, 1H), 7.36 (t, 1H), 7.24 (d, 1H), 7.23 (s, 1H), 6.27 (d, 1H), 4.33(m,1H),3.99(m,1H),2.57(m,2H), 2.34(s,3H), 1.89(s,3H),1.25(d,3H) | -3.41° (c=1.10) |
| 1-248 | 8.35 (br, 1H), 7.99 (d, 1H), 7.66 (d, 1H), 7.62 (d, 1H), 7.32 (t, 1H), 7.24 (s, 1H), 7.23 (d, 1H), 6.85 (d, 1H), 4.57 (m, 1H), 4.00 (m, 1H), 3.31 (dd, 1H), 3.11 (dd, 1H), 2.65 (s, 3H), 2.32 (s, 3H), 1.47 (d, 3H) | -1.66° (c=1.96) |
| 1-249 | 8.51 (br, 1H), 8.22 (d, 1H), 7.74 (d, 1H), 7.41-7.58 (m, 2H), 7.26 (d, 1H), 7.24 (s, 1H), 6.22 (d, 1H), 4.35 (m, 1H), 4.00 (m, 1H), 2.58 (dd, 2H), 2.36 (s, 3H), 1.91 (s, 3H), 1.26 (d, 3H) | -1.51° (c=1.06) |
| 1-251 | 8.32 (br, 1H), 8.02 (d, 1H), 7.37-7.63 (m, 3H), 7.22-7.27 (m, 2H), 6.80 (d, 1H), 4.60 (m, 1H), 4.01(m,1H),3.33(dd,1H),3.13(dd,1H),2.68(s,3H), 2.33 (s,3H),1.48(d,3H) | -3.24° (c=1.08) |

(continued)

| No. | NMR data $^1$H-NMR [$CDCl_3$/TMS, δ (ppm)] | Specific rotation $[\alpha]_D^{20}$($CHCl_3$) |
|---|---|---|
| 1-252 | 8.59(br,1H),8.33(d,1H),7.75(d,1H),7.56(d,1H), 7.41-7.49(m,3H),6.27(d, 1H),4.33(m,1H), 2.60(m,2H),2.39(s,3H),2.30-2.37(m,2H), 1.24(d,3H), 1.08(t,3H) | -3.86° (c=1.04) |
| 1-254 | 8.36(br,1H),8.18(d,1H),7.66(d,1H), 7.41-7.58(m,4H),6.76(d,1H),4.64(m, 1H), 3.30(dd,1H),3.05(dd,1H),2.83(q,2H),2.38(s,3H), 1.51(d,3H),1.21(t, 3H) | -3.86° (c=1.03) |
| 1-255 | 8.56(br,1H),8.18(d,1H),7.71(d,1H),7.53(d,1H), 7.43(t,1H),7.24(d,1H), 7.23(s,1H),6.30(d,1H), 4.32(m,1H),3.99(m,1H),2.60(m,2H),2.34(s,3H), 2.31-2.40(m,2H),1.24(d,3H),1.09(t,3H) | -4.18° (c=1.02) |
| 1-257 | 8.10-8.17(m,2H),7.69(d,1H),7.43-7.60(m,3H), 7.30 (s, 1H), 6.60 (d, 1H), 4.66 (m, 1H), 4.01 (m, 1H), 3.30 (dd, 1H), 3.05 (dd, 1H), 2.82 (q, 2H), 2.35 (s, 3H), 1.53(d,3H),1.22(t,3H) | -6.23° (c=1.17) |
| 1-260 | 8.24(br,1H),8.23(d,1H),7.66(d,1H),7.56(d,1H), 7.42-7.48(m,3H),6.70(d, 1H),4.63(m,1H), 3.34(dd,1H),3.17(dd,1H),2.75(s,3H),2.38(s,3H), 1.48(d, 3H) | +0.48° (c=1.00) |
| 1-263 | 8.14(d,1H),8.13(br,1H),7.66(d,1H),7.56,(d,2H), 7.46(t,1H),7.26(d,1H), 7.25(s,1H),6.64(d,1H),4.63(m,1H),4.01(m,1H),3.34(dd,1H),3.17(dd,1H), 2.73(s,3H),2.35(s,3H),1.49(d,3H) | +2.42° (c=1.02) |
| 1-264 | 8.70 (br, 1H), 8.17 (d, 1H), 7.78 (d, 1H), 7.58 (d, 1H), 7.49(t, 1H), 7.46 (t, 1H), 6.13 (d, 1H), 4.37 (m, 1H), 2.60 (m, 2H), 2.28 (d, 3H), 1.94 (s, 3H), 1.27 (d, 3H) | +2.72° (c=1.04) |
| 1-266 | 8.49 (br, 1H), 7.95 (d, 1H), 7.65 (d, 1H), 7.55 (d, 1H), 7.46 (t, 1H), 7.43 (t, 1H), 6.72 (d, 1H), 4.62 (m, 1H), 3.36 (dd, 1H), 3.19 (dd, 1H), 2.80 (s, 3H), 2.26 (d, 3H), 1.50(d,3H) | +1.76° (c=1.23) |
| 1-267 | 8.42(br,1H),8.09(d,1H),7.76(d,1H),7.55(d,1H), 7.46(t,1H),7.08(d,1H), 7.07(s,1H),6.17(d,1H), 4.36(m,1H),2.62(dd,1H),2.58(dd,1H),2.34(s,3H), 1.98(s,3H),1.26(d,3H) | +6.04° (c=1.05) |
| 1-269 | 8.04(br,1H),7.96(d,1H),7.67(d,1H),7.56(d,1H), 7.46(t,1H),7.09(br,1H), 6.58(d,1H),4.64(m,1H), 3.36(dd,1H),3.19(dd,1H),3.80(s,3H),2.33(s,3H), 1.51(d,3H) | |
| 1-270 | 8.67(br,1H),8.49(d,1H),7.70(d,1H),7.58(d,1H), 7.47(t,1H),7.31(s,1H), 7.19(d,1H),6.08(d,1H), 4.39(m,1H),2.67(dd,1H),2.61(dd,1H),2.02(s,3H), 1.29(d,3H) | -0.30° (c=1.01) |
| 1-272 | 8.46(d,1H),8.39(br,1H),7.65(d,1H),7.60(d,1H), 7.49(t,1H),7.33(s,1H), 7.21(d,1H),6.46(d,1H), 4.64(m,1H),3.48(dd,1H),3.24(dd,1H),2.96(s,3H), 1.54(d,3H) | +12.19° (c=1.06) |
| 1-273 | 8.58 (br, 1H), 8.44 (d, 1H), 7.69 (d, 1H), 7.58 (d, 1H), 7.48 (s, 1H), 7.47 (t, 1H), 7.24 (d, 1H), 6.08 (d, 1H), 4.39 (m, 1H), 2.68 (dd, 1H), 2.62 (dd, 1H), 2.03 (s, 3H), 1.29(d,3H) | -1.81° (c=1.10) |
| 1-275 | 8.43 (d, 1H), 8.33 (br, 1H), 7.66 (d, 1H), 7.60 (d, 1H), 7.46-7.53 (m, 2H), 7.23 (s, 1H), 6.42 (d, 1H), 4.64 (m, 1H), 3.49 (dd, 1H), 3.26 (dd, 1H), 2.97 (s, 3H), 1.55(d,3H) | |
| 1-278 | 8.27 (d, 1H), 8.16 (br, 1H), 7.99 (d, 1H), 7.73 (d, 1H), 7.45 (d, 1H),7.42 (s, 1H), 7.23 (t, 1H), 6.63 (d, 1H), 4.62 (m, 1H), 3.35 (dd, 1H), 3.17 (dd, 1H), 2.74 (s, 3H), 2.38 (s, 3H), 1.50 (d, 3H) | +2.41° (c=2.04) |
| 1-281 | 8.08-8.20 (m, 2H), 7.97 (d, 1H), 7.69 (d, 1H), 7.22-7.27 (m, 2H), 7.21 (t, 1H), 6.70 (d, 1H), 4.60(m,1H),4.00(m,1H),3.34(dd,1H),3.16(dd,1H), 2.70 (s,3H),2.34(s,3H),1.49(d,3H) | -0.39° (c=1.01) |
| 1-282 | 8.30(s,1H),8.26(d,1H),8.19(d,1H),7.98(d,1H), 7.97(d,1H),7.81(d,1H), 7.23(t,1H),7.03(d,1H), 7.02(s,1H),6.19(d,1H),4.36(m,1H),2.67(dd,1H), 2.59(dd,1H),2.35(s,3H),2.01(s,3H),1.29(d,3H) | +3.73° (c=1.06) |

(continued)

| No. | NMR data $^1H$-NMR [$CDCl_3$/TMS, δ (ppm)] | Specific rotation $[\alpha]_D^{20}$($CHCl_3$) |
|---|---|---|
| 1-284 | 8.24(d,1H),8.03(d,1H),7.95-8.00(m,3H), 7.71(d,1H),7.22(t,1H),7.03(s, 1H),7.02(d,1H), 6.60(d,1H),4.65(m,1H),3.39(dd,1H),3.20(dd,1H), 2.84(s, 3H),2.35(s,3H),1.57(d,3H) | -1.98° (c=1.01) |
| 1-285 | 8.39(s,1H),8.26(d,1H),8.13(d,1H),7.97(d,1H), 7.75(d,1H),7.55(d,1H), 7.46(t,1H),7.02(d,1H), 7.01(s,1H),6.23(d,1H),4.37(m,1H),2.69(dd,1H), 2.61(dd,1H),2.37(s,3H),2.05(s,3H),1.31(d,3H) | +4.93° (c=1.02) |
| 1-287 | 8.24 (d, 1H), 8.08 (s, 1H), 7.98 (d, 1H), 7.96 (d, 1H), 7.63 (d, 1H), 7.54 (d, 1H), 7.44 (t, 1H), 7.03 (s, 1H), 7.02 (d, 1H), 6.59 (d, 1H), 4.65 (m, 1H), 3.38 (dd, 1H), 3.19(dd,1H),2.83(s,3H),2.34(s,3H),1.56(d,3H) | |
| 1-288 | 8.78(br,1H),8.77(d,1H),7.80(d,1H),7.60(d,1H), 7.55(d,1H),7.50(t,1H), 6.09(d,1H),4.39(m,1H), 2.64(s,3H),2.59(d,2H),1.90(s,3H),1.28(d,3H) | -4.10° (c=1.02) |
| 2-4 | 8.80(d,1H),8.63(br,1H),8.02(d,1H),7.84(d,1H), 7.54(d,1H),7.26(t,1H), 6.04(d,1H),4.37(m,1H), 2.63(s,3H),2.58(d,2H),1.87(s,3H),1.28(d,3H) | -10.27° (c=1.04) |
| 2-6 | 8.63(d,1H),8.41(br,1H),7.99(d,1H),7.74(d,1H), 7.54(d,1H),7.24(t,1H), 6.72(d,1H),4.64(m,1H), 3.38(dd,1H),3.19(dd,1H),2.78(s,3H),2.61(s,3H), 1.51(d,3H) | |
| 2-10 | 8.70(d,1H),8.62(br,1H),8.01(d,1H),7.83(d,1H), 7.42(d,1H),7.25(t,1H), 6.06(d,1H), 4.31-4.48(m,2H),2.61(s,3H),2.56-2.61(m,2H), 1.87(s,3H), 1.28(d,3H) | -7.39° (c=1.01) |
| 2-12 | 8.52(d,1H),8.30(br,1H),7.99(d,1H),7.74(d,1H), 7.41(d,1H),7.24(t,1H), 6.65(d,1H),4.63(m,1H),4.39(m,1H),3.37(dd,1H),3.18(dd,1H),2.76(s,3H), 2.58(s,3H),1.52(d,3H) | |
| 2-15 | 8.57(d,1H),8.49(br,1H),7.68(d,1H),7.57(d,1H), 7.53(d,1H),7.48(t,1H), 6.71(d,1H),4.65(m,1H), 3.36(dd,1H),3.19(dd,1H),2.78(s,3H),2.62(s,3H), 1.50(d,3H) | +2.66° (c=1.03) |

EXAMPLES

Typical examples of the present invention are described below but they should not be construed as limiting the scope of the invention.

As used in the examples, the terms "part" and "parts" are by weight.

Formulation Example 1

| | |
|---|---|
| Each compound listed in Tables 1 and 2 | 10 parts |
| Xylene | 70 parts |
| N-methylpyrrolidone | 10 parts |
| Mixture of polyoxyethylene nonylphenyl | 10 parts |

ether and calcium alkylbenzenesulfonate

An emulsion was prepared by mixing uniformly the above ingredients to effect dissolution.

Formulation Example 2

| | |
|---|---|
| Each compound listed in Tables 1 and 2 | 3 parts |
| Clay powder | 82 parts |
| Diatomaceous earth powder | 15 parts |

A dust was prepared by mixing uniformly and grinding the above ingredients.

Formulation Example 3

| | |
|---|---|
| Each compound listed in Tables 1 and 2 | 5 parts |
| Mixed powder of bentonite and clay | 90 parts |
| Calcium ligninsulfonate | 5 parts |

Granules were prepared by mixing the above ingredients uniformly, and kneading the resulting mixture together with a suitable amount of water, followed by granulation and drying.

Formulation Example 4

| | |
|---|---|
| Each compound listed in Tables 1 and 2 | 20 parts |
| Mixture of kaolin and synthetic kaoline and high-dispersion silicic acid | 75 parts |
| Mixture of polyoxyethylene nonylphenyl ether and calcium alkylbenzenesulfonate | 5 parts |

A wettable powder was prepared by mixing uniformly and grinding the above ingredients.

Test Example 1: Insecticidal activity on diamond back moth (Plutella xylostella)

A cabbage leaf disc (variety: Shikidori) cut into 7cm-diameter was dipped for about 30 seconds into a chemical solution prepared by diluting a preparation containing each compound listed in Tables 1 and 2 as an active ingredient to adjust the concentration to 50 ppm. After dried, it was put into a plastic Petri dish with a diameter of 9 cm and inoculated with ten of the third instar larvae of diamond back moths. Eight days after the inoculation, the dead and alive larvae were counted. The mortality was calculated according to the following equation and the insecticidal activity was judged according to the criterion shown below. The test was carried out with three replications under the condition of 25°C, 50-60%RH and 16L-8D photoperiod.

$$\text{Mortality (\%)} = \frac{\text{Number of alive larvae in untreated group} - \text{Number of alive larvae in treated group}}{\text{Number of alive larvae in untreated group}} \times 100$$

Criterion:

| | |
|---|---|
| A | Mortality 100% |
| B | Mortality 99%-90% |
| C | Mortality 89%-80% |
| D | Mortality 79%-50% |

As a result, the following compounds were rated A: compound Nos. 1-3, 1-4, 1-5, 1-6, 1-7, 1-8, 1-9, 1-10, 1-11, 1-12, 1-13, 1-14, 1-15, 1-17, 1-18, 1-20, 1-21, 1-22, 1-23, 1-24, 1-25, 1-26, 1-27, 1-28, 1-29, 1-30, 1-31, 1-32, 1-33, 1-34, 1-35, 1-36, 1-38, 1-39, 1-40, 1-41, 1-42, 1-43, 1-44, 1-45, 1-47, 1-48, 1-49, 1-50, 1-51, 1-53, 1-54, 1-56, 1-57,

1-59, 1-60, 1-62, 1-63, 1-64, 1-65, 1-66, 1-68, 1-72, 1-73, 1-74, 1-75, 1-76, 1-77, 1-78, 1-79, 1-80, 1-81, 1-82, 1-83, 1-84, 1-85, 1-86, 1-87, 1-88, 1-89, 1-90, 1-92, 1-93, 1-95, 1-96, 1-98, 1-99, 1-101, 1-102, 1-104, 1-105, 1-222, 1-224, 1-225, 1-227, 1-231, 1-233, 1-234, 1-236, 1-237, 1-240, 1-241, 1-242, 1-243, 1-244, 1-245, 1-246, 1-247, 1-248, 1-249, 1-250, 1-251, 1-252, 1-253, 1-254, 1-255, 1-256, 1-257, 1-260, 1-263, 1-264, 1-266, 1-267, 1-269, 1-270, 1-272, 1-273, 1-275, 1-278, 1-281, 1-282, 1-283, 1-284, 1-285, 1-287, 1-288, 1-290, 1-291, 1-293, 1-306, 1-307, 1-308, 1-315, 1-317, 1-321,1-323,1-327,1-328,1-329,1-330,1-331,1-332,1-333,1-334,1-335,2-4, 2-5, 2-6, 2-10, 2-12, 2-13, 2-15, 2-16, 2-17, 2-18, 2-19, 2-20, 2-21, 2-27, 2-28, 2-30 and 2-32.

Test Example 2: Insecticidal activity on common cutworm (Spodoptera litura)

A cabbage leaf disc (variety: Shikidori) cut into 7cm-diameter was dipped for about 30 seconds into a chemical solution prepared by diluting a preparation containing each compound listed in Tables 1 and 2 as an active ingredient to adjust the concentration to 50 ppm. After dried, it was put into a plastic Petri dish with a diameter of 9 cm and inoculated with ten of the second instar larvae of common cutworm. Eight days after the inoculation, the dead and alive larvae were counted. The mortality was calculated and the insecticidal activity was judged in the same manner as in Test Example 1. The test was carried out with three replications under the condition of 25°C,50-60%RH and 16L-8D photoperiod.

As a result, the following compounds were rated A: compound Nos. 1-3, 1-4, 1-5, 1-6, 1-7, 1-8, 1-9, 1-10, 1-11, 1-12, 1-13, 1-14, 1-15, 1-17, 1-18, 1-20, 1-21, 1-22, 1-23, 1-24, 1-25, 1-26, 1-27, 1-28, 1-29, 1-30, 1-31, 1-32, 1-33, 1-34, 1-35, 1-36, 1-38, 1-39, 1-40, 1-41, 1-42, 1-43, 1-44, 1-45, 1-47, 1-48, 1-49, 1-50, 1-51, 1-53, 1-54, 1-56, 1-57, 1-59, 1-60, 1-62, 1-63, 1-64, 1-65, 1-66, 1-68, 1-72, 1-73, 1-74, 1-75, 1-76, 1-77, 1-78, 1-79, 1-80, 1-81, 1-82, 1-83, 1-84, 1-85, 1-86, 1-87, 1-88, 1-89, 1-90, 1-92, 1-93, 1-95, 1-96, 1-98, 1-99, 1-101, 1-102, 1-104, 1-105, 1-222, 1-225, 1-227, 1-231, 1-233, 1-234, 1-237, 1-240, 1-241, 1-242, 1-243, 1-244, 1-245, 1-246, 1-247, 1-248, 1-249, 1-250, 1-251, 1-252, 1-253, 1-254, 1-255, 1-256, 1-257, 1-260, 1-263, 1-264, 1-266, 1-269, 1-270, 1-272, 1-273, 1-275, 1-278, 1-281, 1-282, 1-283, 1-284, 1-285, 1-287, 1-288, 1-290, 1-291, 1-293, 1-306, 1-307, 1-308, 1-321,1-327,1-328,1-329,1-331,1-332,1-333,1-334,1-335,2-4, 2-5, 2-6, 2-10, 2-12, 2-13, 2-15, 2-16, 2-17, 2-18, 2-28 and 2-30.

Test Example 3: Insecticidal activity on smaller tea tortrix (Adxophyes sp.)

Tea leaves were dipped for about 30 seconds in a chemical solution prepared by diluting a preparation containing each compound listed in Tables 1 and 2 as an active ingredient to adjust the concentration to 50 ppm. After dried, the tea leaves were placed in a plastic Petri dish with a diameter of 9 cm and inoculated with larvae of smaller tea tortrix. Eight days after the inoculation, the dead and alive larvae were counted. The mortality was calculated and the insecticidal activity was judged in the same manner as in Test Example 1. The test was carried out with three replications under the condition of 25°C,50-60%RH and 16L-8D photoperiod.

As a result, the following compounds were rated A: compound Nos. 1-3, 1-4, 1-5, 1-6, 1-7, 1-8, 1-9, 1-10, 1-11, 1-12, 1-13, 1-14, 1-15, 1-17, 1-18, 1-20, 1-21, 1-22, 1-23, 1-24, 1-25, 1-26, 1-27, 1-28, 1-29, 1-30, 1-31, 1-32, 1-33, 1-34, 1-35, 1-36, 1-38, 1-42, 1-44, 1-45, 1-47, 1-48, 1-50, 1-51, 1-53, 1-54, 1-56, 1-60, 1-62, 1-64, 1-66, 1-68, 1-72, 1-73, 1-74, 1-75, 1-76, 1-77, 1-78, 1-79, 1-81, 1-82, 1-83, 1-84, 1-85, 1-86, 1-90, 1-92, 1-93, 1-95, 1-96, 1-98, 1-99, 1-104, 1-105, 1-222, 1-224, 1-225, 1-227, 1-231, 1-233, 1-234, 1-236, 1-237, 1-240, 1-241, 1-242, 1-243, 1-244, 1-245, 1-246, 1-247, 1-248, 1-249, 1-250, 1-251, 1-252, 1-253, 1-254, 1-255, 1-256, 1-257, 1-260, 1-263, 1-264, 1-266, 1-267, 1-269, 1-270, 1-278, 1-281, 1-282, 1-283, 1-284, 1-285, 1-287, 1-288, 1-290, 1-291, 1-293, 1-307, 1-308, 1-327, 1-328, 1-329, 1-330, 1-332, 1-333, 1-334, 1-335, 2-10, 2-12, 2-13, 2-15, 2-16, 2-17, 2-28 and 2-30.

Comparative test 1: Systemic activity of agent in Chinese cabbage

A Chinese cabbage seedling (variety: Aichi) cultured in a paper pot filled with horticultural molding was placed in a cylindrical plastic container. An agent containing, as an active ingredient, each of the compounds shown in the table and a comparative compound 1 (the compound with Compound No. 216 described in JP-A-2001-131141), was diluted with water into concentrations of 0.03, 0.1, 0.3, 1, and 3 ppm, so as to prepare 10 ml each of an agent solution. The plant foot of each Chinese cabbage seedling was drenched in the obtained solution . The Chinese cabbage was inoculated with the third instar larvae of common cutworm (Spodoptera litura). Four days after the inoculation, the number of surviving larvae was counted. The mortality was calculated according to the expression indicated below, and it was then evaluated in accordance with the criterion indicated below. The results are shown in Table 5. The test was carried out with three replications under the condition of 25°C, 50-60%RH and 16L-8D photoperiod.

$$\text{Mortality} = \frac{\text{Number of alive larvae in untreated group} - \text{Number of alive larvae in treated group}}{\text{Number of alive larvae in untreated group}} \times 100$$

Criterion:

| | | | |
|---|---|---|---|
| 10 | Mortality 91%-100% | 9 | Mortality 81%-90% |
| 8 | Mortality 71%-80% | 7 | Mortality 61%-70% |
| 6 | Mortality 51%-60% | 5 | Mortality 41%-50% |
| 4 | Mortality 31%-40% | 3 | Mortality 21%-30% |
| 2 | Mortality 11%-20% | 1 | Mortality 1%-10% |
| 0 | Mortality 0% | | |

Table 5

| Compound | concentration (ppm) | Mortality evaluation (4 days later) |
|---|---|---|
| 1-4 | 3 | 10 |
| | 1 | 10 |
| | 0.3 | 5 |
| | 0.1 | 4 |
| | 0.03 | 0 |
| 1-5 | 3 | 10 |
| | 1 | 10 |
| | 0.3 | 10 |
| | 0.1 | 4 |
| | 0.03 | 3 |
| 1-77 | 3 | 10 |
| | 1 | 10 |
| | 0.3 | 5 |
| | 0.1 | 3 |
| | 0.03 | 0 |
| 1-74 | 3 | 10 |
| | 1 | 10 |
| | 0.3 | 10 |
| | 0.1 | 3 |
| | 0.03 | 2 |
| Comparative compound 1 | 3 | 10 |
| | 1 | 4 |
| | 0.3 | 2 |
| | 0.1 | 0 |
| | 0.03 | 0 |
| Untreated | | 0 |

When compared with the comparative compound, the compound of the present invention clearly exhibited

superiority.

Comparative test 2: Control of common white (Pieris rapae) on cabbage by prick-in-hole application at transplanting

An agent containing, as an active ingredient (AI), each of the compounds shown in the table and a comparative compound 1 (the compound with Compound No. 216 described in JP-A-2001-131141), was put into a prick-in-hole on the ground, and then cabbage seedlings (Variety: YR Seitoku) transplanted onto the treated hole (at the end of April). 27 days after the transplanting, the number of larvae of common white was counted. The control value was calculated according to the expression indicated below, and it was then evaluated in accordance with the criterion noted in Comparative test 1 above. One test group consisted of 20 cabbage loaves. The results are shown in Table 6.

$$\text{Control value} = \frac{\text{Number of parasites in untreated group} - \text{Number of parasites in treated group}}{\text{Number of parasites in untreated group}} \times 100$$

Table 6

| Compound | Dosage (mgAI/plant ) | Evaluation of control value (27 days later) |
|---|---|---|
| 1-4 | 10 | 10 |
| 1-5 | 10 | 10 |
| 1-77 | 10 | 10 |
| 1-74 | 10 | 10 |
| Comparative compound 1 | 10 | 5 |
| Untreated | | 0 |

When compared with the comparative compound, the compound of the present invention clearly exhibited superiority.

Comparative test 3: Control of common cutworm (Spodoptera litura) on cabbage by prick-in-hole application at transplanting

An agent containing, as an active ingredient (AI), each of the compounds shown in the table and a comparative compound 1 (the compound with Compound No. 216 described in JP-A-2001-131141), was put into a prick-in-hole on the ground, and then cabbage seedlings (Variety: YR Seitoku) transplanted onto the treated hole (at the end of April). 30 days after the transplanting, the third leaf from top leaf was cut off and placed into a Petri dish with ten of the third instar larvae of common cutworm. Four days after the inoculation, the number of surviving larvae was counted. The mortality was calculated and evaluated in the same manner as in Comparative test 1 above. The test was carried out with two replications. The results are shown in Table 7.

Table 7

| Compound | Dosage (mgAI/loaf) | Mortality evaluation (30 days later) |
|---|---|---|
| 1-4 | 10 | 10 |
| 1-5 | 10 | 10 |
| 1-77 | 10 | 10 |
| 1-74 | 10 | 10 |

(continued)

| Compound | Dosage (mgAI/loaf) | Mortality evaluation (30 days later) |
|---|---|---|
| Comparative compound 1 | 10 | 2 |
| Untreated | | 0 |

When compared with the comparative compound, the compound of the present invention clearly exhibited superiority.

Comparative test 4: Control of common cutworm (Spodoptera litura) on cabbage by drenching application into nursery box

An agent containing, as an active ingredient, each of the compounds shown in the table and a comparative compound 1 (the compound with Compound No. 216 described in JP-A-2001-131141) was diluted into prescribed concentrations. A cabbage seedling (Variety: YR Seitoku) on the nursery box was drenched in the obtained solution at a rate of 4 ml/plant. Shortly after the drenching, the cabbage plant was transplanted to the field. 20 days after the transplanting, the third leaf from the top leaf was cut off and placed in a Petri dish with ten of the third instar larvae of common cutworm. Four days after the inoculation, the number of surviving larvae was counted. The mortality was calculated and evaluated in the same manner as in Comparative test 1 above. The test was carried out with two replications. The results are shown in Table 8.

Table 8

| Compound | Dosage | | Mortality evaluation (20 days later) |
|---|---|---|---|
| | (mgAI/loaf) | (ppm) | |
| 1-4 | 1.56 | 400 | 10 |
| | 0.78 | 200 | 10 |
| | 0.39 | 100 | 8 |
| 1-5 | 1.56 | 400 | 10 |
| | 0.78 | 200 | 10 |
| | 0.39 | 100 | 9 |
| Comparative compound 1 | 1.56 | 400 | 9 |
| | 0.78 | 200 | 0 |
| | 0.39 | 100 | 0 |
| Untreated | | | 0 |

When compared with the comparative compound, the compound of the present invention clearly exhibited superiority.

Comparative test 5: Control of Myzus persicae - systemic

An agent containing, as an active ingredient, each of representative of the compounds of the present invention (Compound Nos. 1-77 and 1-74), comparative compound 2 (Compound No. 101 described in JP-A-2001-131141) and comparative compound 3 (Compound No. 97 described in JP-A-2001-131141) was diluted into prescribed concentrations.

The preparation containing the active ingredient is mixed into water. The stated concentration is based on the amount of active ingredient per unit volume of water (mg/l = ppm). The diluted agent was filled into a container with an infested pea plant (Pisum sativum) with Green peach aphids (Myzus persicae).

After 6 days, mortality was determined. Table 9 shows the results. 100 % means that all the aphids have been killed; 0 % means that none of the aphids have been killed.

Table 9

| Compound | Dosage | Mortality (%) |
|---|---|---|
| | (ppm) | |
| 1-77 | 20 | 90 |

(continued)

| Compound | Dosage (ppm) | Mortality (%) |
|---|---|---|
| | 4 | 85 |
| | 0.8 | 0 |
| 1-74 | 100 | 70 |
| Comparative compound 2 | 20 | 0 |
| | 4 | 0 |
| | 0.8 | 0 |
| Comparative compound 3 | 100 | 0 |
| Untreated | | 0 |

When compared with the comparative compounds, the compounds of the present invention clearly exhited superiority.

Comparative test 6: Control of Spodoptera exigua

An agent containing, as an active ingredient, each of the compound of the present invention (Compound No. 1-77) and comparative compound 2 (Compound No. 101 described in JP-A-2001-131141) was diluted into prescribed concentrations.
Cabbage leaves (Brassica oleracea) were treated by being dipped into the preparation of the active compound of the desired concentration and were infested with caterpillars of the beet army worm (Spodoptera exigua) while the leaves were still moist.
After 7 days, mortality was determined. Table 10 shows the results. 100 % means that all the caterpillars have been killed; 0 % means that none of the caterpillars have been killed.

Table 10

| Compound | Dosage (ppm) | Mortality (%) |
|---|---|---|
| 1-77 | 4 | 100 |
| | 0.8 | 100 |
| | 0.16 | 100 |
| | 0.032 | 0 |
| | 0.0064 | 0 |
| | 4 | 100 |
| | 0.8 | 100 |
| Comparative compound 2 | 0.16 | 0 |
| | 0.032 | 0 |
| | 0.0064 | 0 |
| Untreated | | 0 |

In this test, the compound of the present invention showed remarkably higher efficacy than its racemate.

Comparative test 7: Control of Phaedon cochleariae

An agent containing, as an active ingredient, each of the compound of the present invention (Compound No. 1-77) and comparative compound 2 (Compound No. 101 described in JP-A-2001-131141) was diluted into prescribed concentrations.
Cabbage leaves (Brassica oleracea) were treated by being dipped into the diluted agent and were infested with mustard beetle larvae (Phaedon cochleariae) as long as the leaves were still moist.

After 7 days, mortality was determined. Table 11 shows the results. 100 % means that all the beetle larvae have been killed; 0 % means that none of the beetle larvae have been killed.

Table 11

| Compound | Dosage (ppm) | Mortality (%) |
|---|---|---|
| 1-77 | 100 | 100 |
| | 20 | 100 |
| | 4 | 100 |
| | 0.8 | 0 |
| | 0.16 | 0 |
| Comparative compound 2 | 100 | 100 |
| | 20 | 100 |
| | 4 | 0 |
| | 0.8 | 0 |
| | 0.16 | 0 |
| Untreated | | 0 |

In this test, the compound of the present invention showed remarkably higher efficacy than its racemate.

## Claims

**1.** An optically active phthalamide derivative represented by the following formula (I) or a salt thereof:

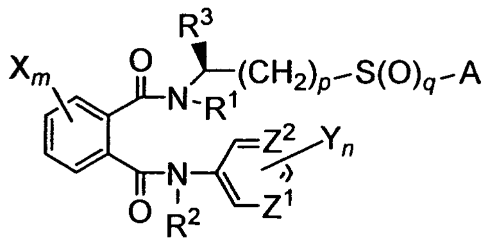

(I)

(wherein $R^1$ and $R^2$ may be same or different, and each of $R^1$ and $R^2$ represents a hydrogen atom; a C1-C6 alkyl group; a substituted C1-C6 alkyl group having one or more substituents that may be same or different and are selected from the group consisting of a halogen atom, a C1-C6 alkoxy group, a C1-C6 alkylthio group, a mono-C1-C6 alkylamino group, and a di-C1-C6 alkylamino group in which the alkyl groups may be same or different; or a C1-C6 alkoxycarbonyl group;
$R^3$ represents a C1-C6 alkyl group;
A represents a hydrogen atom; a C1-C6 alkyl group; a halo C1-C6 alkyl group; a C3-C6 alkenyl group; a halo C3-C6 alkenyl group; a C3-C6 alkynyl group; a halo C3-C6 alkynyl group; a C1-C6 alkoxy C1-C6 alkyl group; a halo C1-C6 alkoxy C1-C6 alkyl group; a C1-C6 alkylthio C1-C6 alkyl group; a halo C1-C6 alkylthio C1-C6 alkyl group; a C1-C6 alkylsulfinyl C1-C6 alkyl group; a halo C1-C6 alkylsulfinyl C1-C6 alkyl group; a C1-C6 alkylsulfonyl C1-C6 alkyl group; a halo C1-C6 alkylsulfonyl C1-C6 alkyl group; a C1-C6 alkylcarbonyl group; a mono-C1-C6 alkylcarbamoyl group; a di-C1-C6 alkylcarbamoyl group in which the alkyl groups may be same or different; a phenoxy C1-C6 alkyl group; a substituted phenoxy C1-C6 alkyl group having one or more substituents that may be same or different and are selected from the group consisting of a halogen atom, a C1-C6 alkyl group, a halo C1-C6 alkyl group, a C1-C6 alkoxy group, a halo C1-C6 alkoxy group, a C1-C6 alkylthio group, a halo C1-C6 alkylthio group, a C1-C6 alkylsulfinyl group, a halo C1-C6 alkylsulfinyl group, a C1-C6 alkylsulfonyl group, a halo C1-C6 alkylsulfonyl group, a mono-C1-C6 alkylamino group, a di-C1-C6 alkylamino group in which the alkyl groups may be same or different, a mono(halo C1-C6 alkyl)amino group, and a di(halo C1-C6 alkyl)amino group in which the alkyl groups

may be same or different; a phenyl C1-C6 alkyl group; or a substituted phenyl C1-C6 alkyl group having, on the ring thereof, one or more substituents that may be same or different and are selected from the group consisting of a halogen atom, a C1-C6 alkyl group, a halo C1-C6 alkyl group, a C1-C6 alkoxy group, a halo C1-C6 alkoxy group, a C1-C6 alkylthio group, a halo C1-C6 alkylthio group, a C1-C6 alkylsulfinyl group, a halo C1-C6 alkylsulfinyl group, a C1-C6 alkylsulfonyl group, a halo C1-C6 alkylsulfonyl group, a mono-C1-C6 alkylamino group, a di-C1-C6 alkylamino group in which the alkyl groups may be same or different, a mono(halo C1-C6 alkyl)amino group, and a di(halo C1-C6 alkyl)amino group in which the alkyl groups may be same or different;

p represents an integer between 0 and 4;

q represents an integer between 0 and 2;

X may be same or different and represents a halogen atom, a cyano group, an amino group, a nitro group, a C1-C6 alkyl group, a halo C1-C6 alkyl group, a C2-C6 alkenyl group, a halo C2-C6 alkenyl group, a C2-C6 alkynyl group, a halo C2-C6 alkynyl group, a C1-C6 alkoxy group, a halo C1-C6 alkoxy group, a C1-C6 alkylthio group, a halo C1-C6 alkylthio group, a C1-C6 alkylsulfinyl group, a halo C1-C6 alkylsulfinyl group, a C1-C6 alkylsulfonyl group, a halo C1-C6 alkylsulfonyl group, a mono-C1-C6 alkylamino group, or a di-C1-C6 alkylamino group in which the alkyl groups may be same or different; and

m represents an integer between 0 and 4; and

X may form a condensed ring together with carbon atoms adjacent thereto on a phenyl ring, and such a condensed ring may have one or more substituents that may be same or different and are selected from the group consisting of a halogen atom; a C1-C6 alkyl group; a halo C1-C6 alkyl group; a C1-C6 alkoxy group; a halo C1-C6 alkoxy group; a C1-C6 alkylthio group; a halo C1-C6 alkylthio group; a C1-C6 alkylsulfinyl group; a halo C1-C6 alkylsulfinyl group; a C1-C6 alkylsulfonyl group; a halo C1-C6 alkylsulfonyl group; a mono-C1-C6 alkylamino group; a di-C1-C6 alkylamino group in which the alkyl groups may be same or different; a mono(halo C1-C6 alkyl)amino group; a di(halo C1-C6 alkyl)amino group in which the alkyl groups may be same or different; a phenyl group; a substituted phenyl group having one or more substituents that may be same or different and are selected from the group consisting of a halogen atom, a C1-C6 alkyl group, a halo C1-C6 alkyl group, a C1-C6 alkoxy group, a halo C1-C6 alkoxy group, a C1-C6 alkylthio group, a halo C1-C6 alkylthio group, a C1-C6 alkylsulfinyl group, a halo C1-C6 alkylsulfinyl group, a C1-C6 alkylsulfonyl group, a halo C1-C6 alkylsulfonyl group, a mono-C1-C6 alkylamino group, a di-C1-C6 alkylamino group in which the alkyl groups may be same or different, a mono(halo C1-C6 alkyl) amino group, and a di(halo C1-C6 alkyl)amino group in which the alkyl groups may be same or different; a heterocyclic group; and a substituted heterocyclic group having one or more substituents that may be same or different and are selected from the group consisting of a halogen atom, a C1-C6 alkyl group, a halo C1-C6 alkyl group, a C1-C6 alkoxy group, a halo C1-C6 alkoxy group, a C1-C6 alkylthio group, a halo C1-C6 alkylthio group, a C1-C6 alkylsulfinyl group, a halo C1-C6 alkylsulfinyl group, a C1-C6 alkylsulfonyl group, a halo C1-C6 alkylsulfonyl group, a mono-C1-C6 alkylamino group, a di-C1-C6 alkylamino group in which the alkyl groups may be same or different, a mono(halo C1-C6 alkyl)amino group, and a di(halo C1-C6 alkyl)amino group in which the alkyl groups may be same or different;

Y may be same or different and represents a hydrogen atom, a halogen atom; a cyano group; an amino group; a nitro group; a C1-C6 alkyl group; a halo C1-C6 alkyl group; a C2-C6 alkenyl group; a halo C2-C6 alkenyl group; a C2-C6 alkynyl group; a halo C2-C6 alkynyl group; a cyclo C3-C6 alkyl group; a halocyclo C3-C6 alkyl group; a C1-C6 alkoxy group; a halo C1-C6 alkoxy group; a C1-C6 alkylthio group; a halo C1-C6 alkylthio group; a C1-C6 alkylsulfinyl group; a halo C1-C6 alkylsulfinyl group; a C1-C6 alkylsulfonyl group; a halo C1-C6 alkylsulfonyl group; a mono-C1-C6 alkylamino group; a di-C1-C6 alkylamino group in which the alkyl groups may be same or different; a tri-C1-C6 alkylsilyl group in which the alkyl groups may be same or different; a phenyl group; a substituted phenyl group having one or more substituents that may be same or different and are selected from the group consisting of a halogen atom, a C1-C6 alkyl group, a halo C1-C6 alkyl group, a C1-C6 alkoxy group, a halo C1-C6 alkoxy group, a C1-C6 alkylthio group, a halo C1-C6 alkylthio group, a C1-C6 alkylsulfinyl group, a halo C1-C6 alkylsulfinyl group, a C1-C6 alkylsulfonyl group, a halo C1-C6 alkylsulfonyl group, a mono-C1-C6 alkylamino group, a di-C1-C6 alkylamino group in which the alkyl groups may be same or different, a mono(halo C1-C6 alkyl)amino group, and a di(halo C1-C6 alkyl)amino group in which the alkyl groups may be same or different; a heterocyclic group; or a substituted heterocyclic group having one or more substituents that may be same or different and are selected from the group consisting of a halogen atom, a C1-C6 alkyl group, a halo C1-C6 alkyl group, a C1-C6 alkoxy group, a halo C1-C6 alkoxy group, a C1-C6 alkylthio group, a halo C1-C6 alkylthio group, a C1-C6 alkylsulfinyl group, a halo C1-C6 alkylsulfinyl group, a C1-C6 alkylsulfonyl group, a halo C1-C6 alkylsulfonyl group, a mono-C1-C6 alkylamino group, a di-C1-C6 alkylamino group in which the alkyl groups may be same or different, a mono(halo C1-C6 alkyl)amino group, and a di(halo C1-C6 alkyl)amino group in which the alkyl groups may be same or different; and

n represents an integer between 1 and 5; and

Y may form a condensed ring together with carbon atoms adjacent thereto on a phenyl ring, and such a condensed

ring may have one or more substituents that may be same or different and are selected from the group consisting of a halogen atom; a C1-C6 alkyl group; a halo C1-C6 alkyl group; a C1-C6 alkoxy group; a halo C1-C6 alkoxy group; a C1-C6 alkylthio group; a halo C1-C6 alkylthio group; a C1-C6 alkylsulfinyl group; a halo C1-C6 alkylsulfinyl group; a C1-C6 alkylsulfonyl group; a halo C1-C6 alkylsulfonyl group; a mono-C1-C6 alkylamino group; a di-C1-C6 alkylamino group in which the alkyl groups may be same or different; a mono(halo C1-C6 alkyl)amino group; a di(halo C1-C6 alkyl)amino group in which the alkyl groups may be same or different; a phenyl group; a substituted phenyl group having one or more substituents that may be same or different and are selected from the group consisting of a halogen atom, a C1-C6 alkyl group, a halo C1-C6 alkyl group, a C1-C6 alkoxy group, a halo C1-C6 alkoxy group, a C1-C6 alkylthio group, a halo C1-C6 alkylthio group, a C1-C6 alkylsulfinyl group, a halo C1-C6 alkylsulfinyl group, a C1-C6 alkylsulfonyl group, a halo C1-C6 alkylsulfonyl group, a mono-C1-C6 alkylamino group, a di-C1-C6 alkylamino group in which the alkyl groups may be same or different, a mono(halo C1-C6 alkyl) amino group, and a di(halo C1-C6 alkyl)amino group in which the alkyl groups may be same or different; a heterocyclic group; or a substituted heterocyclic group having one or more substituents that may be same or different and are selected from the group consisting of a halogen atom, a C1-C6 alkyl group, a halo C1-C6 alkyl group, a C1-C6 alkoxy group, a halo C1-C6 alkoxy group, a C1-C6 alkylthio group, a halo C1-C6 alkylthio group, a C1-C6 alkylsulfinyl group, a halo C1-C6 alkylsulfinyl group, a C1-C6 alkylsulfonyl group, a halo C1-C6 alkylsulfonyl group, a mono-C1-C6 alkylamino group, a di-C1-C6 alkylamino group in which the alkyl groups may be same or different, a mono(halo C1-C6 alkyl)amino group, and a di(halo C1-C6 alkyl)amino group in which the alkyl groups may be same or different; and

$Z^1$ and $Z^2$ may be same or different, and each of $Z^1$ and $Z^2$ represents C-Y (wherein Y has the same meaning as described above) or nitrogen atom;

provided that, when both $R^1$ and $R^2$ represent hydrogen atoms, $R^3$ represents a methyl group, X represents an iodine atom, m represents 1, Yn represents a 2-methyl-4-[1,2,2,2-tetrafluoro-1-(trifluoromethyl)ethyl] group, p represents 1, and q represents 0, then A is not a methyl group and ethyl group).

**2.** The optically active phthalamide derivative or a salt thereof according to claim 1, wherein $R^1$ and $R^2$ may be same or different, and each of $R^1$ and $R^2$ represents a hydrogen atom or C1-C6 alkyl group; $R^3$ represents a methyl group or ethyl group; A represents a C1-C6 alkyl group, a C3-C6 alkenyl group, a C3-C6 alkynyl group, a C1-C6 alkoxy C1-C6 alkyl group, a C1-C6 alkylthio C1-C6 alkyl group, a C1-C6 alkylsulfinyl C1-C6 alkyl group, a C1-C6 alkylsulfonyl C1-C6 alkyl group, or a mono-C1-C6 alkylcarbamoyl group; p represents an integer between 1 and 4; X may be same or different and represents a halogen atom, a nitro group, a halo C1-C6 alkyl group, or a halo C1-C6 alkoxy group; m represents an integer between 0 and 2; and Y may be same or different and represents a halogen atom, a cyano group, a C1-C6 alkyl group, a halo C1-C6 alkyl group, a C2-C6 alkenyl group, a halo C2-C6 alkenyl group, a C2-C6 alkynyl group, a halo C2-C6 alkynyl group, a C1-C6 alkoxy group, a halo C1-C6 alkoxy group, a C1-C6 alkylthio group, a halo C1-C6 alkylthio group, a C1-C6 alkylsulfinyl group, a halo C1-C6 alkylsulfinyl group, a C1-C6 alkylsulfonyl group, a halo C1-C6 alkylsulfonyl group, a mono(halo C1-C6 alkyl)amino group, or a di(halo C1-C6 alkyl)amino group in which the alkyl groups may be same or different, and Y may form a condensed ring together with carbon atoms adjacent thereto on a phenyl ring, and such a condensed ring may have one or more substituents that may be same or different and are selected from the group consisting of a halogen atom, a C1-C6 alkyl group, a halo C1-C6 alkyl group, a C1-C6 alkoxy group, a halo C1-C6 alkoxy group, a C1-C6 alkylthio group, a halo C1-C6 alkylthio group, a C1-C6 alkylsulfinyl group, a halo C1-C6 alkylsulfinyl group, a C1-C6 alkylsulfonyl group, a halo C1-C6 alkylsulfonyl group, a mono-C1-C6 alkylamino group, a di-C1-C6 alkylamino group in which the alkyl groups may be same or different, a mono(halo C1-C6 alkyl)amino group, and a di(halo C1-C6 alkyl)amino group in which the alkyl groups may be same or different; and n represents an integer between 1 and 4.

**3.** The optically active phthalamide derivative or a salt thereof according to claim 1, wherein $R^1$ and $R^2$ may be same or different, and each of $R^1$ and $R^2$ represents a hydrogen atom or a C1-C6 alkyl group; $R^3$ represents a methyl group; A represents a C1-C6 alkyl group, a C3-C6 alkenyl group, a C3-C6 alkynyl group, a C1-C6 alkoxy C1-C6 alkyl group, a C1-C6 alkylthio C1-C6 alkyl group, a C1-C6 alkylsulfinyl C1-C6 alkyl group, or a C1-C6 alkylsulfonyl C1-C6 alkyl group; p represents an integer between 1 and 4; q represents 1 or 2; X may be same or different and represents a halogen atom, a nitro group, a halo C1-C6 alkyl group, or a halo C1-C6 alkoxy group; m represents an integer between 0 and 2; and Y may be same or different and represents a halogen atom, a cyano group, a C1-C6 alkyl group, a halo C1-C6 alkyl group, a C2-C6 alkenyl group, a halo C2-C6 alkenyl group, a C2-C6 alkynyl group, a halo C2-C6 alkynyl group, a C1-C6 alkoxy group, a halo C1-C6 alkoxy group, a C1-C6 alkylthio group, a halo C1-C6 alkylthio group, a C1-C6 alkylsulfinyl group, a halo C1-C6 alkylsulfinyl group, a C1-C6 alkylsulfonyl group, or a halo C1-C6 alkylsulfonyl group, and Y may form a condensed ring together with carbon atoms adjacent thereto on a phenyl ring, and such a condensed ring may have one or more substituents that may be same or different and are selected from the group consisting of a halogen atom, a C1-C6 alkyl group, a halo C1-C6 alkyl

group, a C1-C6 alkoxy group, a halo C1-C6 alkoxy group, a C1-C6 alkylthio group, a halo C1-C6 alkylthio group, a C1-C6 alkylsulfinyl group, a halo C1-C6 alkylsulfinyl group, a C1-C6 alkylsulfonyl group, and a halo C1-C6 alkylsulfonyl group; and n represents an integer between 1 and 4.

**4.** An optically active phthalamide derivative selected from the group consisting of
(S)-3-iodo-$N^1$-{2-methyl-4-[1,2,2,2-tetrafluoro-1-(trifluoromethyl)ethyl]phenyl}-$N^2$-(1-methyl-2-methylsulfonylethyl) phthalamide,
(S)-3-chloro-N'-{2-methyl-4-[1,2,2,2-tetrafluoro-1-(trifluoromethyl)ethyl]phenyl}-$N^2$-(1-methyl-2-methylsulfonylethyl)phthalamide,
(S)-3-bromo-$N^1$-{2-methyl-4-[1,2,2,2-tetrafluoro-1-(trifluoromethyl)ethyl]phenyl}-$N^2$-(1-methyl-2-methylsulfonylethyl)phthalamide, and
(S)-3-iodo-$N^1$-{2-methyl-4-[1,2,2,2-tetrafluoro-1-(trifluoromethyl)ethyl]phenyl}-$N^2$-(1-methyl-2-methylsulfinylethyl) phthalamide, or a salt thereof.

**5.** An agrohorticultural insecticide, which comprises, as an active ingredient, the optically active phthalamide derivative or a salt thereof according to any one of claims 1 to 4.

**6.** A method of using an agrohorticultural insecticide, which comprises treating a target plant or soil with an effective amount of the agrohorticultural insecticide according to claim 5, so as to control insect pests from useful plants.

## INTERNATIONAL SEARCH REPORT

International application No.
PCT/JP2005/015208

A. CLASSIFICATION OF SUBJECT MATTER
Int.Cl$^7$ C07C317/28, A01N37/18, 41/10, 43/40, C07C323/42, C07D213/64, 213/70, 213/75, 239/42//C07M7:00

According to International Patent Classification (IPC) or to both national classification and IPC

B. FIELDS SEARCHED
Minimum documentation searched (classification system followed by classification symbols)
Int.Cl$^7$ C07C317/28, A01N37/18, 41/10, 43/40, C07C323/42, C07D213/64, 213/70, 213/75, 239/42

Documentation searched other than minimum documentation to the extent that such documents are included in the fields searched

Electronic data base consulted during the international search (name of data base and, where practicable, search terms used)
CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X | JP 2004-18506 A (Bayer AG.), 22 January, 2004 (22.01.04), Claims; examples & WO 2004/000796 A1 & EP 1517886 A1 | 1-6 |
| X | JP 2004-51614 A (Nissan Chemical Industries, Ltd.), 19 February, 2004 (19.02.04), Claims; examples & WO 2003/011028 A1 | 1-6 |
| X | JP 2003-34673 A (Nihon Nohyaku Co., Ltd.), 07 February, 2003 (07.02.03), Claims; examples & WO 2002/094765 A2 & EP 1390342 A2 & US 2004/152598 A1 | 1-6 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 07 September, 2005 (07.09.05) | 27 September, 2005 (27.09.05) |
| Name and mailing address of the ISA/ Japanese Patent Office | Authorized officer |
| Facsimile No. | Telephone No. |

Form PCT/ISA/210 (second sheet) (January 2004)

INTERNATIONAL SEARCH REPORT

International application No.
PCT/JP2005/015208

C (Continuation). DOCUMENTS CONSIDERED TO BE RELEVANT

| Category* | Citation of document, with indication, where appropriate, of the relevant passages | Relevant to claim No. |
|---|---|---|
| X | JP 2001-335563 A (Nihon Nohyaku Co., Ltd.), 04 December, 2001 (04.12.01), Claims; examples & WO 2001/002354 A1 & EP 1193254 A1 & US 6875768 B1 | 1-6 |
| X | JP 2001-131141 A (Nihon Nohyaku Co., Ltd.), 15 May, 2001 (15.05.01), Claims; examples & EP 1006107 A2 & US 6603044 B1 | 1-6 |
| X | JP 2001-163854 A (Nihon Nohyaku Co., Ltd.), 19 June, 2001 (19.06.01), Referential examples & WO 2001/023350 A1 & EP 1216988 A1 & US 6639109 B1 | 1-6 |
| X<br>A | JP 2001-335559 A (Nihon Nohyaku Co., Ltd.), 04 December, 2001 (04.12.01), Production method (Family: none) | 1-4<br>5-6 |

Form PCT/ISA/210 (continuation of second sheet) (January 2004)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 11240857 A
- JP 2001131141 A


### Non-patent literature cited in the description


- *J. Med. Chem.,* 1967, vol. 10, 982
- *Tetrahedron Lett.,* 1989, vol. 30, 2653